# EUROPÄISCHE PATENTSCHRIFT

(11) **EP 0 796 856 B1**
(45) Veröffentlichungstag und Bekanntmachung des Hinweises auf die Patenterteilung: **18.04.2001**
(21) Anmeldenummer: 97810139.2
(22) Anmeldetag: 12.03.1997
(51) Int. Cl.: C07D 405/04, C07D 307/79, C07D 307/85, C07D 231/18, C07D 231/12, C07D 231/14, C07D 231/16, A01N 43/56

(54) **Herbizid werksame (Dihydro-)Benzofuranyl substituierte Verbindungen**
Herbicidal, active (dihydro-)benzofuranyl substituted compounds
Composés substitués par des restes (dihydro)-benzofurannes, actifs comme herbicides

(30) Priorität: 19.03.1996 CH 72596
(43) Veröffentlichungstag der Anmeldung: 24.09.1997
(73) Patentinhaber: Syngenta Participations AG, 4058 Basel (CH)
(72) Erfinder: Pissiotas, Georg, 79539 Lörrach (DE); Nebel, Kurt, 4146 Hochwald (CH); Brunner, Hans-Georg, 4415 Lausen (CH)

(56) Entgegenhaltungen:
- EP-A- 0 271 170
- EP-A- 0 561 319
- EP-A- 0 617 033
- WO-A-92/06962
- JOURNAL OF HETEROCYCLIC CHEMISTRY, Bd. 21, Nr. 1, Januar 1984, PROVO US, Seiten 177-180, XP002033970 J.-P. BACHELET ET AL.: "SUR L'ORIENTATION ET L'ACETYLATION DES NITRILES ET AMIDES COUMARILIQUES"

## Beschreibung

Die vorliegende Erfindung betrifft neue, herbizid wirksame Benzofuranyl- und Dihydrobenzofuranyl-substituierte Pyrazolderivate, Verfahren zu ihrer Herstellung, Mittel, die diese Verbindungen enthalten, sowie ihre Verwendung zum Bekämpfen von Unkräutern, vor allem in Nutzpflanzenkulturen oder zum Hemmen des Pflanzenwachstums.

Benzofuranyl- und Dihydrobenzofuranyl-substituierte Uracile, Phthalimide, Oxadiazolone, Triazolone, Triazindione und Imidazolidindione mit herbizider Wirkung sind beispielsweise in US-A-4 881 967, EP-A-0 271 170, EP-A-0 476 697, EP-A-0 561 319, EP-A-0 617 033 und WO 95/05079 beschrieben.

Es wurden nun neue Benzofuranyl- und Dihydrobenzofuranyl-substituierte Pyrazolderivate mit herbiziden und wuchshemmenden Eigenschaften gefunden.

Gegenstand der vorliegenden Erfindung sind somit Verbindungen der Formel I 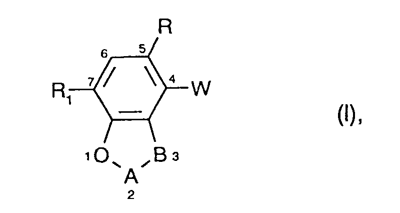 worin
R Wasserstoff, Fluor oder Chlor;
R₁ Wasserstoff, Halogen, Trifluormethyl, C₁-C₄-Halogenalkoxy, Cyano, Nitro oder Amino;
A-B eine Gruppe 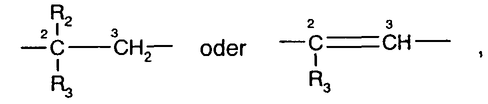 wobei das 2-Kohlenstoffatom an das Sauerstoffatom gebunden ist;
W eine Gruppe 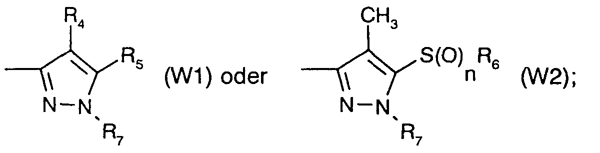
R₂ Wasserstoff oder C₁-C₆-Alkyl;
R₃ C₁-C₆-Alkyl, C₁-C₆-Halogenalkyl, Cyano-C₁-C₄-alkyl, Hydroxy-C₁-C₆-alkyl, C₁-C₆-Alkoxy-C₁-C₆-alkyl, C₃-C₆-Alkenyloxy-C₁-C₄-alkyl, C₃-C₆-Alkinyloxy-C₁-C₄-alkyl, C₁-C₆-Alkylcarbonyloxy-C₁-C₆-alkyl, Carboxyl, C₁-C₆-Alkoxycarbonyl, C₁-C₆-Halogenalkoxycarbonyl, C₃-C₆-Alkenyloxycarbonyl, C₃-C₆-Alkinyloxycarbonyl, C₃-C₆-Cycloalkoxycarbonyl, C₁-C₄-Alkoxy-C₁-C₄-alkoxycarbonyl, C₁-C₄-Alkylaminocarbonyl, Di-C₁-C₄-Alkylaminocarbonyl, Aminocarbonyl, Benzyloxycarbonyl, Phenyloxycarbonyl, C₁-C₄-Alkyl-SO₂NHC(O)-, C₁-C₆-Alkyl-ON=CH-, C₁-C₆-Alkoxycarbonyl-C₁-C₄-alkyl, C₁-C₃-Alkylcarbonyl, CIC(O)-, NH₂C(S)-, OHC- oder Cyano;
R₄ Wasserstoff, Fluor, Chlor oder Brom;
R₅ Carboxyl, C₁-C₆-Alkoxycarbonyl, NH₂C(O)-, NH₂C(S)-, HON=CH-, OHC- oder Cyano;
R₆ Wasserstoff oder C₁-C₄-Alkyl;
n 0, 1 oder 2, mit der Massgabe, dass n 0 ist, wenn R₆ Wasserstoff bedeutet; und
R₇ Wasserstoff oder C₁-C₄-Alkyl ist,
sowie agronomisch verträgliche Salze und Stereoisomere dieser Verbindungen.

Die in den Substituentendefinitionen vorkommenden Alkylgruppen können geradkettig oder verzweigt sein, wobei dies auch für den Alkylteil der Halogenalkyl-, Alkyl-SO₂NHC(O)-, Alkyl-ON=CH-, Alkylaminocarbonyl-, Dialkylaminocarbonyl-, Cyanoalkyl-, Hydroxyalkyl- und Alkylcarbonyl-Gruppen gilt.
Alkylgruppen stehen beispielsweise für Methyl, Ethyl, n-Propyl, iso-Propyl, n-Butyl, sek.-Butyl, iso-Butyl, tert.-Butyl sowie die verschiedenen isomeren Pentyl- und Hexyl-Reste. Bevorzugt sind Methyl, Ethyl, n-Propyl, iso-Propyl und n-Butyl.

Unter Halogen ist Jod und vorzugsweise Fluor, Chlor und Brom zu verstehen.

Als Halogenalkyl kommen 1- oder mehrfach, insbesondere 1-, 2- oder 3-fach durch Halogen substituierte Alkylgruppen in Betracht, wobei Halogen im einzelnen Jod und insbesondere Fluor, Chlor und Brom bedeutet, beispielsweise Fluormethyl, Difluormethyl, Trifluormethyl, Chlormethyl, Dichlormethyl, Trichlormethyl, 2,2-Difluorethyl, 2-Fluorethyl, 2-Chlorethyl und 2,2-Dichlorethyl.

Die Alkenyl- und Alkinyl-Gruppen können geradkettig oder verzweigt sein, wobei dies auch für den Alkenyl- und Alkinyl-Teil der Alkenyloxy-alkyl-, Alkinyloxy-alkyl-, Alkenyloxycarbonyl- und Alkinyloxycarbonyl-Gruppen gilt.

Alkenyloxy-alkyl ist beispielsweise Allyloxy-alkyl, Methallyloxy-alkyl und But-2-en-1-yloxyalkyl.

Alkinyloxy-alkyl ist beispielsweise Propargyloxy-alkyl und 1-Methylpropargyloxy-alkyl.

Alkenyloxycarbonyl ist beispielsweise Allyloxycarbonyl, Methallyloxycarbonyl, But-2-en-1-yl-oxycarbonyl, Pentenyloxycarbonyl und 2-Hexenyloxycarbonyl.

Alkinyloxycarbonyl ist beispielsweise Propargyloxycarbonyl, 3-Butinyloxycarbonyl, But-2-in-1-yl-oxycarbonyl und 2-Methylbutin-2-yl-oxycarbonyl.

Alkylaminocarbonyl ist beispielsweise Methylaminocarbonyl, Ethylaminocarbonyl und die isomeren Propyl- und Butylaminocarbonyle.

Dialkylaminocarbonyl ist beispielsweise Dimethylaminocarbonyl, Diethylaminocarbonyl und die isomeren Dipropyl- und Dibutylaminocarbonyle.

Alkoxyalkoxycarbonyl ist beispielsweise Methoxymethoxycarbonyl, Ethoxymethoxycarbonyl, Ethoxyethoxycarbonyl, Propoxymethoxycarbonyl, Propoxyethoxycarbonyl, Propoxypropoxycarbonyl und Butoxyethoxycarbonyl.

Halogenalkoxy ist beispielsweise Fluormethoxy, Difluormethoxy, Trifluormethoxy, 2,2,2-Trifluorethoxy, 1,1,2,2-Tetrafluorethoxy, 2-Fluorethoxy, 2-Chlorethoxy und 2,2,2-Trichlorethoxy.

Bei den als Substituenten in Betracht kommenden Cycloalkoxycarbonyl-Resten handelt es sich beispielsweise um Cyclopropyloxycarbonyl, Cyclobutyloxycarbonyl, Cyclopentyloxycarbonyl und Cyclohexyloxycarbonyl.

Entsprechende Bedeutungen können auch den Substituenten in zusammengesetzten Definitionen, wie z.B. Halogenalkoxy, Alkoxycarbonyl-alkyl, Alkoxyalkoxycarbonyl, Alkylcarbonyloxy-alkyl, Alkoxycarbonyl, Alkoxyalkyl und Halogenalkoxycarbonyl zugeordnet werden.

Die Erfindung umfaßt ebenfalls die Salze, die die Verbindungen der Formel I mit azidem Wasserstoff, insbesondere die Derivate mit Carbonsäure- und Sulfonamidgruppen (z.B. Carboxyl- und Alkyl-SO₂NHC(O)-substituierte Benzofuranyl- und Dihydrobenzofuranyl-Pyrazolyl-Gruppen), mit Basen bilden können. Bei diesen Salzen handelt es sich beispielsweise um Alkalimetallsalze, wie z.B. Natrium- und Kaliumsalze; Erdalkalimetallsalze, wie z.B. Calcium- und Magnesiumsalze; Ammoniumsalze, d.h. unsubstituierte Ammoniumsalze und mono- oder mehrfach-substituierte Ammoniumsalze, wie z.B. Triethylammonium- und Methylammoniumsalze; oder um Salze mit anderen organischen Basen.

Unter den Alkali- und Erdalkalimetallhydroxiden als Salzbildner sind die Hydroxide von Lithium, Natrium, Kalium, Magnesium oder Calcium hervorzuheben, insbesondere aber die von Natrium und Kalium.

Als Beispiele für zur Ammoniumsalzbildung geeignete Amine kommen sowohl Ammoniak wie auch primäre, sekundäre und tertiäre C₁-C₁₈-Alkylamine, C₁-C₄-Hydroxyalkylamine und C₂-C₄-Alkoxyalkylamine in Betracht, beispielsweise Methylamin, Ethylamin, n-Propylamin, iso-Propylamin, die vier isomeren Butylamine, n-Amylamin, iso-Amylamin, Hexylamin, Heptylamin, Octylamin, Nonylamin, Decylamin, Pentadecylamin, Hexadecylamin, Heptadecylamin, Octadecylamin, Methyl-ethylamin, Methyl-iso-propylamin, Methylhexylamin, Methyl-nonylamin, Methyl-pentadecylamin, Methyl-octadecylamin, Ethylbutylamin, Ethyl-heptylamin, Ethyl-octylamin, Hexyl-heptylamin, Hexyl-octylamin, Dimethylamin, Diethylamin, Di-n-propylamin, Di-iso-propylamin, Di-n-butylamin, Di-n-amylamin, Di-iso-amylamin, Dihexylamin, Diheptylamin, Dioctylamin, Ethanolamin, n-Propanolamin, iso-Propanolamin, N,N-Diethanolamin, N-Ethylpropanolamin, N-Butylethanolamin, Allylamin, n-Butenyl-2-amin, n-Pentenyl-2-amin, 2,3-Dimethylbutenyl-2-amin, Di-butenyl-2-amin, n-Hexenyl-2-amin, Propylendiamin, Trimethylamin, Triethylamin, Tri-n-propylamin, Tri-iso-propylamin, Tri-n-butylamin, Tri-iso-butylamin, Tri-sek.-butylamin, Tri-n-amylamin, Methoxyethylamin und Ethoxyethylamin; heterocyclische Amine wie z.B. Pyridin, Chinolin, iso-Chinolin, Morpholin, Thiomorpholin, Piperidin, Pyrrolidin, Indolin, Chinuclidin und Azepin; primäre Arylamine wie z.B. Aniline, Methoxyaniline, Ethoxyaniline, o,m,p-Toluidine, Phenylendiamine, Benzidine, Naphthylamine und o,m,p-Chloraniline; insbesondere aber Triethylamin, iso-Propylamin und Di-iso-propylamin.

Bei den Salzen der Verbindungen der Formel I mit basischen Gruppen, insbesondere mit basischen Pyrazolylringen W1 oder W2, oder der Derivate mit Aminogruppen wie z.B. Anilin-Derivate für R₁ = Amino, handelt es sich beispielsweise um Salze mit anorganischen und organischen Säuren wie z.B. Halogenwasserstoffsäuren wie Fluorwasserstoffsäure, Chlorwasserstoffsäure, Bromwasserstoffsäure oder Jodwasserstoffsäure sowie Schwefelsäure, Phosphorsäure, Salpetersäure und organische Säuren wie Essigsäure, Trifluoressigsäure, Trichloressigsäure, Propionsäure, Glycolsäure, Thiocyansäure, Zitronensäure, Benzoesäure, Oxalsäure, Ameisensäure, Benzolsulfonsäure, p-Toluolsulfonsäure und Methansulfonsäure.

Das mögliche Vorhandensein mindestens eines asymmetrischen Kohlenstoffatoms in den Verbindungen der Formel I, z.B. im Dihydrobenzofuranylteil am 2-Kohlenstoffatom oder im Alkylteil des Substituenten R₃, hat zur Folge, dass die Verbindungen sowohl in optisch aktiven Einzelisomeren, als auch in Form von racemischen Gemischen auftreten können. In der vorliegenden Erfindung sind unter den Wirkstoffen der Formel I sowohl die reinen optischen Antipoden als auch die Racemate bzw. Diastereomeren zu verstehen.

Liegt eine aliphatische C=C- oder C=N-O-Doppelbindung (syn/anti) vor, so kann geometrische Isomerie auftreten. In der vorliegenden Erfindung sind auch diese Isomeren umfasst.

Bevorzugt sind Verbindungen der Formel I, worin R₁ Wasserstoff, Fluor, Chlor, Brom, Trifluormethyl oder Cyano ist.

Bevorzugt sind auch Verbindungen der Formel I, worin R Wasserstoff oder Fluor ist.

Insbesondere sind von diesen Verbindungen diejenigen bevorzugt, in denen R Fluor ist.

Bevorzugte Verbindungen der Formel I sind auch diejenigen, worin W eine Gruppe 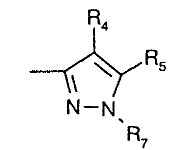 (W1) ist; und R₄, R₅ und R₇ die unter Formel I angegebene Bedeutung haben.

Insbesondere sind davon diejenigen besonders bevorzugt, worin R₇ Wasserstoff, Methyl oder Ethyl ist.

Ebenfalls besonders bevorzugte Verbindungen der Formel I sind diejenigen, worin R₅ Carboxyl, C₁-C₃-Alkoxycarbonyl, NH₂C(S)-, HON=CH-, OHC- oder Cyano ist.

Ganz besonders bevorzugte Verbindungen der Formel I sind hiervon diejenigen, worin R₅ Cyano ist.

Auch besonders bevorzugte Verbindungen der Formel I sind diejenigen, worin R₄ Wasserstoff oder Chlor ist.

Ebenfalls bevorzugt sind Verbindungen der Formel I, worin W eine Gruppe 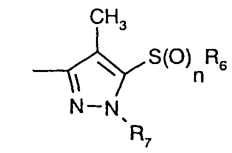 (W2) ist; und R₆, R₇ und n die unter Formel I angegebene Bedeutung haben.

Hiervon sind diejenigen Verbindungen besonders bevorzugt, in denen n 0 oder 2 ist.

Auch besonders bevorzugte Verbindungen der Formel I sind diejenigen, worin R₆ Methyl ist.

Ferner sind besonders bevorzugt Verbindungen der Formel I, worin R₇ Methyl oder Ethyl ist.

Insbesondere sind davon diejenigen ganz besonders bevorzugt, worin R₇ Methyl ist.

Wichtige Verbindungen der Formel I sind diejenigen, worin A-B eine Gruppe 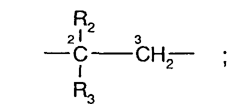 R₂ Wasserstoff oder Methyl; und R₃ Wasserstoff, C₁-C₃-Alkyl, C₁-oder C₂-Halogenalkyl, Cyano-C₁-C₄-alkyl, C₁-C₃-Alkylcarbonyloxy-C₁-oder-C₂-alkyl, Carboxyl, C₁-C₃-Alkoxycarbonyl, C₁-C₃-Alkyl-ON=CH-, C₁-C₃-Alkoxycarbonyl-C₁-oder-C₂-alkyl, C₁-C₃-Alkylcarbonyl oder OHC- ist.

Das erfindungsgemässe Verfahren zur Herstellung von Verbindungen der Formel I erfolgt in Analogie zu bekannten Verfahren und ist dadurch gekennzeichnet, dass man zwecks Herstellung derjenigen Verbindungen der Formel I, in denen A-B eine Gruppe 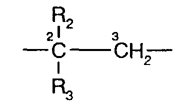 ist; R₂ die unter Formel I angegebene Bedeutung hat; und R₃ C₁-C₆-Alkyl ist, eine Verbindung der Formel II 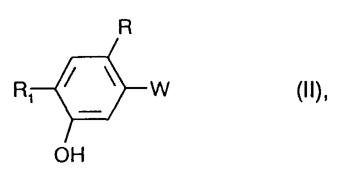 worin R, R₁ und W die unter Formel I angegebene Bedeutung haben, mit einer Verbindung der Formel III 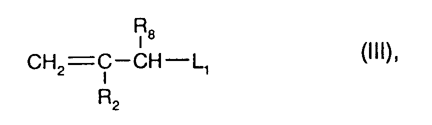 worin R₂ die angegebene Bedeutung hat; R₈ Wasserstoff oder C₁-C₅-Alkyl; und L₁ eine Abgangsgruppe wie z.B. Halogen, insbesondere Brom oder Chlor ist, gegebenenfalls in Gegenwart eines inerten organischen Lösungsmittels und einer Base zur Verbindung der Formel IVa 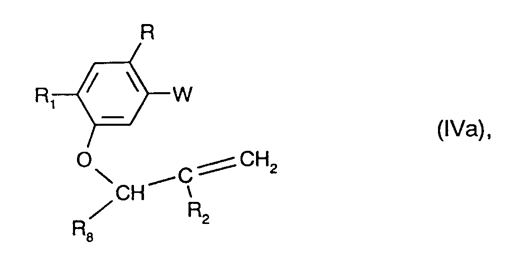 worin R, R₁, R₂, R₈ und W die angegebene Bedeutung haben, reagieren lässt, diese thermisch oder säurekatalytisch in die Verbindung der Formel Va 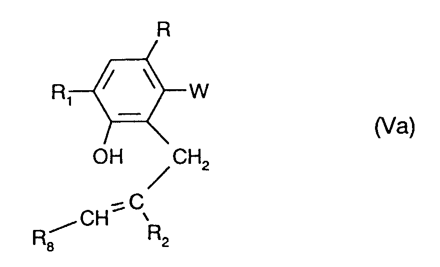 umlagert und anschliessend cyclisiert.

Ein anderes erfindungsgemässes Verfahren zur Herstellung von Verbindungen der Formel I erfolgt in Analogie zu bekannten Verfahren und ist dadurch gekennzeichnet, dass man zwecks Herstellung derjenigen Verbindungen der Formel I, in denen A-B eine Gruppe 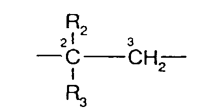 ist; R₂ die unter Formel I angegebene Bedeutung hat; und R₃ Hydroxy-C₁-C₆-Alkyl ist, eine Verbindung der Formel Va 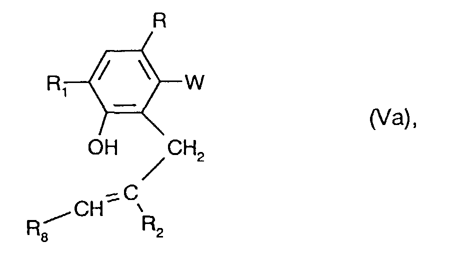 worin R, R₁, R₂ und W die unter Formel I angegebene Bedeutung haben; und R₈ Wasserstoff oder C₁-C₅-Alkyl ist, epoxidiert und anschliessend gegebenenfalls in Gegenwart eines Katalysators cyclisiert.

Das erfindungsgemässe Verfahren zur Herstellung von Verbindungen der Formel I erfolgt in Analogie zu bekannten Verfahren und ist dadurch gekennzeichnet, dass man zwecks Herstellung derjenigen Verbindungen der Formel I, in denen A-B eine Gruppe 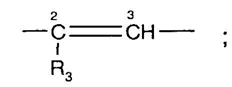 und R₃ C₁-C₆-Alkyl ist, eine Verbindung der Formel IVb 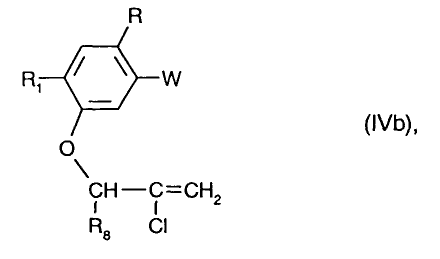 worin R, R₁ und W die unter Formel I angegebene Bedeutung haben; und R₈ Wasserstoff oder C₁-C₅-Alkyl ist, thermisch in die Verbindung der Formel Vb 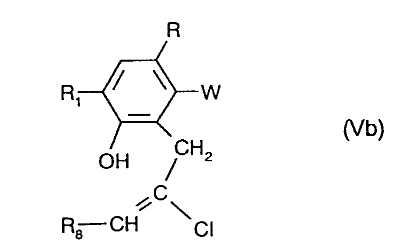 umlagert und anschliessend cyclisiert.

Das erfindungsgemässe Verfahren zur Herstellung von Verbindungen der Formel I erfolgt in Analogie zu bekannten Verfahren und ist dadurch gekennzeichnet, dass man zwecks Herstellung derjenigen Verbindungen der Formel I, in denen W eine Gruppe 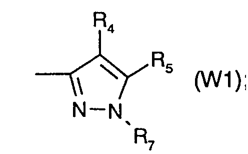 (W1); R₄ Wasserstoff, Fluor, Chlor oder Brom; R₅ C₁-C₆-Alkoxycarbonyl, NH₂C(O)- oder Cyano; und R₇ Wasserstoff oder C₁-C₄-Alkyl bedeutet, eine Verbindung der Formel VI 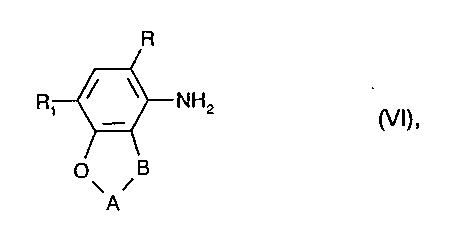 worin R, R₁ und A-B die unter Formel I angegebene Bedeutung besitzen, diazotiert und die Diazoniumgruppe in Gegenwart eines Kupfer(I)-Salzes durch ein Halogen ersetzt und die Verbindung der Formel Vll 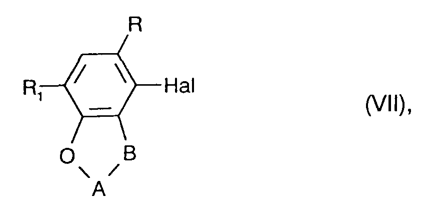 worin Hal Halogen bedeutet, erhält, diese in Gegenwart von Palladium(ll)-chlorid, Triphenylphosphin und n-Butylvinylether zur Verbindung der Formel VIII 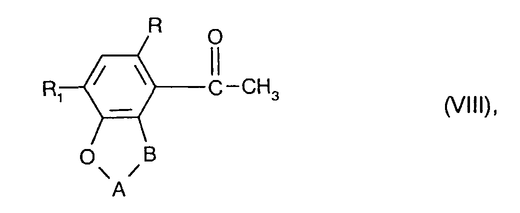 reagieren lässt, anschliessend mit einem Oxalsäuredialkylester der Formel IX 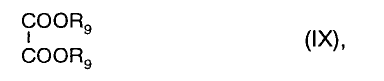 worin R₉ C₁-C₆-Alkyl ist, vorzugsweise Oxalsäuredimethylester, in Gegenwart einer Base, insbesondere das entsprechende Natriumalkoholat, vorzugsweise Natriummethylat in einem Lösungsmittel wie z.B. dem entsprechenden Alkohol, vorzugsweise Methanol zur Verbindung der Formel X 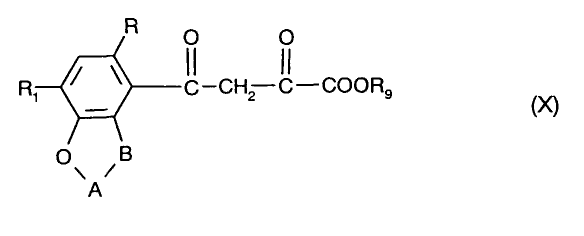 umsetzt, diese in Gegenwart von Hydrazin zur Verbindung der Formel la 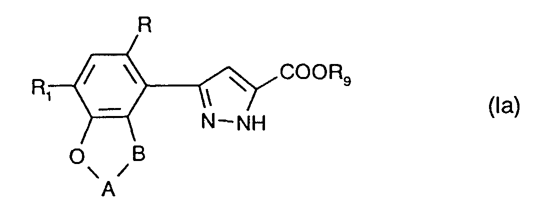 cyclisiert, anschliessend zuerst mit einem Alkylierungsreagens der Formel Xlla

R₇-L₂ (XIIa)

oder der Formel Xllb

R₇OSO₂OR₇ (XIIb),

wobei in den Verbindungen der Formeln Xlla und Xllb der Rest R₇ C₁-C₄-Alkyl und L₂ eine Abgangsgruppe, vorzugsweise Chlor, Brom, Jod, CH₃SO₂O- oder 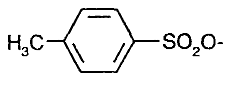 bedeutet, zur Verbindung der Formel Ib 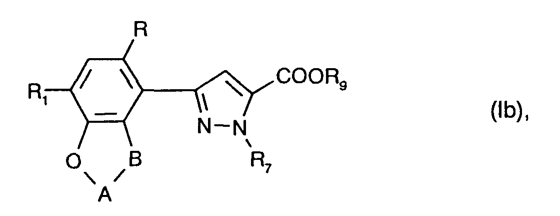 worin R, R₁, R₇, R₉ und A-B die angegebene Bedeutung haben, überführt, und nachfolgend einer Halogenierungsreaktion unterwirft und die Verbindung der Formel Ic 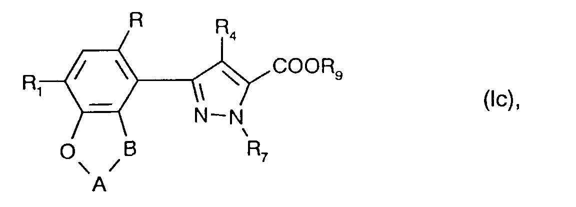 worin R, R₁, R₇, R₉ und A-B die angegebene Bedeutung haben; und R₄ Fluor, Chlor oder Brom bedeutet, erhält, und diese entweder direkt oder via die entsprechende Carbonsäure bzw. das entsprechende Carbonsäurehalogenid der Formel lc₁ 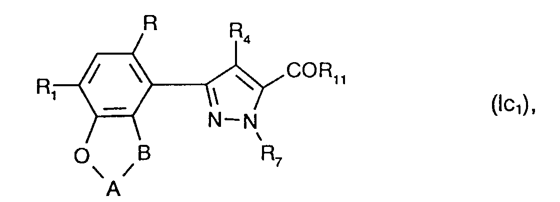 worin R, R₁, R₄, R₇ und A-B die angegebene Bedeutung haben; und R₁₁ Hydroxy oder Halogen, vorzugsweise Chlor bedeutet, mit Ammoniak zum Amid der Formel Id 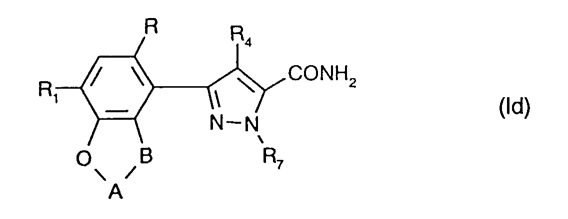 umsetzt und anschliessend dehydratisiert.

Das erfindungsgemässe Verfahren zur Herstellung von Verbindungen der Formel I erfolgt in Analogie zu bekannten Verfahren und ist dadurch gekennzeichnet, dass man zwecks Herstellung derjenigen Verbindungen der Formel I, in denen W eine Gruppe 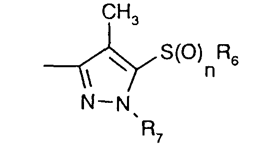 (W2) ist; und R₆, R₇ und n die unter Formel I angegebene Bedeutung haben, eine Verbindung der Formel Xl 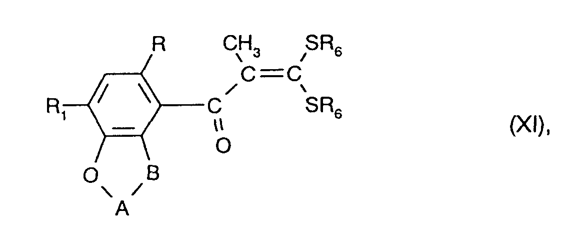 worin R, R₁, R₆ und A-B die unter Formel I angegebene Bedeutung besitzen,
a) mit Hydrazin gegebenenfalls in Gegenwart eines geeigneten Lösungsmittels zur Verbindung der Formel le 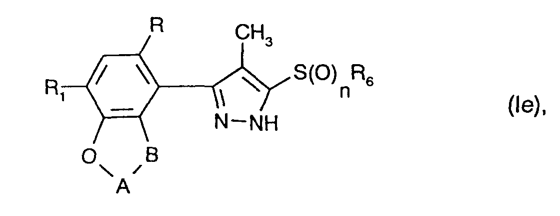 worin n 0 ist, cyclisiert und diese anschliessend mit einer Verbindung der Formel Xlla

   R₇-L₂ (XIIa)

   oder der Formel Xllb

   R₇OSO₂OR₇ (XIIb),

   wobei in den Verbindungen der Formeln Xlla und Xllb der Rest R₇ C₁-C₄-Alkyl und L₂ eine Abgangsgruppe, vorzugsweise Chlor, Brom, Jod, CH₃SO₂O- oder 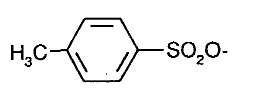 bedeutet, zur Verbindung der Formel If 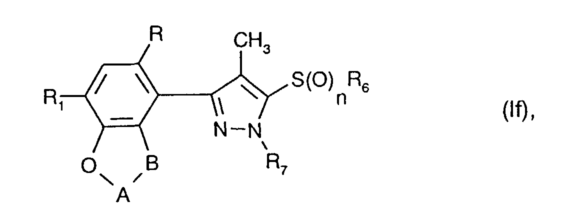 worin R, R₁, R₆, R₇ und A-B die angegebene Bedeutung haben; und n 0 ist, umsetzt und diese anschliessend oxidiert; oder
b) mit einer Verbindung der Formel XIII

   NH₂-NH-R₇ (XIII),

   worin R₇ die angegebene Bedeutung hat, gegebenenfalls in Gegenwart eines geeigneten Lösungsmittels zur Verbindung der Formel If cyclisiert und anschliessend oxidiert.

Ein weiteres erfindungsgemässes Verfahren zur Herstellung von Verbindungen der Formel I erfolgt in Analogie zu bekannten Verfahren und ist dadurch gekennzeichnet, dass man zwecks Herstellung derjenigen Verbindungen der Formel I, in denen W eine Gruppe 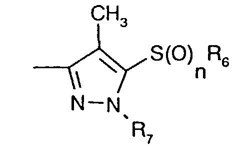 (W2); R₇ Wasserstoff ist; und R₆ und n die unter Formel I angegebene Bedeutung haben, eine Verbindung der Formel Vllla 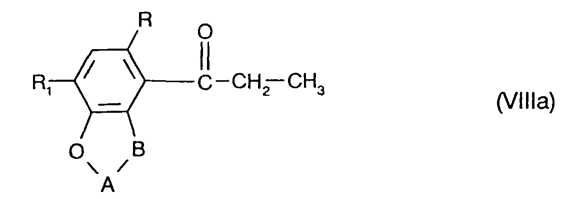 gegebenenfalls in Gegenwart eines Lösungsmittels und einer Base zur Verbindung der Formel XIV 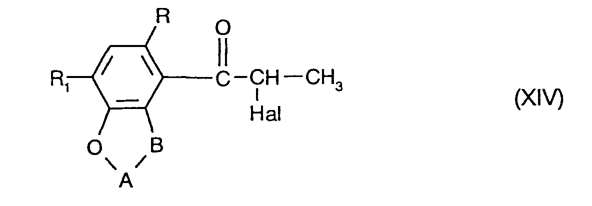 halogeniert, wobei in den Verbindungen der Formeln Vllla und XIV R, R₁ und A-B die unter Formel I angegebene Bedeutung haben, und Hal Halogen, vorzugsweise Chlor oder Brom ist, und diese mit der Verbindung der Formel XV

NH₂-NH-C(S)S-R₆ (XV),

worin R₆ die angegebene Bedeutung hat, gegebenenfalls in Gegenwart eines Lösungsmittels und einer Base zur Verbindung der Formel XVI 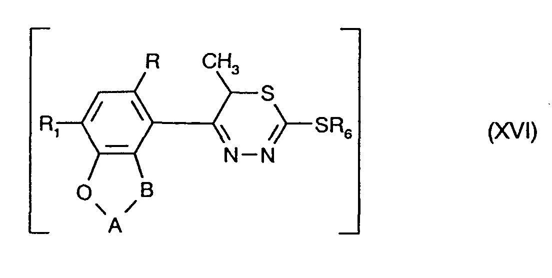 cyclisiert und diese thermisch oder säurekatalytisch einer Ringkontraktion unterwirft (n=0) und anschliessend oxidiert (n=1 bzw. 2).

Die Herstellung der Benzofuran- und Dihydrobenzofuranringe der Verbindungen der Formel I wird in den folgenden Reaktionsschemata 1, 2 und 3 näher erläutert.

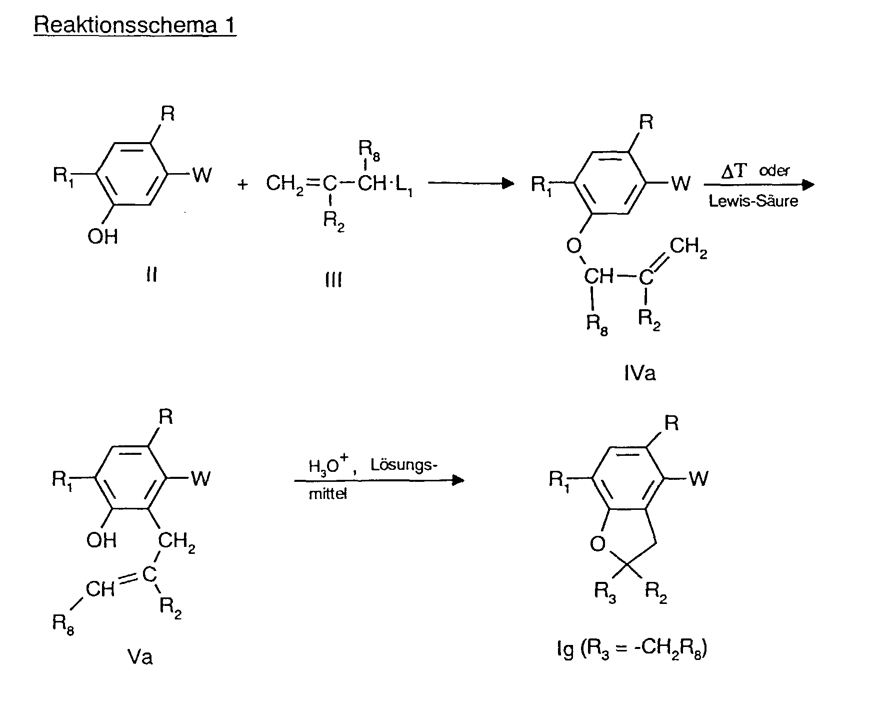

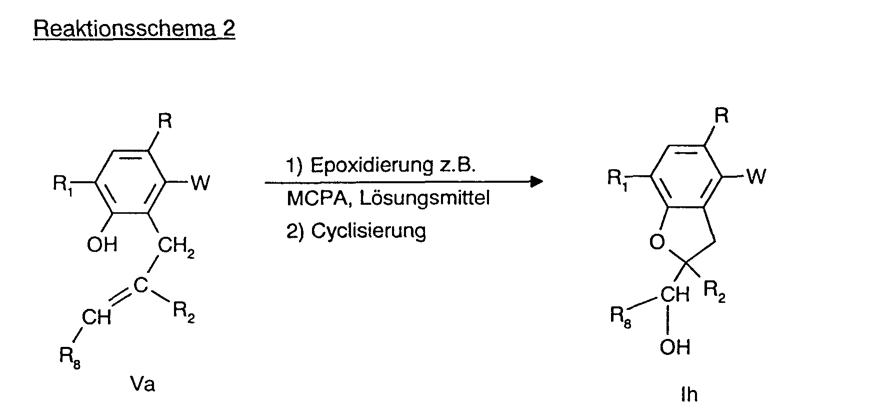

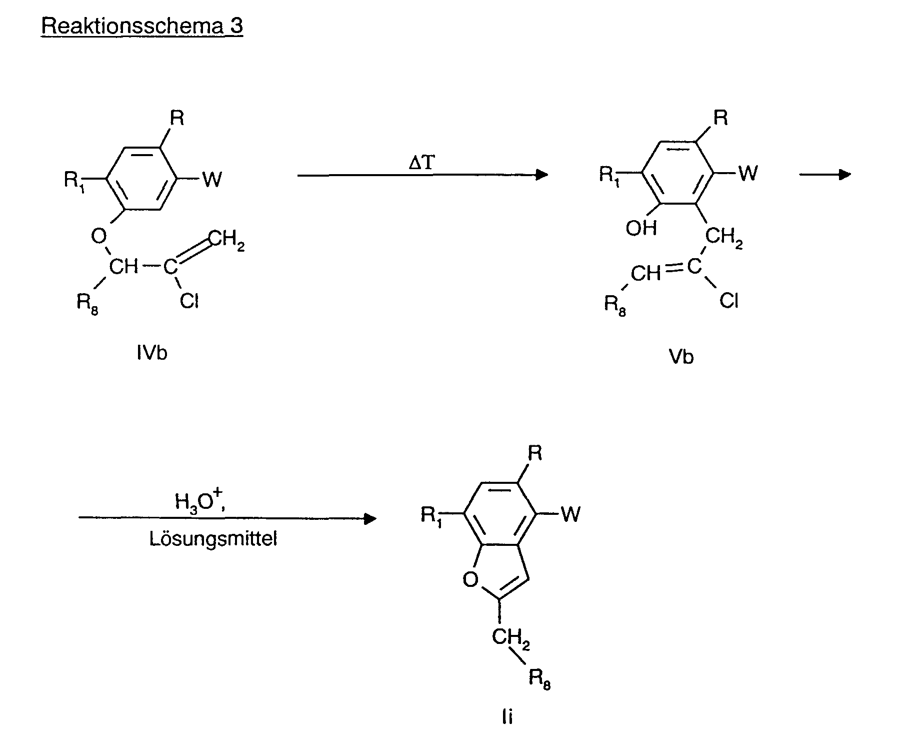

Die Herstellung der Pyrazolringe W1 und W2 der Verbindungen der Formel I wird in den folgenden Reaktionsschemata 4, 5 und 6 näher erläutert. 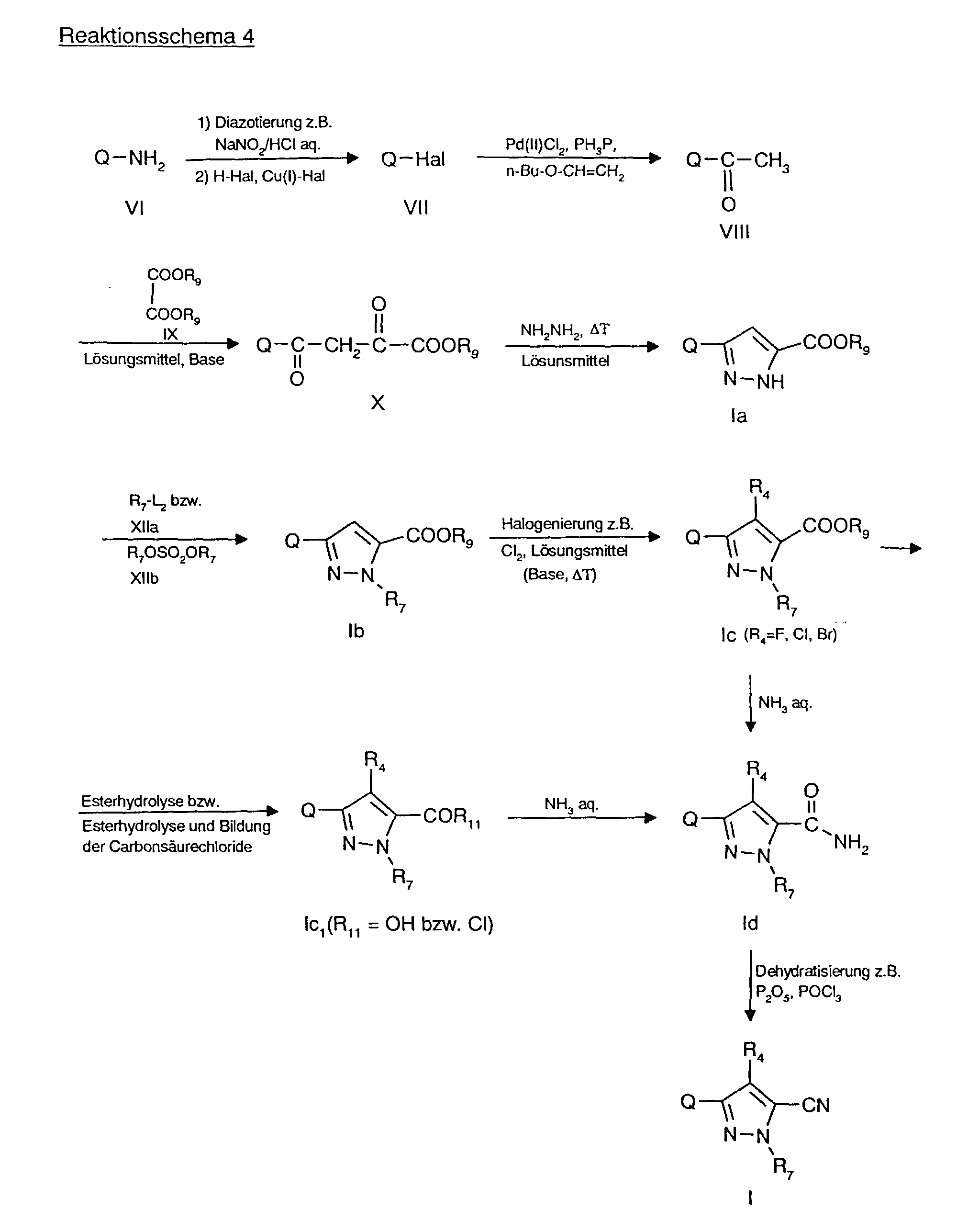 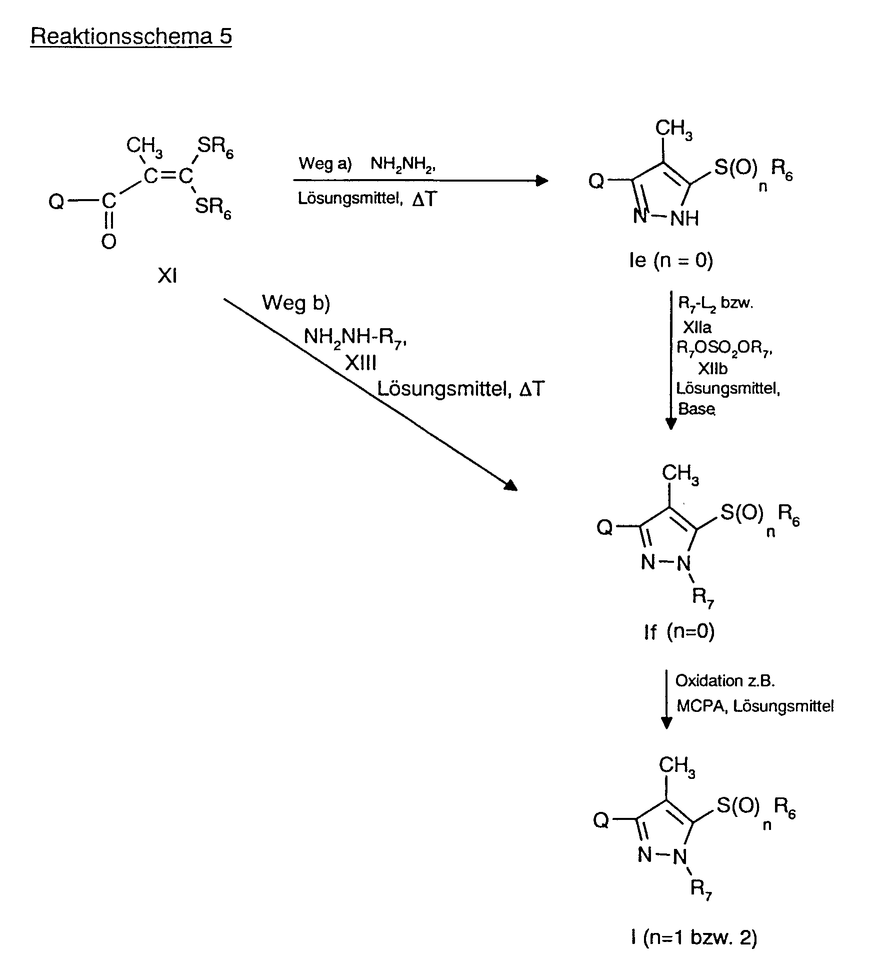 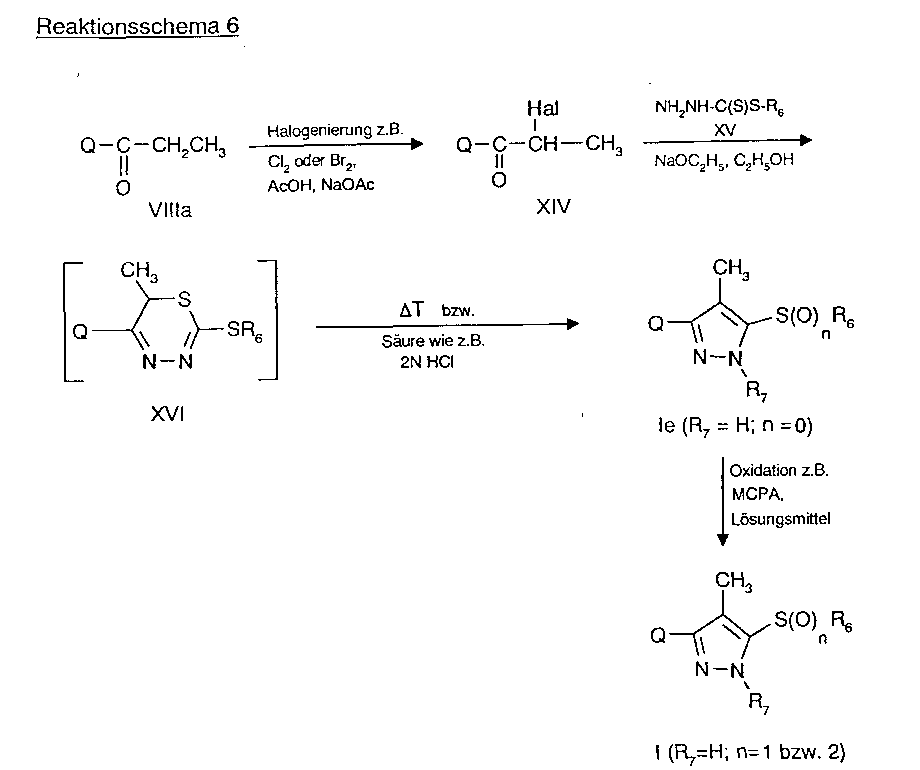 Q in Reaktionsschemata 4, 5 und 6 bedeutet die Gruppe

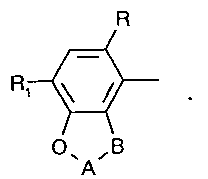

Die Allylether der Formel IVa können gemäss Reaktionsschema 1 z.B. in Analogie zu EP-A-0 617 033 (Seite 3, Zeilen 45 und 46) oder US-A-4 881 967 (Kolonne 11, Zeilen 17-39) mittels Umsetzung der Verbindungen der Formel II mit einem Allylderivat der Formel III, wobei L₁ eine Abgangsgruppe wie z.B. Halogen, insbesondere Chlor oder Brom bedeutet, gegebenenfalls in einem inerten organischen Lösungsmittel wie z.B. Aceton, Acetonitril oder N,N-Dimethylformamid in Gegenwart einer Base wie z.B. Kaliumcarbonat erhalten werden.

Die allylierten Phenolderivate der Formel Va werden durch thermische Umlagerung aus den entsprechenden Allylethern der Formel IVa erhalten. Diese Umlagerung (Claisen-Umlagerung) erfolgt z.B. in Analogie zu EP-A-0 617 033 (Seite 3, Zeilen 17-44) oder US-A-4 881 967 (Kolonne 10, Zeile 30 bis Ende Kolonne 10) gegebenenfalls in einem Lösungsmittel wie z.B. Toluol, Xylole, Mesitylen oder Tetralin sowie tertiäre Amine beispielsweise N,N-Diethylanilin oder Gemische davon bei Temperaturen von 20°C bis 300°C, vorzugsweise bei 100°C bis 250°C während 0,5 bis 48 Stunden. Gegebenenfalls kann die Umlagerungsreaktion in einem geschlossenen Druckgefäss durchgeführt werden.

Alternativ kann diese Umlagerung auch in Gegenwart eines Lewissäure-Katalysators wie z.B. Bortrichlorid in einem inerten Lösungsmittel wie z.B. Dichlormethan bei Temperaturen von 0°C bis 25°C durchgeführt werden z.B. in Analogie zu US-A-4 881 967 (Kolonne 10, Zeile 66 bis Ende Kolonne 10, und Kolonne 11, Zeilen 1-7).

Die nachfolgende Cyclisierungsreaktion der Verbindungen der Formel Va kann nach einer oder mehreren Methoden wie z.B. in US-A-4 881 967 (Kolonne 8, Zeilen 56 bis Ende Kolonne 8, und Kolonne 9, Zeilen 1-3) beschrieben, insbesondere aber säurekatalytisch in einem inerten organischen Lösungsmittel wie z.B. Xylole in Gegenwart von Säuren wie z.B. p-Toluolsulfonsäure durchgeführt werden.

Die Herstellung der Verbindungen der Formel I, worin R₃ Hydroxy-C₁-C₆-alkyl (R₃= = -CH(OH)-R₈) ist, erfolgt gemäss Reaktionsschema 2 durch Epoxidierung beispielsweise mit m-Chlorperbenzoesäure (MCPA) der Verbindung der Formel Va in Gegenwart eines organischen Lösungsmittels und nachfolgende Cyclisierung in Analogie z.B. zu EP-A-0 617 033 (Seite 3, letzter Abschnitt, und Seite 4, Zeilen 1-50).

Die Allylether der Formel IVb in Reaktionsschema 3 können z.B. in Analogie zu EP-A-0 561 319 aus den entsprechenden Phenolen der Formel II und den Allylderivaten der Formel III (Reaktionsschema 1; R₂ = Chlor) erhalten werden.

Durch Erhitzen der Allylether der Formel IVb können die Phenole der Formel Vb, analog wie unter Reaktionsschema 1 beschrieben, erhalten werden. Diese thermische Umlagerung erfolgt bei Temperaturen von 150°C bis 250°C während 2 bis 100 Stunden gegebenenfalls in einem inerten organischen Lösungsmittel.

Die anschliessende Cyclisierung der Phenole der Formel Vb erfolgt zweckmässigerweise in Gegenwart einer Säure, beispielsweise Mineralsäuren wie z.B. Salzsäure, Schwefelsäure oder Polyphosphorsäure, organische Säuren wie z.B. p-Toluolsulfonsäure oder Trifluormethansulfonsäure sowie Carbonsäuren wie z.B. Ameisensäure, Essigsäure oder Trifluoressigsäure. Die Menge verwendeter Säure zu Phenole der Formel Vb beträgt 1,1:1 bis 100:1.
Diese Cyclisierungsreaktion erfolgt gegebenenfalls in einem Lösungsmittel beispielsweise aromatische Kohlenwasserstoffe wie z.B. Benzol oder Toluol, halogenierte Kohlenwasserstoffe wie z.B. Chloroform oder Tetrachlorkohlenstoff, Mineralsäuren wie z.B. Salzsäure oder Schwefelsäure, organische Säuren wie z.B. Essigsäure, und Wasser. Diese Lösungsmittel können auch im Gemisch eingesetzt werden.
Diese Cyclisierung gelingt bei Temperaturen von 0°C bis 100°C, vorzugsweise von 5°C bis 80°C während 0,5 bis 24 Stunden.

Alle weiteren Funktionalisierungen des Substituenten R₃ (bzw. -CH₂R₈ oder -CH(OH)-R₈) in 2-Position des Benzofuranyl- bzw. Dihydrobenzofuranyl-Inkrements zu den Verbindungen der Formel I können ausgehend von den Verbindungen der Formeln li bzw. lg und Ih in Reaktionsschemata 1, 2 und 3 auf analoge Weise wie beispielsweise in EP-A-0 617 033 (Seite 3, letzter Abschnitt, bis Seite 8), EP-A-0 561 319 (Seite 3, letzter Abschnitt, bis Seite 10) oder US-A-4 881 967 (Kolonnen 13 und 14) beschrieben, erfolgen.

Für die Herstellung der Pyrazolringe der Verbindungen der Formel I, worin W eine Gruppe 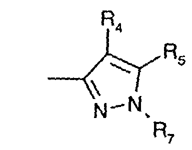 (W1) ist, wird gemäss Reaktionsschema 4 von den Anilinderivaten der Formel VI ausgegangen. Aus diesen lassen sich die 4-Halogenbenzofuran- und -dihydrobenzofuran-Derivate, insbesondere die 4-Brombenzofuran- und -dihydrobenzofuran-Derivate der Formel Vll, nach Standardmethoden durch Diazotierung beispielsweise mit Natriumnitrit in wässriger Salzsäure und Umsetzung des erhaltenen Diazoniumsalzes mit Halogenwasserstoffsäure wie z.B. Bromwasserstoffsäure in Gegenwart eines Kupfer(I)-Salzes wie z.B. Kupferbromid (Sandmeyer-Reaktion) erhalten.

Die weitere Umsetzung der halogenierten Benzofuran- bzw. Dihydrobenzofuran-Derivate der Formel Vll in Gegenwart von Palladium(11)-chlorid, Triphenylphosphin und n-Butylvinylether erfolgt z.B. in Analogie zu Indian J. Chem. B 31, 363 (1992) und ergibt die Acetophenon-Derivate der Formel VIII.

Diese werden anschliessend mit einem Oxalsäuredialkylester, vorzugsweise Oxalsäuredimethylester in Gegenwart einer Base, insbesondere das entsprechende Natriumalkoholat, vorzugsweise Natriummethylat in einem Lösungsmittel wie z.B. dem entsprechenden Alkohol, vorzugsweise Methanol zusammen mit einem Zweitlösungsmittel wie z.B. einem Ether oder Kohlenwasserstoff bei Temperaturen von 0°C bis zur Siedetemperatur des jeweiligen Lösungsmittels reagieren gelassen.
Diese Kondensationsreaktion sowie alle nachfolgenden Reaktionsschritte bis zu den 5-Nitrilpyrazolderivaten der Formel I gemäss Reaktionsschema 4 können analog wie z.B. in WO 96/01254 (Seite 20 ff.) beschrieben, erfolgen.

Demnach werden die Diketoester der Formel X mit Hydrazin bei erhöhter Temperatur (Rückfluss) vorzugsweise in Eisessig, Toluol oder einem Alkohol als Lösungsmittel in die Verbindungen der Formel la cyclisiert.
Gegebenenfalls kann als Katalysator eine Säure wie z.B. Schwefelsäure oder p-Toluolsulfonsäure eingesetzt werden.

Die N-Alkylierung der Pyrazole der Formel la erfolgt z.B. analog wie in WO 96/01254, Seiten 20 und 34 ff., beschrieben bei 22°C oder leicht erhöhten Temperaturen in Gegenwart eines Lösungsmittels wie z.B. Aceton, Methylethylketon, N,N-Dimethylformamid, N-Methylpyrrolidon oder Dimethylsulfoxid, einer Base wie z.B. Natrium- oder Kaliumcarbonat, Natrium- oder Kaliumhydroxid, und eines Alkylierungsmittels der Formel Xlla oder Xllb, vorzugsweise Methyljodid oder Dimethylsulfat.

Die nachfolgende Halogenierung in 4-Position des Pyrazolrings (R₄) gemäss Reaktionsschema 4 erfolgt z.B. in Analogie zu WO 96/01254, Seiten 23 und 37 ff., mittels eines Dihalogenmolekül, vorzugsweise Cl₂, Br₂, J₂, F-J oder Cl-J, wobei mit den beiden letzten Reagenzien vorwiegend das Jodidderivat gebildet wird, in einem geeigneten Lösungsmittel, vorzugsweise Eisessig oder Tetrachlorkohlenstoff bei Temperaturen von 10°C bis Rückflusstemperatur des jeweiligen Reaktionsgemisches. In gewissen Fällen ist es vorteilhaft, in Gegenwart einer Base wie z.B. Natriumacetat zu halogenieren, wobei die Basenzugabe sowohl vor wie auch im Verlaufe der Halogenierung erfolgen kann. Zur Beschleunigung der Halogenierung kann gegebenenfalls ein Katalysator wie z.B. Aluminiumchlorid, Eisen(ll)-chlorid oder Eisenpulver zum Reaktionsgemisch zugegeben werden.

Die nachfolgende Ueberführung der Esterderivate der Formel lc in die entsprechenden Amide der Formel Id gemäss Reaktionsschema 4 kann beispielsweise entweder direkt durch Erhitzen der Esterderivate in wässrigem Ammoniak erfolgen oder alternativ via Hydrolyse der Esterderivate der Formel Ic zu den entsprechenden Carbonsäurederivaten der Formel lc₁ (R₁₁ = OH) und nachfolgendes Erhitzen der erhaltenen Carbonsäurederivate in wässrigem Ammoniak oder via Ueberführung der Carbonsäurederivate der Formel lc₁ (R₁₁ = OH) in die entsprechenden Carbonsäurehalogenide der Formel lc₁ (R₁₁ = Halogen, insbesondere Chlor) und nachfolgendes Erhitzen der erhaltenen Carbonsäurehalogenide in wässrigem Ammoniak.

Die gewünschten 5-Nitrilpyrazol-Derivate der Formel I können mittels Dehydratisierung der auf obige Weise gebildeten Amide der Formel Id z.B. in Analogie zu WO 96/01254, Seiten 23 und 41 ff. und 'Advanced Organic Chemistry', Editor J. March, Mc Graw-Hill Book Company, N.Y., 1985, Seite 932 ff., erhalten werden

Die Herstellung des Pyrazolrings der Verbindungen der Formel I, worin W eine Gruppe 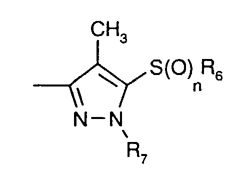 (W2) ist (Reaktionsschema 5, Weg a)) erfolgt durch Umsetzung der Verbindungen der Formel Xl mit Hydrazin oder Hydrazinhydrat gegebenenfalls in Gegenwart eines geeigneten Lösungsmittels bei erhöhter Temperatur, vorzugsweise mit Hydrazinhydrat in alkoholischer Lösung bei erhöhter Temperatur (R₇ = Wasserstoff).

Für die Herstellung der am Stickstoffatom substituierten Pyrazolringe (R₇ = C₁-C₄-Alkyl; Reaktionsschema 5, Weg b)), wird analog wie unter Reaktionsschema 5, Weg a) angegeben, vorgegangen, wobei als Reagens die Verbindung der Formel XIII, wie z.B. N-Alkylhydrazin, bevorzugt N-Methylhydrazin eingesetzt wird.

Im Reaktionsschema 5, Weg b) bedeutet der Rest R₇ im Hydrazinderivat der Formel XIII bzw. in den Alkylierungsmitteln der Formeln Xlla und Xllb (Reaktionsschema 5, Weg a)) C₁-C₄-Alkyl, und L₂ bedeutet eine Abgangsgruppe wie z.B. Chlor, Brom, Jod, CH₃SO₂O- oder

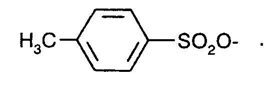

Die N-Alkylierung der Pyrazolringe in den Verbindungen der Formel le in Reaktionsschema 5 erfolgt bei Raumtemperatur oder leicht erhöhten Temperaturen in Gegenwart eines Lösungsmittels wie z.B. Aceton, Methylethylketon, N,N-Dimethylformamid, N-Methylpyrrolidon oder Dimethylsulfoxid, einer Base, wie z.B. Kaliumcarbonat, Natriumcarbonat, Natrium- oder Kaliumhydroxid, und einem Alkylierungsmittel der Formel Xlla oder Xllb, vorzugsweise Methyljodid oder Dimethylsulfat.

Die Herstellung der an den Stickstoffatomen unsubstituierten Pyrazol-Ringe der Formeln I und le (Reaktionsschema 6) kann z.B. auch durch Halogenierung der Verbindungen der Formel Vllla vorzugsweise mit Chlor oder Brom gegebenenfalls in Gegenwart eines geeigneten Lösungsmittels und einer Base wie z.B. Essigsäure und Natriumacetat, nachfolgende Cyclisierung mit einer Verbindung der Formel XV gegebenenfalls in einem Lösungsmittel wie z.B. einem Alkohol, vorzugsweise Ethanol und in Gegenwart einer Base wie z.B. ein Alkoholat, vorzugsweise Ethanolat, und durch Ringkontraktion (Extrusionsreaktion) in Analogie zu bekannten Verfahren wie z.B. in Chem. Ber. 92, 2593 (1959) oder Acta Chem. Scand. 16, 2395 (1962) beschrieben, erfolgen. Diese in Reaktionsschema 6 beschriebene Methode eignet sich für die Herstellung von am Phenylring Halogen-substituierten, insbesondere Fluor- und Chlor-substituierten Derivaten der Formel le bzw. I.

Die Wahl der geeigneten Herstellungsmethode und der entsprechenden Reaktionsbedingungen richtet sich dabei nach den Eigenschaften (Reaktivitäten) der Substituenten in den jeweiligen Zwischenprodukten.

Die nachfolgende Oxidation der Verbindungen der Formeln le und If, worin n 0 ist (Reaktionsschemata 5 und 6), erfolgt z.B. mit Persäuren wie z.B. m-Chlorperbenzoesäure (MCPA) oder Wasserstoffperoxid in Gegenwart eines geeigneten Lösungsmittels wie z.B. Dichlormethan, Chloroform oder Tetrachlorkohlenstoff bei Temperaturen von -40°C bis zur Rückflusstemperatur des jeweiligen Lösungsmittels, vorzugsweise von 0°C bis 35°C. Dabei kann der Grad der Oxidation am Schwefelatom durch die Menge Oxidationsmittel kontrolliert werden: bei aequimolarer Menge Oxidationsmittel werden Verbindungen der Formel I, worin n 1, und bei einem Ueberschuss (mindestens 2 Mol) Oxidationsmittel werden Verbindungen der Formel I, worin n 2 ist, erhalten.

Die Ausgangsverbindung der Formel Xl in Reaktionsschema 5 kann in Analogie zu bekannten Verfahren z.B. gemäss der im nachfolgenden Reaktionsschema 7 aufgeführten Methode hergestellt werden.

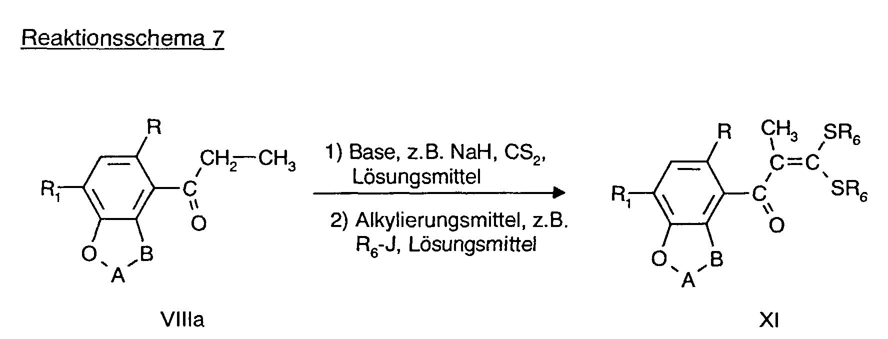

Die Umsetzung in Reaktionsschema 7 erfolgt z.B. analog zu WO 92/02509. Demnach wird das Ketonderivat der Formel Vllla in Gegenwart einer Base wie z.B. Natriumhydrid oder Kalium-tert.-butylat und eines aprotischen Lösungsmittels, wie z.B. Tetrahydrofuran, mit Schwefelkohlenstoff bei Temperaturen von 0°C bis 80°C reagieren gelassen und gleich anschliessend ein Alkylierungsmittel wie z.B. R₆-Hal oder R₆OSO₂OR₆, wobei R₆ die unter Formel I angegebene Bedeutung hat und Hal Halogen, insbesondere Chlor, Brom oder Jod bedeutet, bei Temperaturen von 0°C bis Rückflusstemperatur des verwendeten Lösungsmittels, zugegeben.

Die Verbindungen der Formel XV in Reaktionsschema 6 können nach bekannten Methoden (z.B. Chem. Ber. 92, 2593 (1959) oder Acta Chem. Scand. 16, 2395 (1962)) hergestellt werden, z.B. durch Umsetzung von Hydrazin bzw. Hydrazinhydrat mit Schwefelkohlenstoff und anschliessender Alkylierung mit dem Reagens R₆-Hal oder R₆OSO₂OR₆, worin R₆ die unter Formel I angegebene Bedeutung hat und Hal Halogen, insbesondere Chlor oder Brom bedeutet, in Gegenwart einer Base. Als Lösungsmittel eignen sich beispielsweise Alkohole wie z.B. Ethanol, und als Basen Alkoholate wie z.B. Natriummethylat oder Natriumethylat, oder Kalium- oder Natriumhydroxid.

Die Ausgangsverbindungen der Formel Vllla in Reaktionsschema 7 können in Analogie zu bekannten Verfahren, z.B. gemäss der im nachfolgenden Reaktionsschema 8 aufgeführten Methode hergestellt werden.

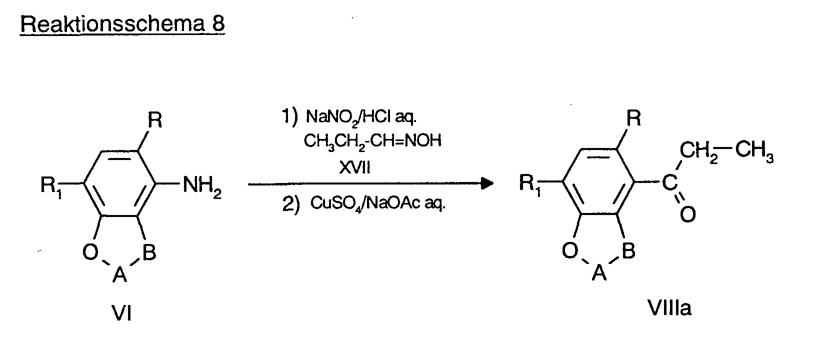

Im Reaktionsschema 8 sind die Reste R, R₁ und A-B wie unter Formel I angegeben, wobei zu beachten ist, dass nicht jede Substituentendefinition mit dem aufgeführten Verfahren verträglich ist. Die Wahl der geeigneten Herstellungsmethode richtet sich nach den Eigenschaften (Reaktivitäten) der Substituenten in den jeweiligen Zwischenprodukten.

Die Umsetzung nach Reaktionsschema 8 erfolgt analog zu J. Chem. Soc. 1954, 1297. Die Amine der Formel Vl werden demnach zuerst zu den entsprechenden Diazoniumsalzen diazotiert und mit dem Propionaldehydoxim der Formel XVII reagieren gelassen. Anschliessende Hydrolyse z.B. mit wässrigem Natriumacetat und Kupfersulfat liefert das entsprechende Ketonderivat der Formel Vllla.

Weitere Herstellungsmethoden für die Ausgangsverbindungen der Formel Vllla können ausgehend vom entsprechenden Benzofuran- bzw. Dihydrobenzofuranderivat via Lewis-Säure katalysierte Acylierung z.B. analog zu 'Vogel's Textbook of Practical Organic Chemistry', Longman 1989, Seite 1006 ff.;
bzw. ausgehend vom entsprechenden Benzofuran- bzw. Dihydrobenzofurancarbonsäurederivat via Umsetzung mit Ethyllithium oder Ethylmagnesium-chlorid oder -bromid z.B. analog zu Organic Reactions 18, 1 (1970); Organic Synthesis 49, 81 (1969); und 'Comprehensive Organic Transformations', Editor R. C. Larock, VCH 1989, Seite 685, bzw. ausgehend vom entsprechenden Benzofuran- bzw. Dihydrobenzofuranaldehyd via Umsetzung mit Ethylmagnesium-chlorid oder -bromid z.B. analog zu 'Advanced Organic Chemistry', Editor J. March, McGraw-Hill Book Company, New York, 1985, Seite 816 ff. und 1057 ff., erfolgen.

Die Ausgangsphenole der Formel II (Reaktionsschema 1) können z.B. wie in Reaktionsschema 9 gezeigt aus den entsprechenden Methoxy- bzw. Benzyloxysubstituierten Derivaten der Formel II₁ bzw. II₂, worin R, R₁ und W die unter Formel I angegebene Bedeutung besitzen, erhalten werden.

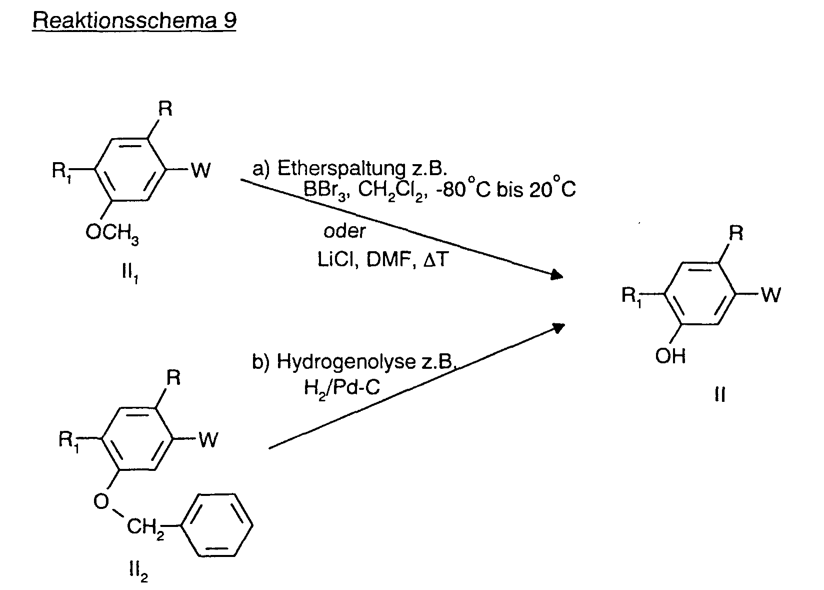

Demnach werden gemäss Weg a) die Verbindungen der Formel II₁ einer Etherspaltung mittels Lithiumchlorid in N,N-Dimethylformamid (DMF) bei erhöhter Temperatur wie beispielsweise in Synthesis 1989, 287, oder mittels Bortribromid in Dichlormethan bei Temperaturen von -80°C bis 20°C wie beispielsweise in Org. Synth., Collect. Vol. V, 412, 1973, beschrieben, oder gemäss Weg b) die Verbindungen der Formel II₂ einer Hydrogenolyse mittels Wasserstoff in Gegenwart eines Katalysators wie z.B. Palladium auf Kohlenstoff wie beispielsweise in J. Am. Chem. Soc. 93, 746 (1971) beschrieben, unterworfen.

Die Verbindungen der Formeln II₁ und II₂ in Reaktionsschema 9 können nach Standardmethoden wie z.B. in US-A-4 452 981 und EP-A-0 061 741 beschrieben aus den bekannten Phenolen der Formel II₃ 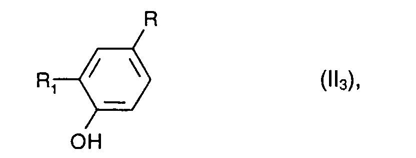 worin R und R₁ die unter Formel I angegebene Bedeutung haben, mittels Nitrierung des Benzolrings und Methylierung bzw. Benzylierung der Phenolfunktion und anschliessende Reduktion der Nitrogruppe zum entsprechenden Anilinderivat der Formel II₄ 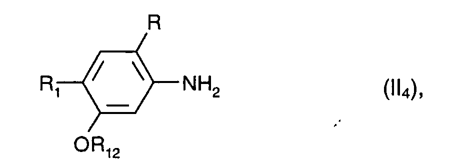 worin R und R₁ die angegebene Bedeutung haben und R₁₂ Methyl oder Benzyl bedeutet, und nachfolgendem Aufbau der Pyrazolringe W1 und W2 wie oben beschrieben, hergestellt werden.

Die Ausgangsverbindungen der Formeln III, Vl, IX, Xlla, Xllb, Xlll und XVII in den Reaktionsschemata 1, 4, 5 und 8 sind bekannt oder können nach offenbarten Verfahren hergestellt werden.

Die Zwischenprodukte der Formeln Va, Vb, VIII und X sind neu, wobei die Verbindung 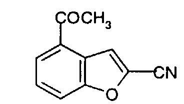 bei den Zwischenprodukten der Formel VIII ausgeschlossen ist . Sie stellen wichtige Zwischenprodukte für die Synthese der Verbindungen der Formel I dar. Die Erfindung betrifft somit auch diese Verbindungen.
Für die Zwischenprodukte der Formeln Va, Vb, VIII und X gelten die gleichen Bevorzugungen wie für die Verbindungen der Formel I.

Für die Herstellung aller weiteren in 2-Position des Benzofuranyl- bzw. Dihydrobenzofuranylrings substituierten Verbindungen der Formel I (R₃) bietet sich eine Vielzahl von bekannten Standardverfahren wie z.B. Alkylierung, Halogenierung, Acylierung, Amidierung, Oximierung, Oxidation und Reduktion an, wobei die Wahl der geeigneten Herstellungsverfahren sich nach den Eigenschaften (Reaktivitäten) der Substituenten in den jeweiligen Zwischenprodukten richtet.

Die Endprodukte der Formel I können auf übliche Weise durch Einengen oder Verdampfen des Lösungsmittels isoliert und durch Umkristallisieren oder Zerreiben des festen Rückstandes in Lösungsmitteln, in denen sie sich nicht gut lösen, wie Ether, aromatischen Kohlenwasserstoffe oder chlorierten Kohlenwasserstoffe, durch Destillation oder mittels Säulenchromatographie und einem geeigneten Elutionsmittel, gereinigt werden.

Ferner ist dem Fachmann geläufig, in welcher Reihenfolge gewisse Umsetzungen z.B. in den Reaktionsschemata 1, 4, 5 und 6 zweckmässig durchzuführen sind, um möglicherweise Nebenreaktionen zu vermeiden.

Sofern keine gezielte Synthese zur Isolierung reiner Isomerer durchgeführt wird, kann das Produkt als Gemisch zweier oder mehrerer Isomerer anfallen. Die Isomeren können nach an sich bekannten Methoden aufgetrennt werden.

Für die erfindungsgemäße Verwendung der Verbindungen der Formel I oder diese enthaltende Mittel kommen alle in der Landwirtschaft üblichen Applikationsmethoden wie z.B. preemergente Applikation, postemergente Applikation und Saatbeizung, sowie verschiedene Methoden und Techniken in Betracht, wie beispielsweise die kontrollierte Wirkstoffabgabe. Dazu wird der Wirkstoff in Lösung auf mineralische Granulatträger oder polymerisierte Granulate (Harnstoff/Formaldehyd) aufgezogen und getrocknet. Gegebenenfalls kann zusätzlich ein Überzug aufgebracht werden (Umhüllungsgranulate), der es erlaubt, den Wirkstoff über einen bestimmten Zeitraum dosiert abzugeben.

Die Verbindungen der Formel I können in unveränderter Form, d.h. wie sie in der Synthese anfallen, eingesetzt werden, vorzugsweise verarbeitet man sie aber auf übliche Weise mit den in der Formulierungstechnik gebräuchlichen Hilfsmitteln z.B. zu emulgierbaren Konzentraten, direkt versprühbaren oder verdünnbaren Lösungen, verdünnten Emulsionen, Spritzpulvern, löslichen Pulvern, Stäubemitteln, Granulaten oder Mikrokapseln. Die Anwendungsverfahren wie Versprühen, Vernebeln, Verstäuben, Benetzen, Verstreuen oder Gießen werden gleich wie die Art der Mittel, den angestrebten Zielen und den gegebenen Verhältnissen entsprechend gewählt.

Die Formulierungen, d.h. die den Wirkstoff der Formel I bzw. mindestens einen Wirkstoff der Formel I und in der Regel einen oder mehrere feste oder flüssige Formulierungshilfsmittel enthaltenden Mittel, Zubereitungen oder Zusammensetzungen werden in bekannter Weise hergestellt, z.B. durch inniges Vermischen und/oder Vermahlen der Wirkstoffe mit den Formulierungshilfsmitteln wie z.B. Lösungsmittel oder festen Trägerstoffe. Ferner können zusätzlich oberflächenaktive Verbindungen (Tenside) bei der Herstellung der Formulierungen verwendet werden.

Als Lösungsmittel können in Frage kommen: Aromatische Kohlenwasserstoffe, bevorzugt die Fraktionen C₈ bis C₁₂, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole, sowie deren Ether und Ester wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder N,N-Dimethylformamid, sowie gegebenenfalls epoxidierte Pflanzenöle wie epoxidiertes Kokosnußöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften der Formulierung können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside und Tensidgemische mit guten Emulgier-, Dispergier- und Netzeigenschaften in Betracht.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren (C₁₀-C₂₂), wie z.B. die Na- oder K-Salze der Öl- oder Stearinsäure, oder von natürlichen Fettsäuregemischen genannt, die z.B. aus Kokosnuß- oder Talgöl gewonnen werden können. Ferner sind auch die Fettsäure-methyl-taurinsalze zu erwähnen.

Häufiger werden jedoch sog. synthetische Tenside verwendet, insbesondere Fettalkoholsulfonate, Fettalkoholsulfate, sulfonierte Benzimidazolderivate oder Alkylarylsulfonate.

Die Fettalkoholsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschließt, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxid-Adduktes oder Phospholipide in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxidaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi-(2-chlorethyl)-ethylammoniumbromid.

Die in der Formulierungstechnik gebräuchlichen Tenside, die auch in den erfindungsgemäßen Mitteln verwendet werden können, sind u.a. in "Mc Cutcheon's Detergents and Emulsifiers Annual" MC Publishing Corp., Ridgewood New Jersey, 1981, Stache, H., "Tensid-Taschenbuch", Carl Hanser Verlag, München/Wien, 1981 und M. und J. Ash, "Encyclopedia of Surfactants", Vol I-lll, Chemical Publishing Co., New York, 1980-81 beschrieben.

Die herbiziden Formulierungen enthalten in der Regel 0,1 bis 99 Gew%, insbesondere 0,1 bis 95 Gew.-% Herbizid, 1 bis 99,9 Gew.-%, insbesondere 5 bis 99,8 Gew.-%, eines festen oder flüssigen Formulierungshilfsstoffes und 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren z.B. gegebenenfalls epoxydierte Pflanzenöle (epoxydiertes Kokosnußöl, Rapsöl oder Sojaöl), Entschäumer, z.B. Silikonöl, Konservierungsmittel, Viskositätsregulatoren, Bindemittel, Haftmittel sowie Dünger oder andere Wirkstoffe enthalten.

Insbesondere setzen sich bevorzugte Formulierungen folgendermaßen zusammen:
(% = Gewichtsprozent)

| Emulgierbare Konzentrate | |
|---|---|
| Aktiver Wirkstoff | 1 bis 90 %, vorzugsweise 5 bis 50 % |
| oberflächenaktives Mittel | 5 bis 30 %, vorzugsweise 10 bis 20 % |
| Lösungsmittel | 15 bis 94 %, vorzugsweise 70 bis 85 % |

| Stäube | |
|---|---|
| Aktiver Wirkstoff | 0,1 bis 50 %, vorzugsweise 0,1 bis 1 % |
| festes Trägermittel | 99,9 bis 90 %, vorzugsweise 99,9 bis 99 % |

| Suspensions-Konzentrate | |
|---|---|
| Aktiver Wirkstoff | 5 bis 75 %, vorzugsweise 10 bis 50 % |
| Wasser | 94 bis 24 %, vorzugsweise 88 bis 30 % |
| oberflächenaktives Mittel | 1 bis 40 %, vorzugsweise 2 bis 30 % |

| Benetzbare Pulver: | |
|---|---|
| Aktiver Wirkstoff | 0,5 bis 90 %, vorzugsweise 1 bis 80 % |
| oberflächenaktives Mittel | 0,5 bis 20 %, vorzugsweise 1 bis 15 % |
| festes Trägermaterial | 5 bis 95 %, vorzugsweise 15 bis 90 % |

| Granulate | |
|---|---|
| Aktiver Wirkstoff | 0,1 bis 30 %, vorzugsweise 0,1 bis 15 % |
| festes Trägermittel | 99,5 bis 70 %, vorzugsweise 97 bis 85 % |

Die Wirkstoffe der Formel I werden in der Regel auf die Pflanze oder deren Lebensraum mit Aufwandmengen von 0,001 bis 4 kg/ha, insbesondere 0,005 bis 2 kg/ha erfolgreich eingesetzt. Die für die erwünschte Wirkung erforderliche Dosierung kann durch Versuche ermittelt werden. Sie ist abhängig von der Art der Wirkung, dem Entwicklungsstadium der Kulturpflanze und des Unkrauts sowie von der Applikation (Ort, Zeit, Verfahren) und kann, bedingt durch diese Parameter, innerhalb weiter Bereiche variieren.

Die Verbindungen der Formel I zeichnen sich durch herbizide und wuchshemmende Eigenschaften aus, die sie zum Einsatz in Kulturen von Nutzpflanzen, insbesondere in Getreide, Baumwolle, Soja, Zuckerrüben, Zuckerrohr, Plantagenkulturen, Raps, Mais und Reis sowie zur nicht-selektiven Unkrautkontrolle befähigen.

Unter Kulturen sind auch solche zu verstehen, die durch konventionelle züchterische oder gentechnologische Methoden gegen Herbizide bzw. Herbizidklassen tolerant gemacht worden sind. Bei den zu bekämpfenden Unkräutern kann es sich sowohl um mono- als auch um dikotyle Unkräuter handeln, wie zum Beispiel Stellaria, Nasturtium, Agrostis, Digitaria, Avena, Setaria, Sinapis, Lolium, Solanum, Phaseolus, Echinochloa, Scirpus, Monochoria, Sagittaria, Bromus, Alopecurus, Sorghum halepense, Rottboellia, Cyperus, Abutilon, Sida, Xanthium, Amaranthus, Chenopodium, Ipomoea, Chrysanthemum, Galium, Viola und Veronica.

Die folgenden Beispiele erläutern die Erfindung weiter, ohne sie zu beschränken.

### Herstellungsbeispiele:

### Beispiel H1: 3-(4-Chlor-2-fluor-5-(2'-chlorallyloxy)phenyl)-4-chlor-5-cyano-1-methyl-[1H]-pyrazol

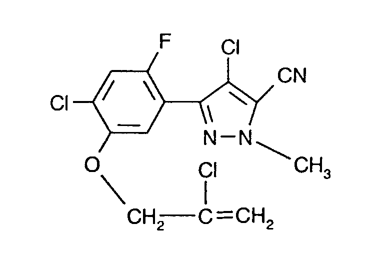

2,0 g (0,007 Mol) 3-(4-Chlor-2-fluor-5-hydroxyphenyl)-4-chlor-5-cyano-1-methyl-[1H]-pyrazol wird in 20 ml N-Methylpyrrolidon (NMP) gelöst. Unter Rühren und Kühlen im Eisbad wird 2,90 g (3 Aequivalente) wasserfreies Kaliumcarbonat zugegeben, dann tropft man 0,75 ml 2,3-Dichlorpropan langsam dazu und lässt über Nacht bei 50°C rühren. Das analytische Dünnschichtchromatogramm einer aufgearbeiteten Probe zeigt vollständigen Reaktionsumsatz. Nach dem Abkühlen auf 22°C wird mit Diethylether verdünnt und zuerst mit Eiswasser, dann mit Sole gewaschen. Man trocknet über Natriumsulfat, filtriert ab und engt zusammen mit 5 g Kieselgel ein. Nach dem Aufbringen des Adsorbats auf eine Flash-Kieselgelsäule wird mit n-Hexan/Essigsäureethylester 4/1 eluiert. Man erhält nach dem Einengen der entsprechenden Fraktionen 1,91 g des gewünschten Produkts als weissen Feststoff (76% der Theorie).

### Beispiel H2: 3-(4-Chlor-2-fluor-5-hydroxy-6-allylphenyl)-4-chlor-5-cyano-1-methyl-[1H]-pyrazol

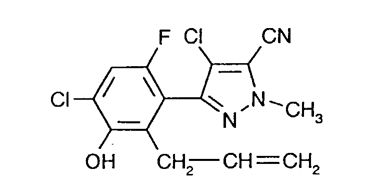

4,0 g (0,0123 Mol) 3-(4-Chlor-2-fluor-5-allyloxyphenyl)-4-chlor-5-cyano-1-methyl-[1H]-pyrazol wird in einen Kolben eingewogen und mit aufgesetztem Kühler zuerst geschmolzen und dann in einem Oelbad auf 195°C erhitzt. Nach 3 Stunden zeigt die Analyse mittels Dünnschichtchromatogramm dass alles Edukt umgesetzt ist. Es wird auf 22°C abgekühlt und der Rückstand in Dichlormethan aufgenommen und auf Kieselgel aufgezogen. Dieses wird auf eine Flash-Kieselgelsäule aufgetragen. Zuerst wird mit n-Hexan/Essigsäureethylester 5/1 mit 1% Triethylamin gewaschen und dann mit Tetrachlorkohlenstoff/Essigsäureethylester 5/1 eluiert. Nach dem Einengen wird zwischen Diethylether und Wasser verteilt, ausgeschüttelt und die Phasen getrennt. Die Etherphase wird mit Sole gewaschen, über Natriumsulfat getrocknet, filtriert und eingeengt. Man erhält 2,83 g des gewünschten Produkts als rosa gefärbten Feststoff (85% der Theorie) mit Smp. 109-110°C.

### Analog zu Beispiel H2 kann auch

### Beispiel H3: 3-(4-Chlor-2-fluor-5-hydroxy-6-(2'-chlorallyl)phenyl)-4-chlor-5-cyano-1-methyl-[1H]-pyrazol

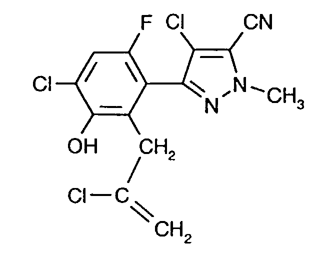

als Harz erhalten werden; Ausbeute 82% der Theorie.

### Beispiel H4: 3-(4-Chlor-2-fluor-5-hydroxy-6-but-2-enylphenyl)-4-chlor-5-cyano-1-methyl-[1H]-pyrazol

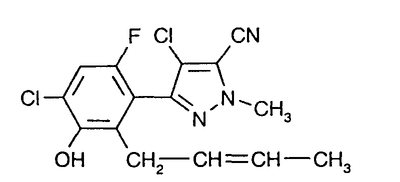

100 mg 3-(4-Chlor-2-fluor-5-methallyloxyphenyl)-4-chlor-5-cyano-1-methyl-[1H]-pyrazol wird in einem Kolben mit aufgesetztem Kühler zuerst geschmolzen und dann während 3 Stunden in einem Oelbad von 195°C erhitzt. Danach wird auf 22°C abgekühlt und in Dichlormethan aufgenommen. Die Dichlormethan-Lösung wird zusammen mit Kieselgel eingeengt. Nach dem Aufbringen des Kieselgels auf eine Flash-Kieselgelsäule wird mit n-Hexan/Essigsäureethylester 5/1 eluiert. Man erhält nach dem Einengen der entsprechenden Fraktionen 0,07 g des gewünschten Produkts in Form eines Harzes (70% der Theorie).

### Beispiel H5: 4-Chlor-3-(7-chlor-5-fluor-2-hydroxymethyl-2,3-dihydrobenzofuran-4-yl)-1-methyl-[1H]-pyrazol-5-carbonitril

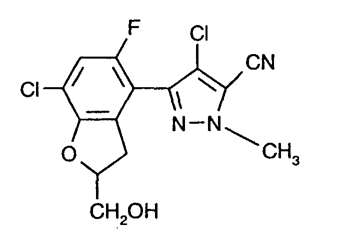

2,16 g (0,0066 Mol) 3-(4-Chlor-2-fluor-5-hydroxy-6-allylphenyl)-4-chlor-5-cyano-1-methyl-[1 H]-pyrazol (Beispiel H2) wird in 60 ml Chloroform vorgelegt. Dann wird 2,29 g (1,1 Aequivalente) meta-Chlorperbenzoesäure (MCPA) zugegeben. Das Gemisch wird während 3 Stunden unter Erhitzen zum Rückfluss gerührt. Danach erfolgt die Zugabe von weiteren 0,21 g (0,1 Aequivalente) MCPA. Nach 2 Stunden Rückflussieren wird das Gemisch mit Diethylether verdünnt, mit wässriger Natriumhydrogencarbonat-Lösung und dann mit Sole gewaschen. Nach dem Trocknen über Natriumsulfat wird filtriert und eingeengt. Man erhält 2,98 g Rohprodukt als gelbes Harz. Das Rohprodukt wird mit Dichlormethan auf Kieselgel aufgezogen und nach dem Aufbringen auf eine Flash-Kieselgelsäule mit n-Hexan/Essigsäureethylester 1/1 bis 1/2 eluiert. Man erhält 2,07 g des gewünschten Produkts in Form eines leicht gelben Harzes (86% der Theorie).

### Beispiel H6: 7-Chlor-4-(4-chlor-5-cyano-1-methyl-[1H]-pyrazol-3-yl)-5-fluor-2,3-dihydrobenzofuran-2-ylmethyl-essigsäureester

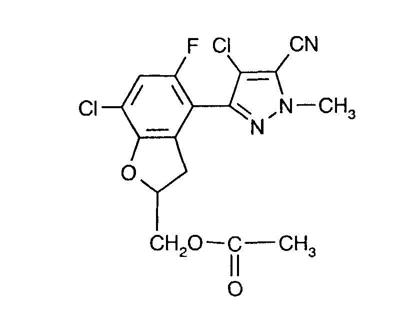

0,66 g (0,00193 Mol) 4-Chlor-3-(7-chlor-5-fluor-2-hydroxymethyl-2,3-dihydrobenzofuran-4-yl)-1-methyl-[1H]-pyrazol-5-carbonitril (Beispiel H5) wird in 10 ml Pyridin gelöst. Unter Rühren und Eiskühlung wird 0,20 ml Essigsäureanhydrid (1,1 Aequivalente) zugegeben. Man rührt während 30 Minuten unter Eisbadkühlung, dann während 6 Stunden bei 22°C. Danach wird das Gemisch in verdünnte Salzsäure gegeben und mit Diethylether extrahiert. Die organische Phase wird mit Sole gewaschen, über Natriumsulfat getrocknet, filtriert und auf Kieselgel aufgezogen. Nach dem Aufbringen auf eine Flash-Kieselgelsäule wird mit n-Hexan/Essigsäureethylester 5/1 bis 2/1 eluiert. Man erhält 0,50 g des gewünschten Produkts als farbloses Oel (67% der Theorie).

### Beispiel H7: 4-Chlor-3-(7-chlor-5-fluor-2-carboxyl-2,3-dihydrobenzofuran-4-yl)-1-methyl-[1H]-pyrazol-5-carbonitril

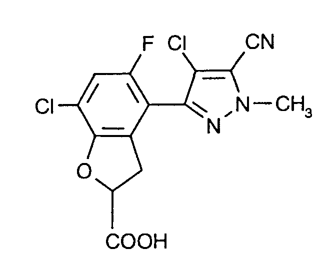

Zu 2,07 g 4-Chlor-3-(7-chlor-5-fluor-2-hydroxymethyl-2,3-dihydrobenzofuran-4-yl)-1-methyl[1 H]-pyrazol-5-carbonitril (Beispiel H5) in 20 ml Aceton wird 3 ml einer Lösung von Jones-Reagens (0,59 g Chromtrioxid, 0,53 ml konzentrierte Schwefelsäure, 3 ml Wasser) unter Rühren und Kühlen im Eisbad zugegeben. Zuerst wird bei Temperaturen unterhalb 5°C, dann während 12 Stunden bei 22°C gerührt. Danach wird nochmals 3 ml des obigen Jones-Reagenses zugegeben und während 9 Stunden bei 22°C gerührt. Das Gemisch wird mit Wasser und dann mit Diethylether versetzt, ausgeschüttelt und die Phasen abegtrennt. Die Etherphase wird mit 1 M Natriumhydroxid-Lösung extrahiert. Nach dem Ansäuern der Natriumhydroxid-Lösung mit Salzsäure auf pH 1 wird mit Essigsäureethylester extrahiert, die organische Phase mit Sole gewaschen, über Natriumsulfat getrocknet, filtriert und eingeengt. Man erhält das Rohprodukt als braunes Harz in 1,0 g Ausbeute. Das Rohprodukt wird über eine Flash-Kieselgelsäule mit einem Gemisch aus Essigsäureethylester/Ethanol/Essigsäure im Verhältnis 95/5/1 gereinigt. Man erhält 0,51 g des gewünschten Produkts als Feststoff (37% der Theorie).

### Beispiel H8: 4-Chlor-3-(7-chlor-5-fluor-2-methyl-2,3-dihydrobenzofuran-4-yl)-1-methyl-[1H]-pyrazol-5-carbonitril

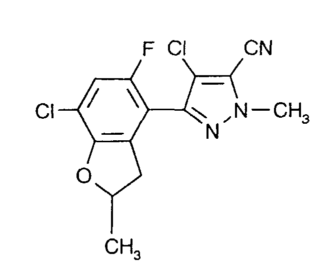

0,38 g p-Toluolsulfonsäure (0,81 Aequivalente) wird in 30 ml Xylol vorgelegt. Die Mischung wird unter Rühren und Erhitzen zum Rückfluss am Wasserabscheider absolutiert. Danach wird der Wasserabscheider entfernt und 0,80 g (0,00245 Mol) 3-(4-Chlor-2-fluor-5-hydroxy-6-allylphenyl)-4-chlor-5-cyano-1-methyl-[1H]-pyrazol (Beispiel H2) zugegeben. Es wird während 24 Stunden am Wasserabscheider zum Rückfluss erhitzt. Danach wird auf 22°C abgekühlt und im Vakuum das Lösungsmittel entfernt. Der Rückstand wird zwischen Diethylether und verdünnter wässriger Natriumhydrogencarbonat-Lösung verteilt. Nach dem Ausschütteln und Trennen der Phasen wird die organische Phase mit Sole gewaschen, über Natriumsulfat getrocknet, filtriert und auf Kieselgel aufgezogen und auf eine Flash-Kieselgelsäule aufgebracht. Es wird mit einem Gradienten von n-Hexan/Essigsäureethylester 5/1 bis 1/1 eluiert. Die Fraktionen mit einem Rf-Wert von 0,33 (gegenüber Rf = 0,041 des Edukts; Kieselgel 60 F254; n-Hexan/Essigsäureethylester 5/2) werden gesammelt. 0,32 g eines farblosen Harzes wird als Rohprodukt erhalten. Die weitere Reinigung mittels RP-HPLC (C18; 25 x 4 cm; Acetonitril/Wasser 90/1 bis 100/0; Flussrate 30 ml/Min) liefert 0,06 g eines gelben Harzes (7,5% der Theorie).

Auf analoge Weise können auch die in den folgenden Tabellen aufgeführten Verbindungen hergestellt werden.

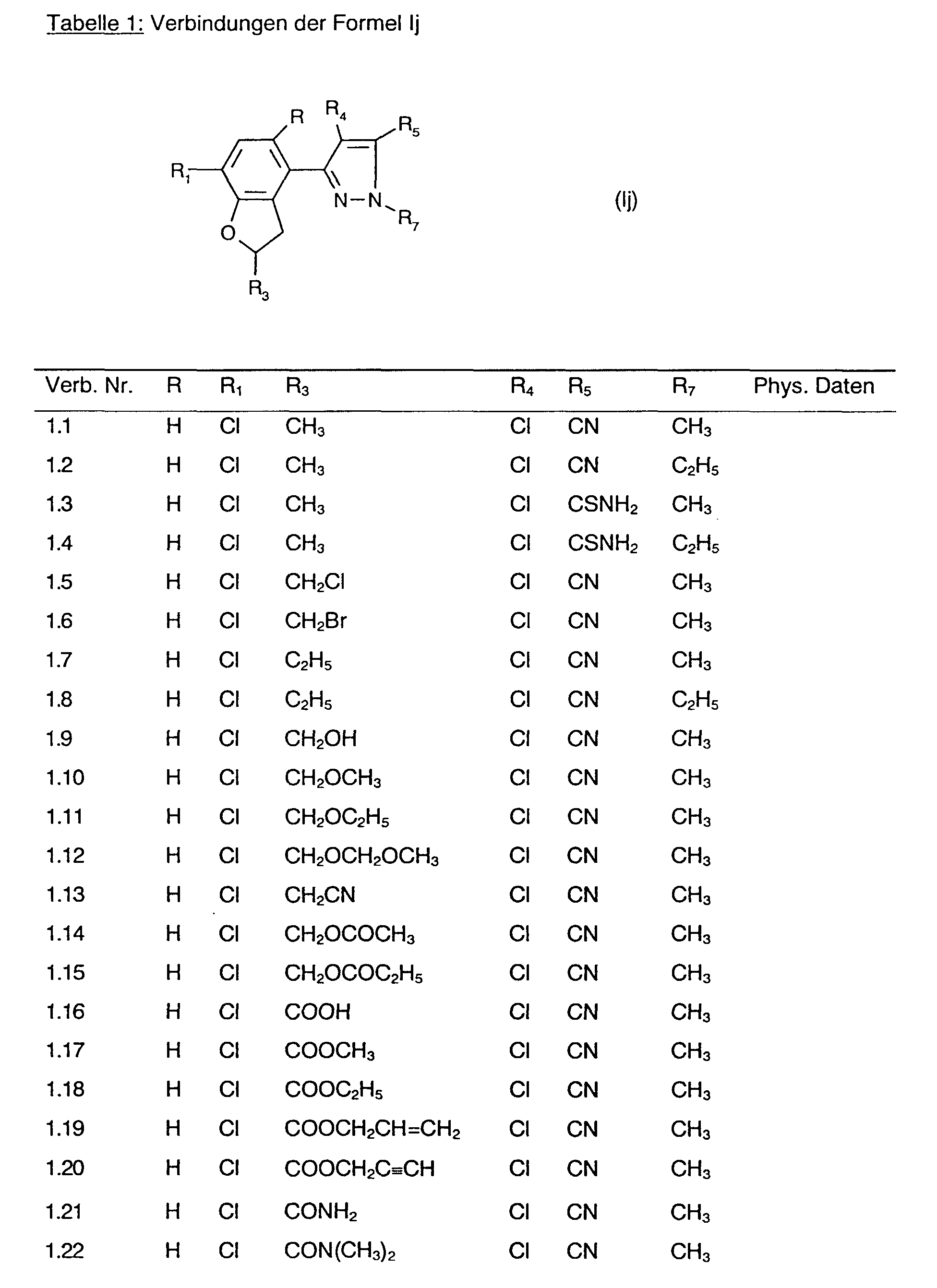

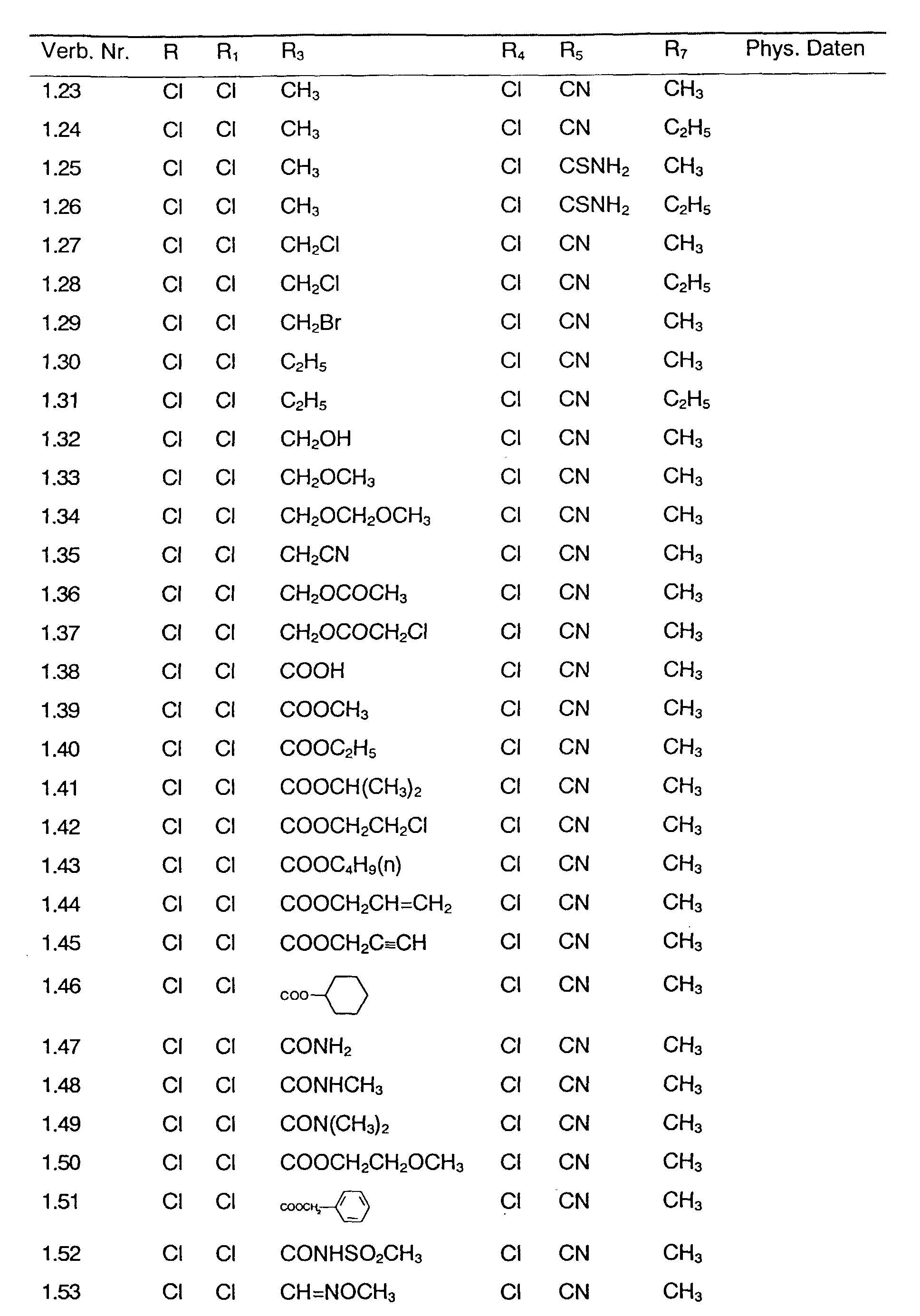

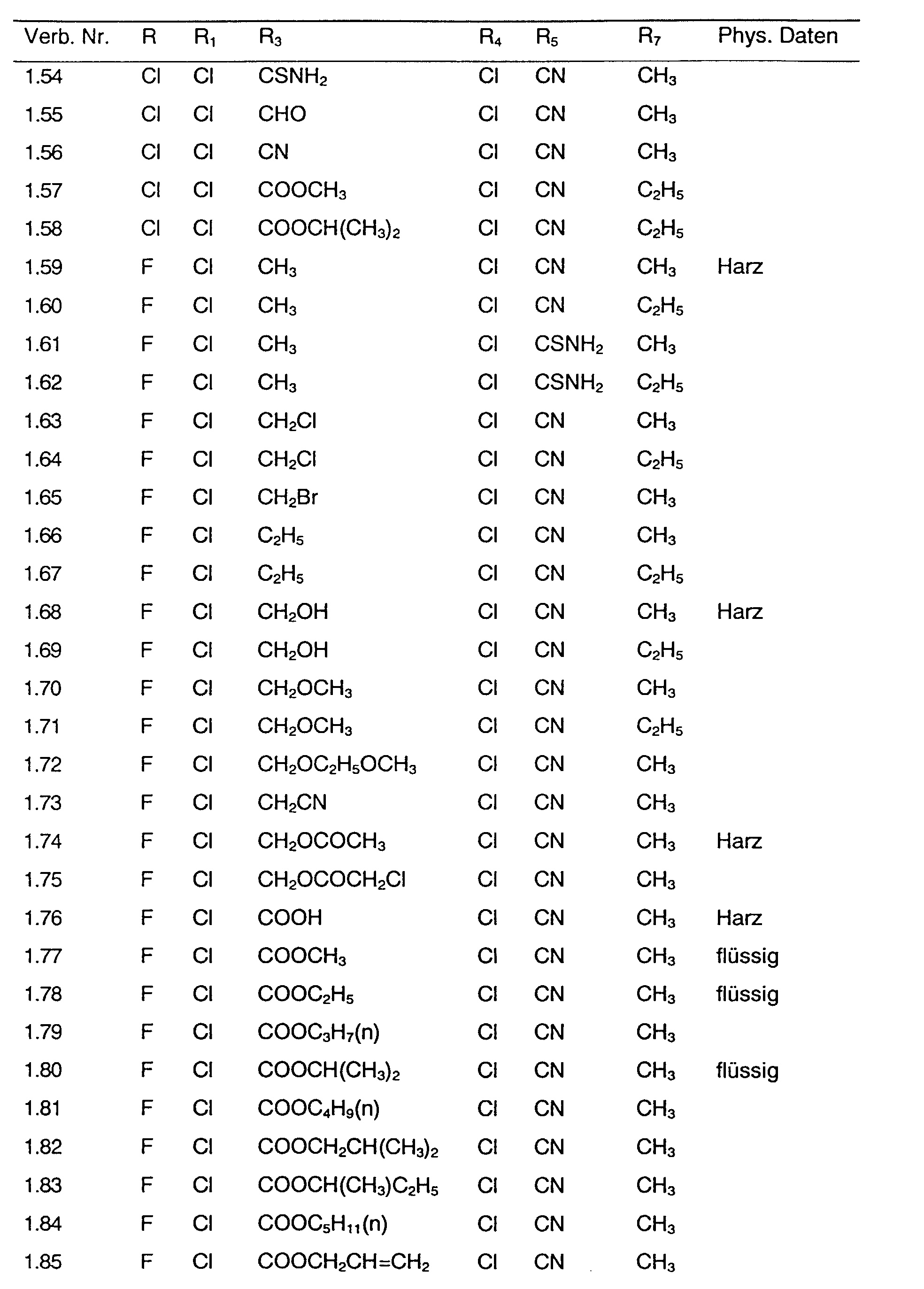

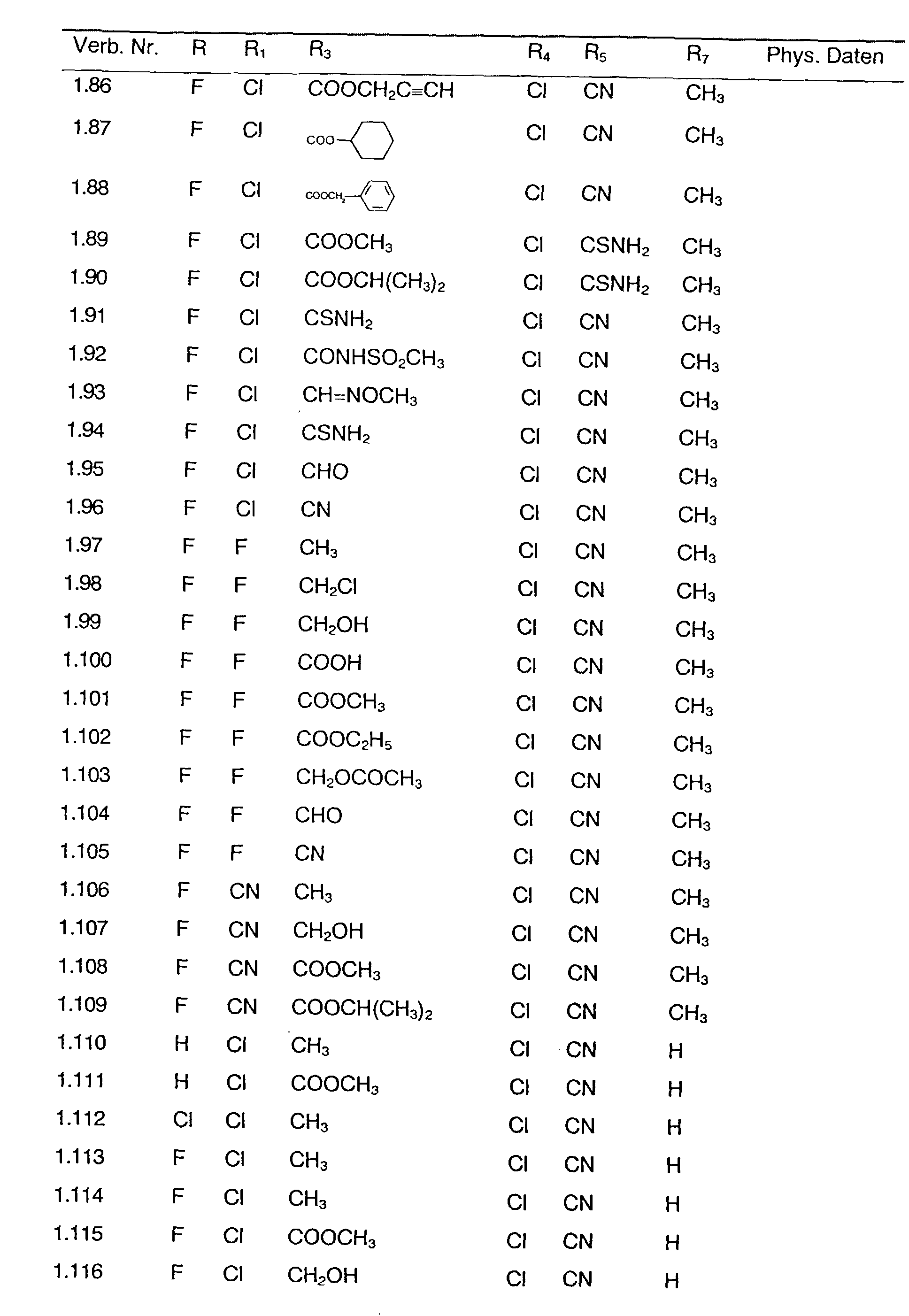

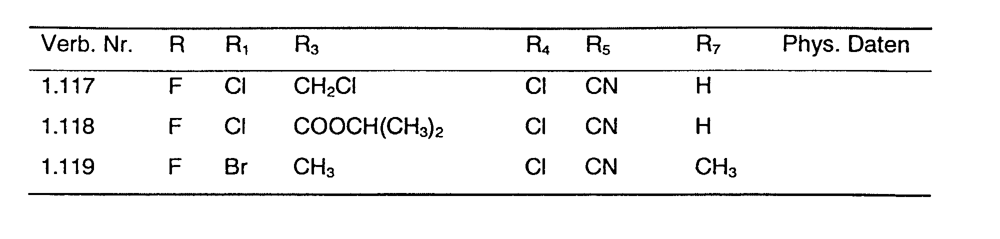

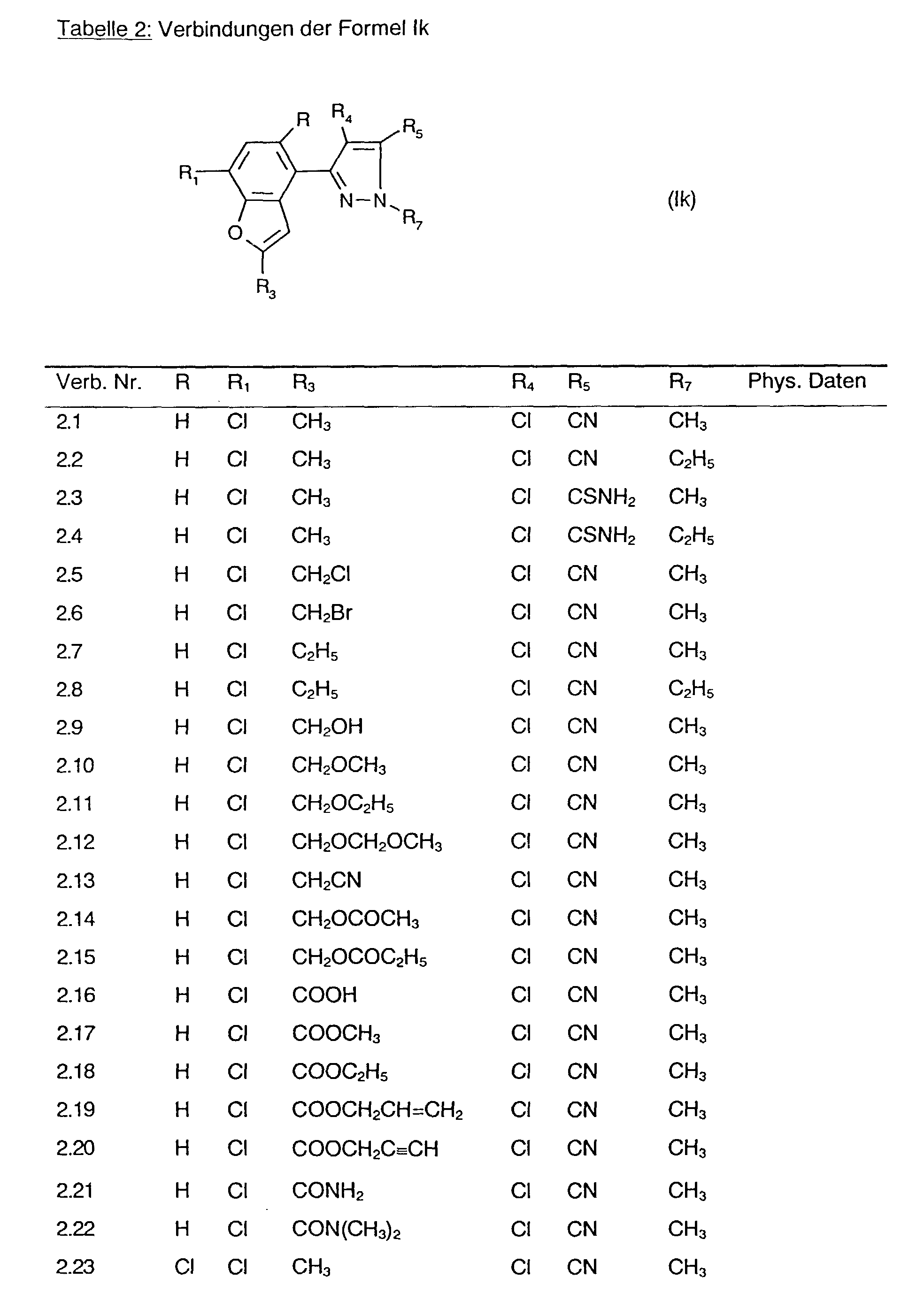

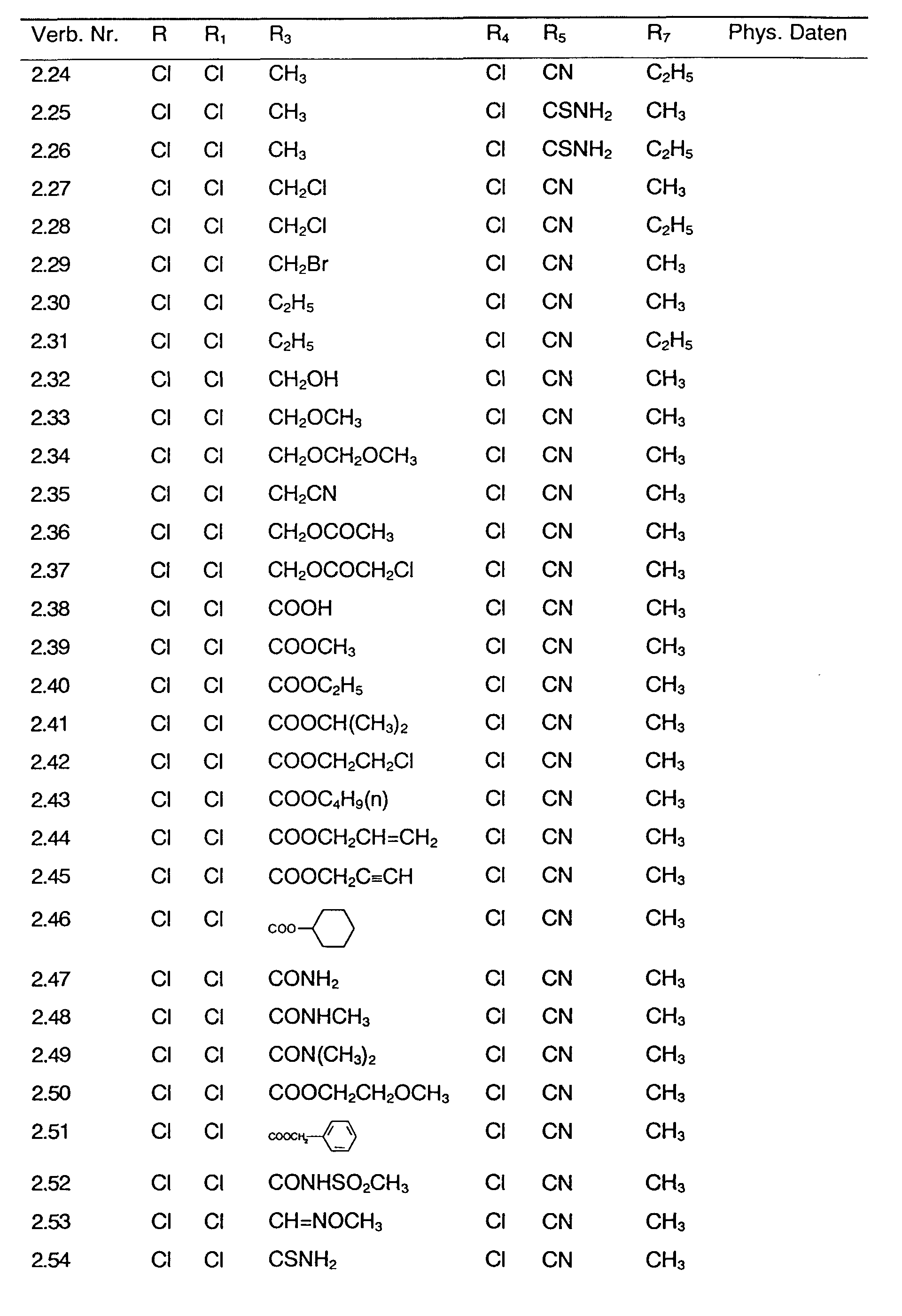

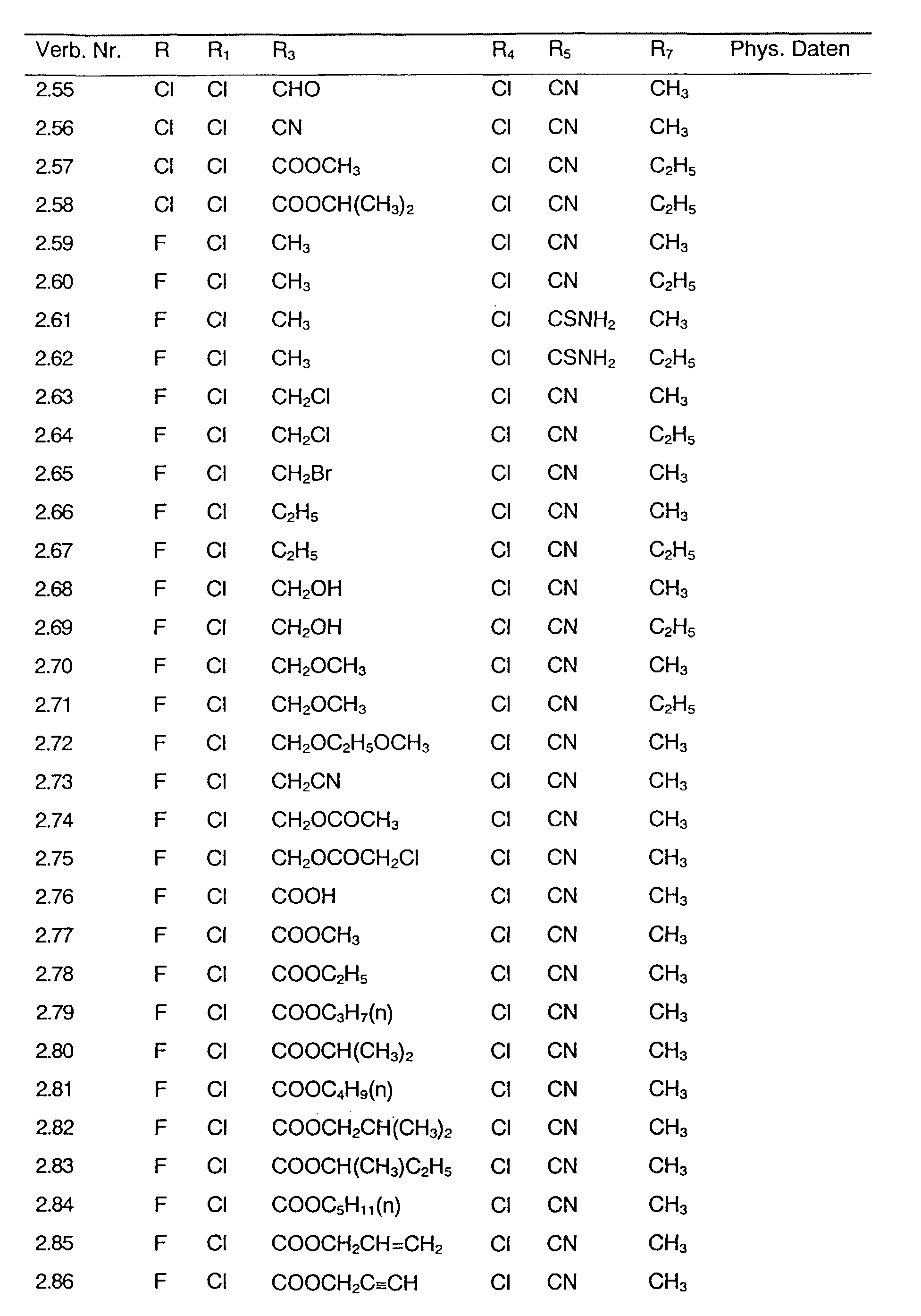

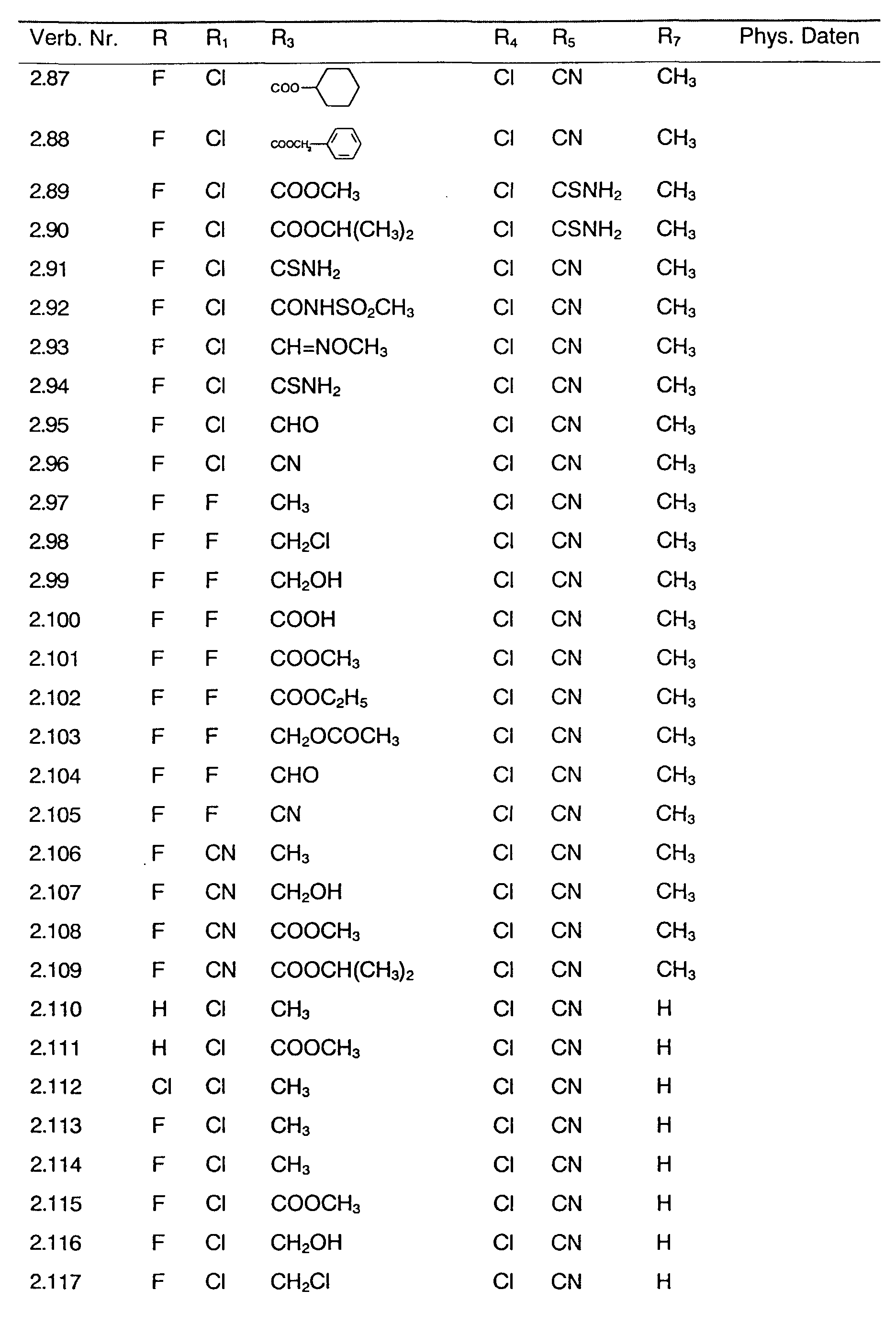

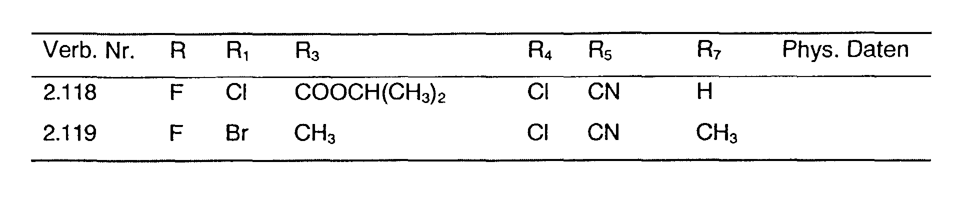

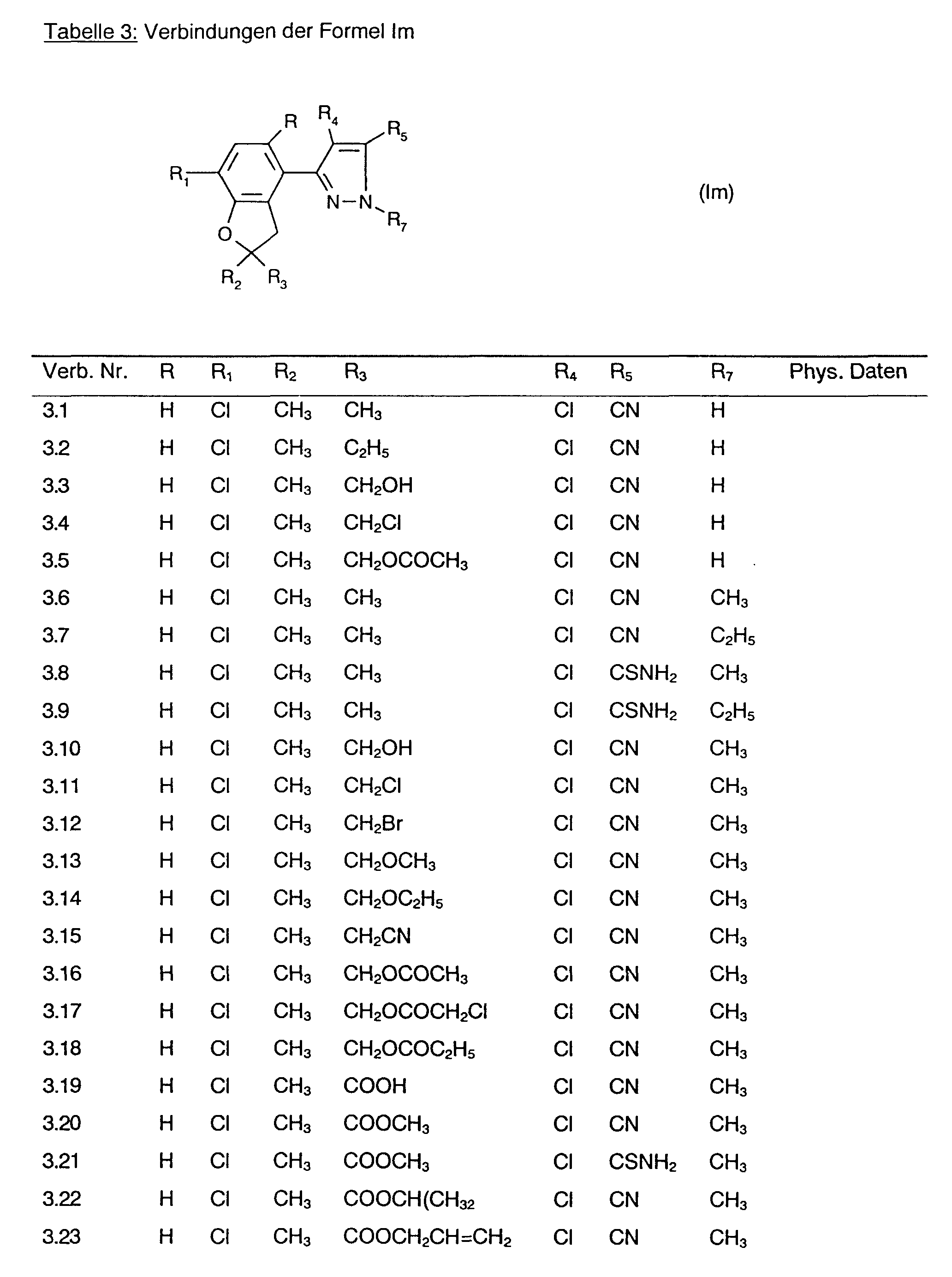

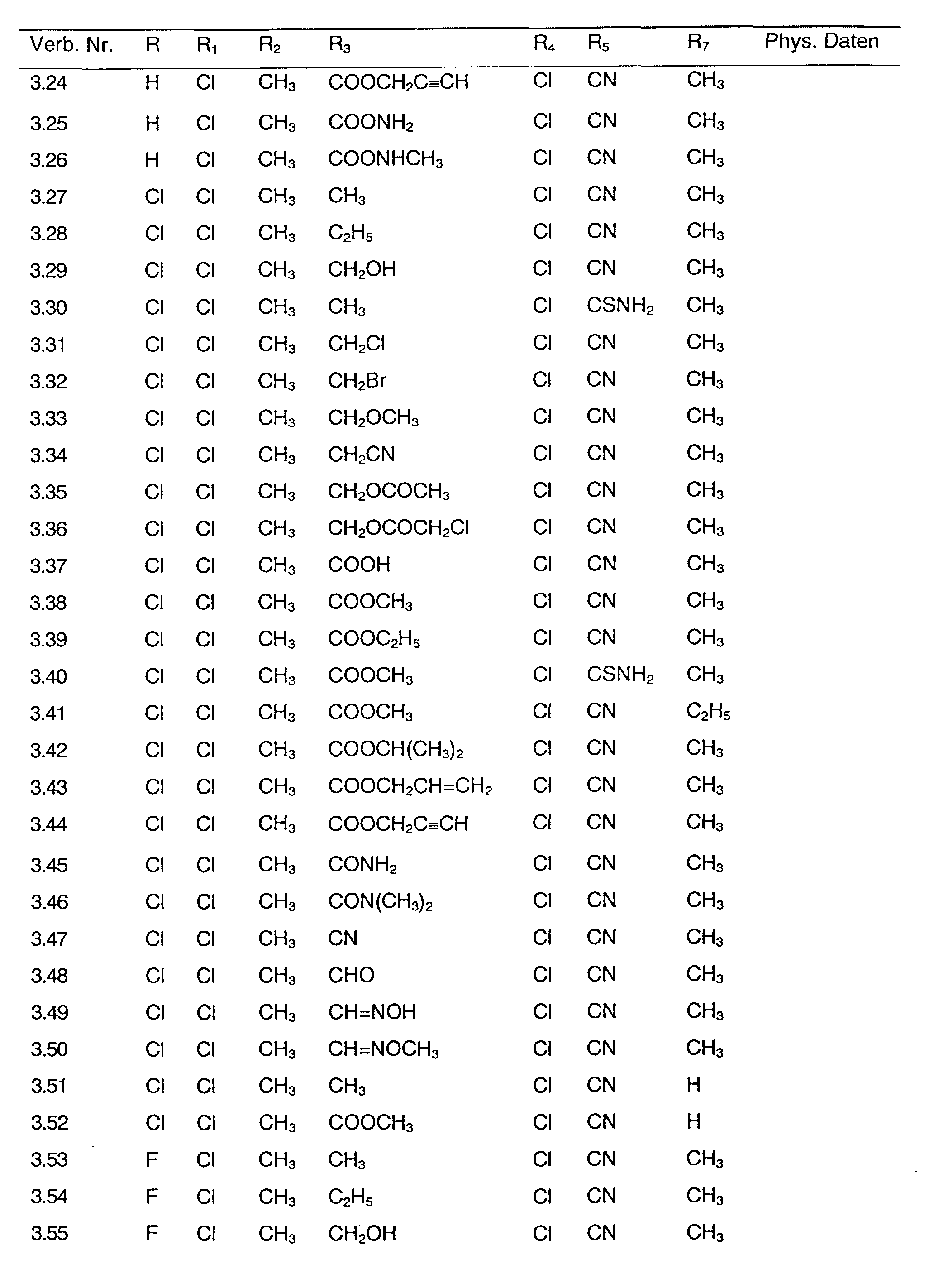

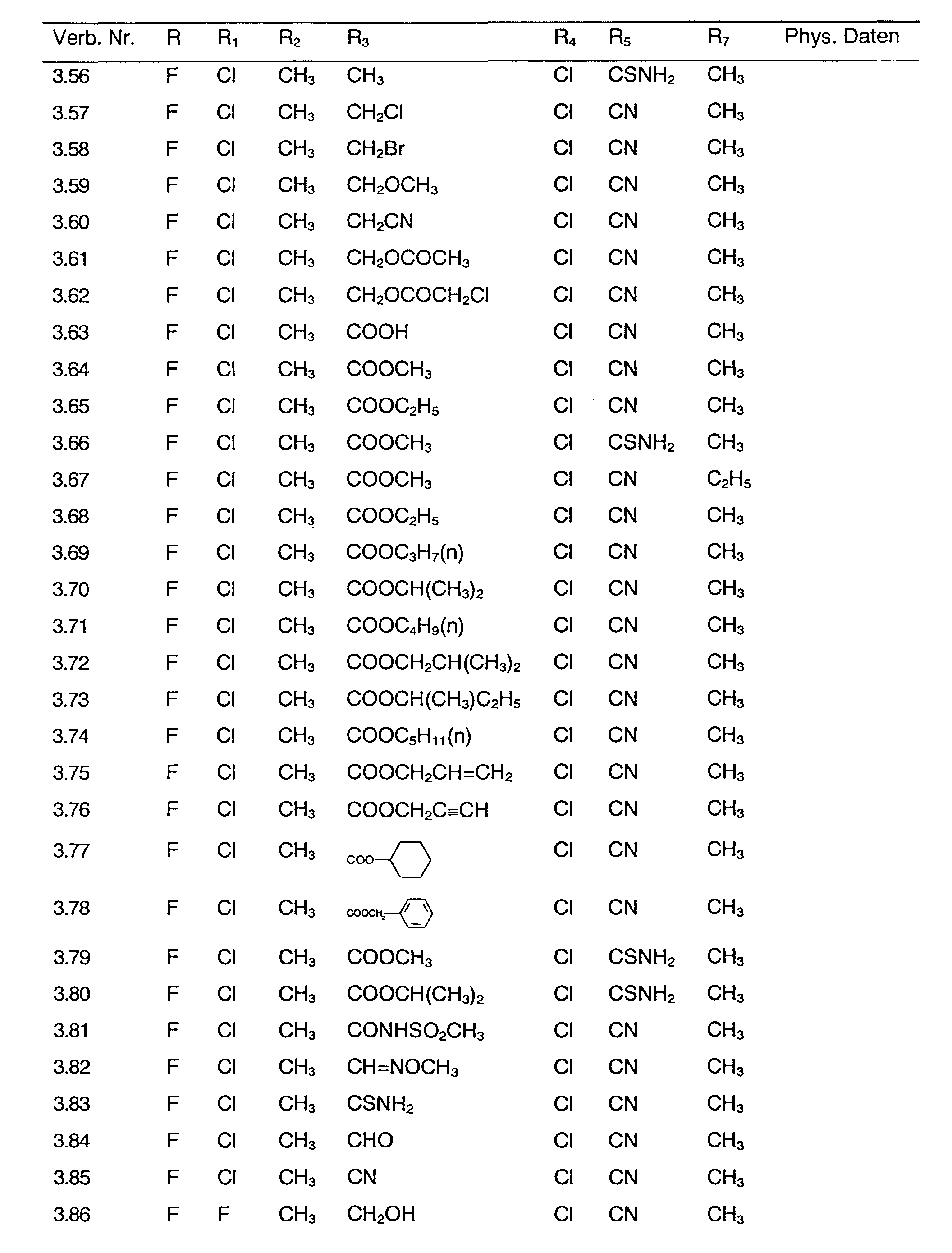

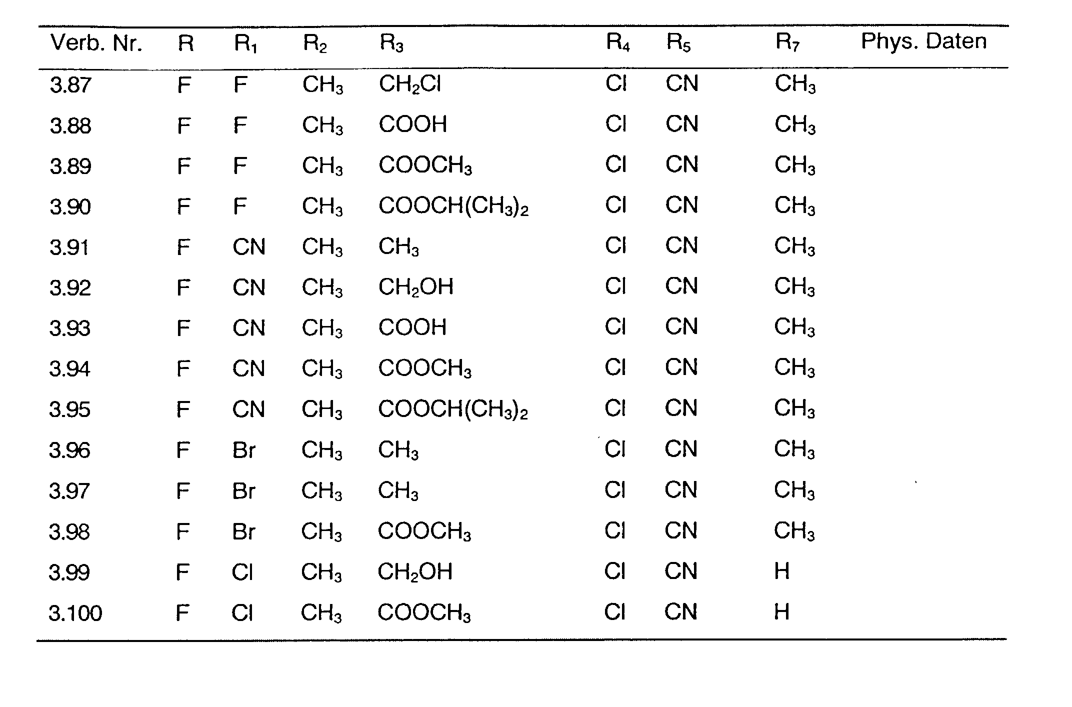

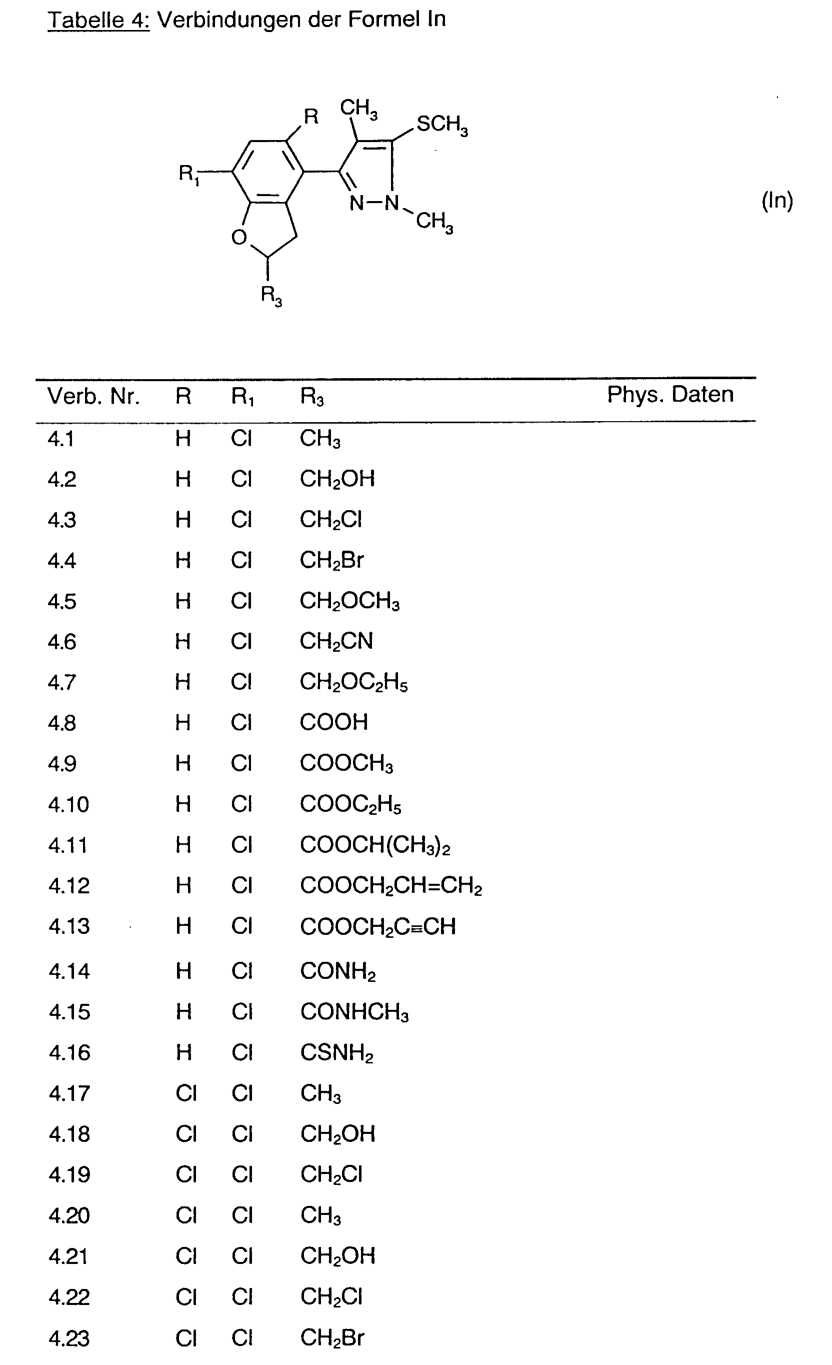

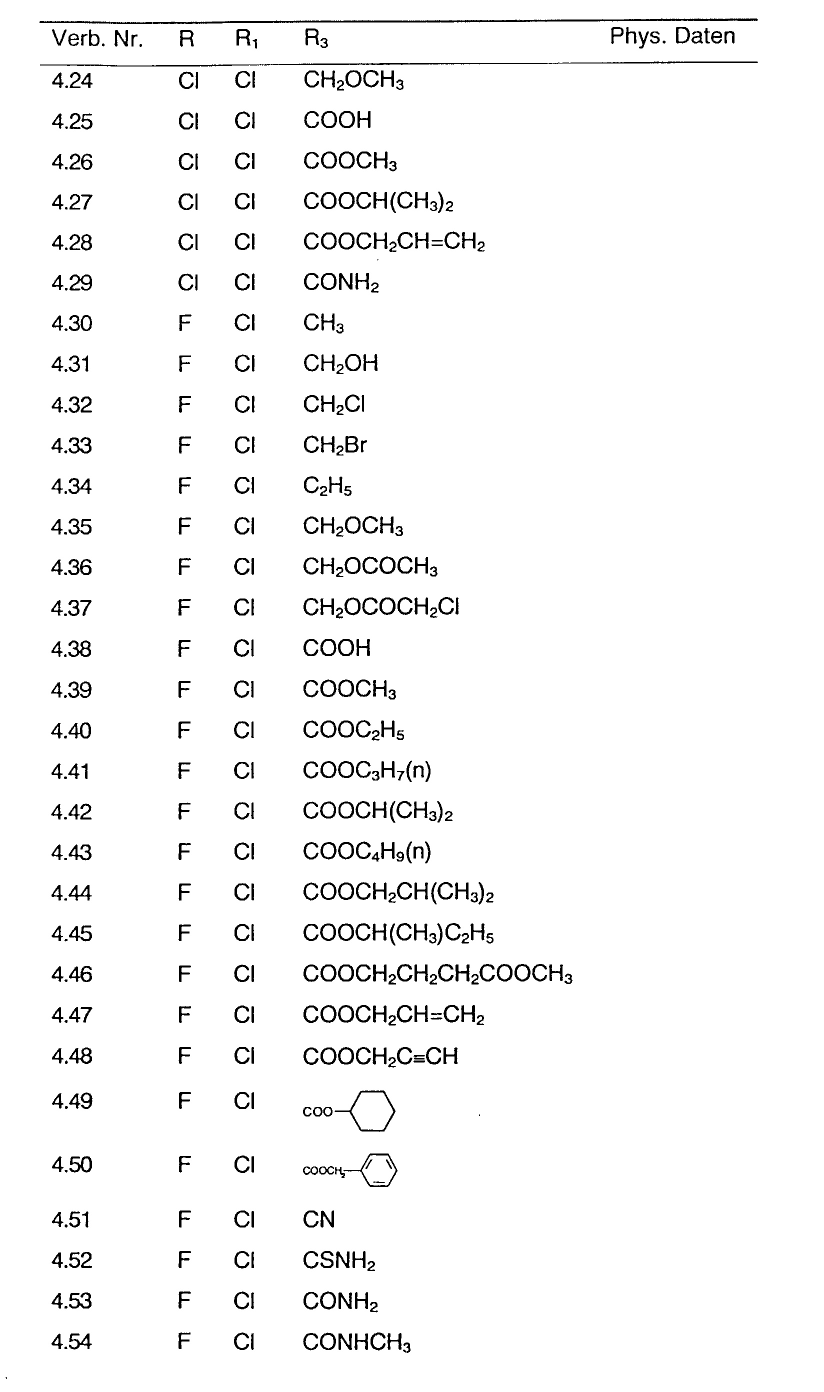

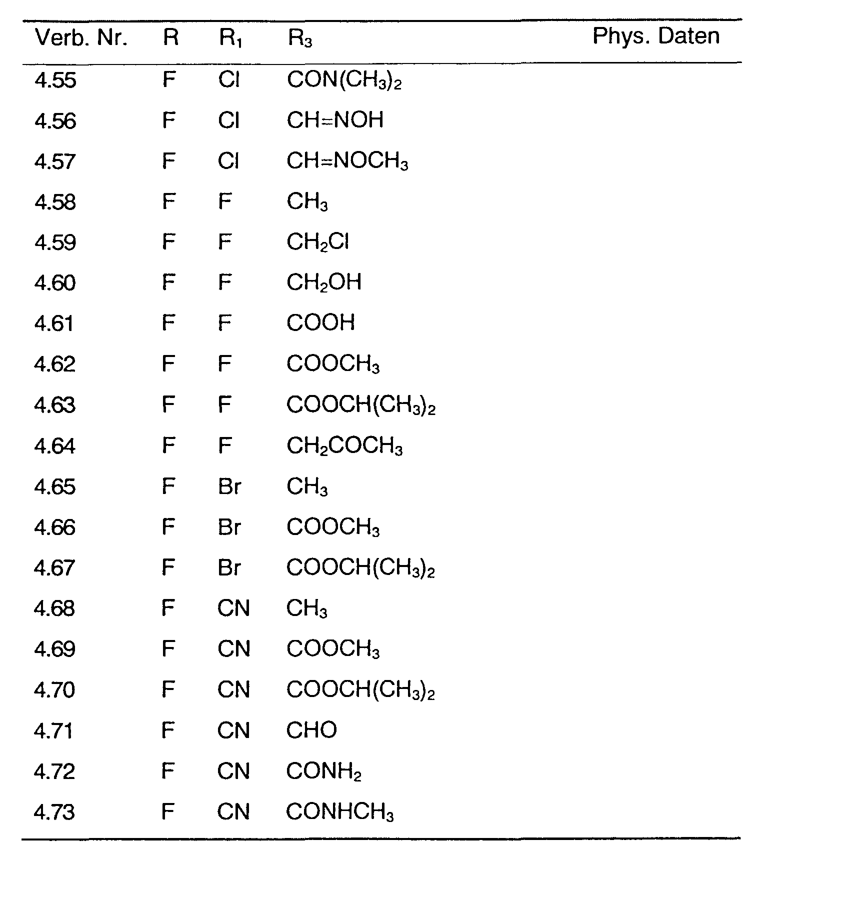

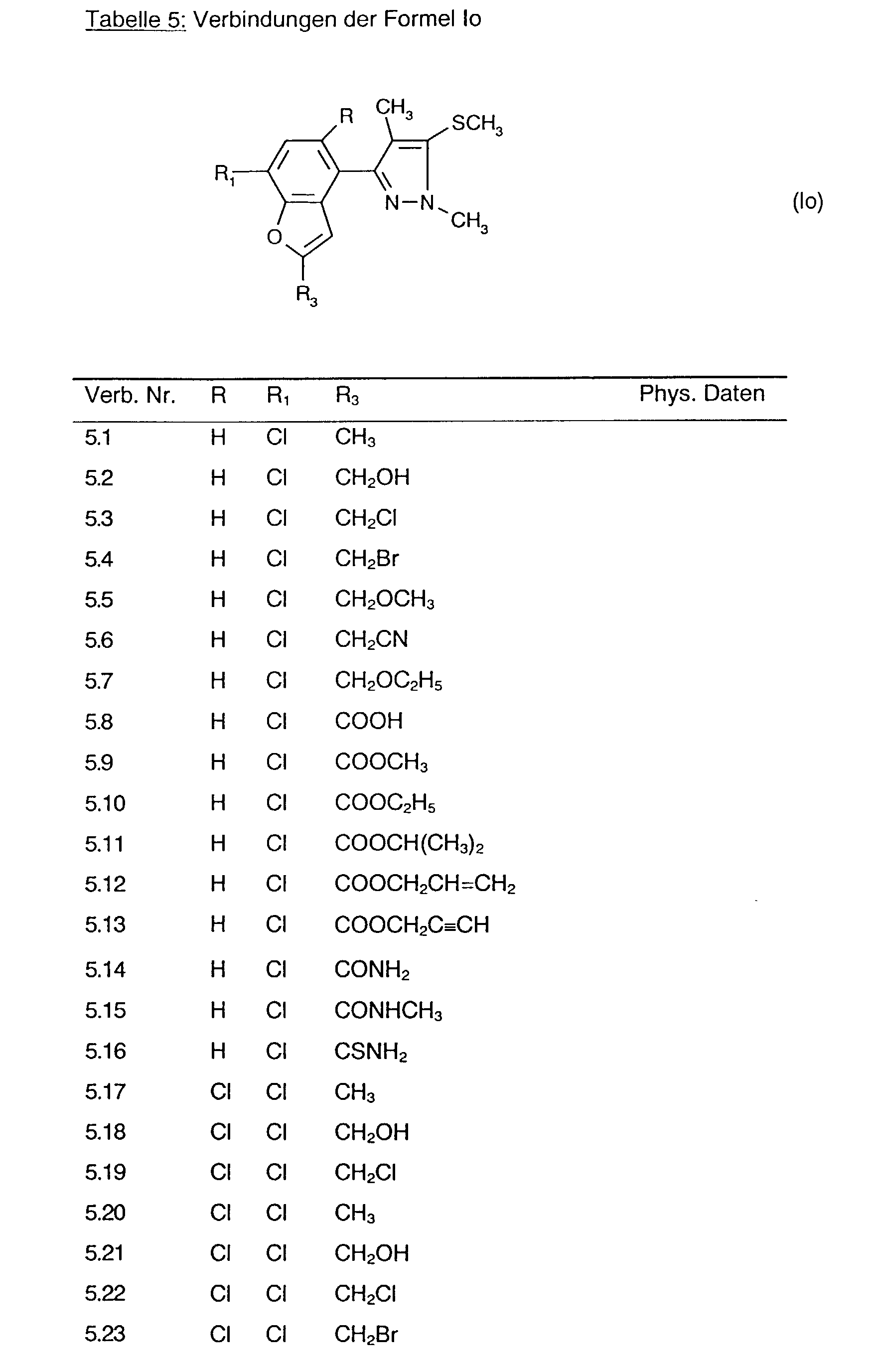

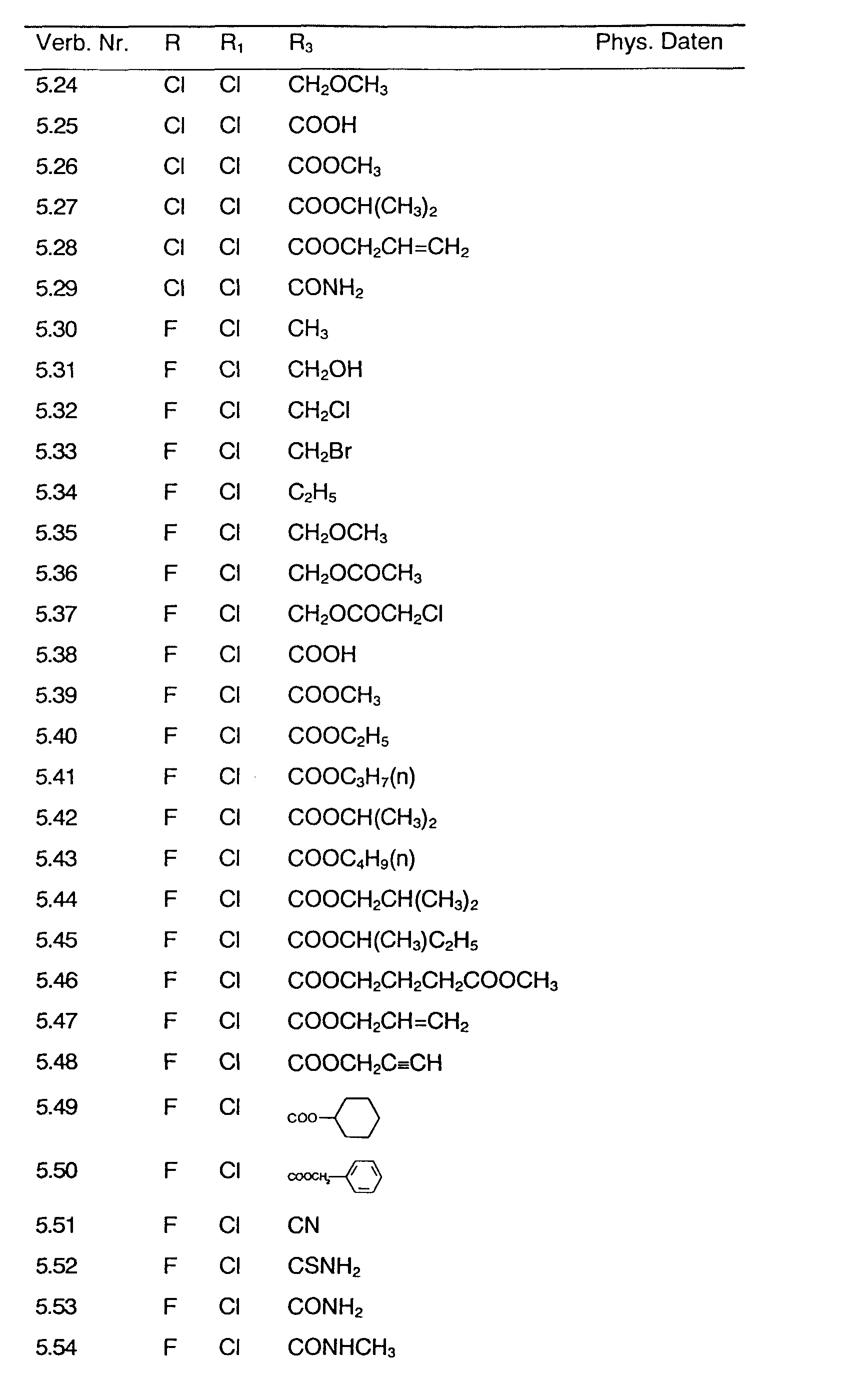

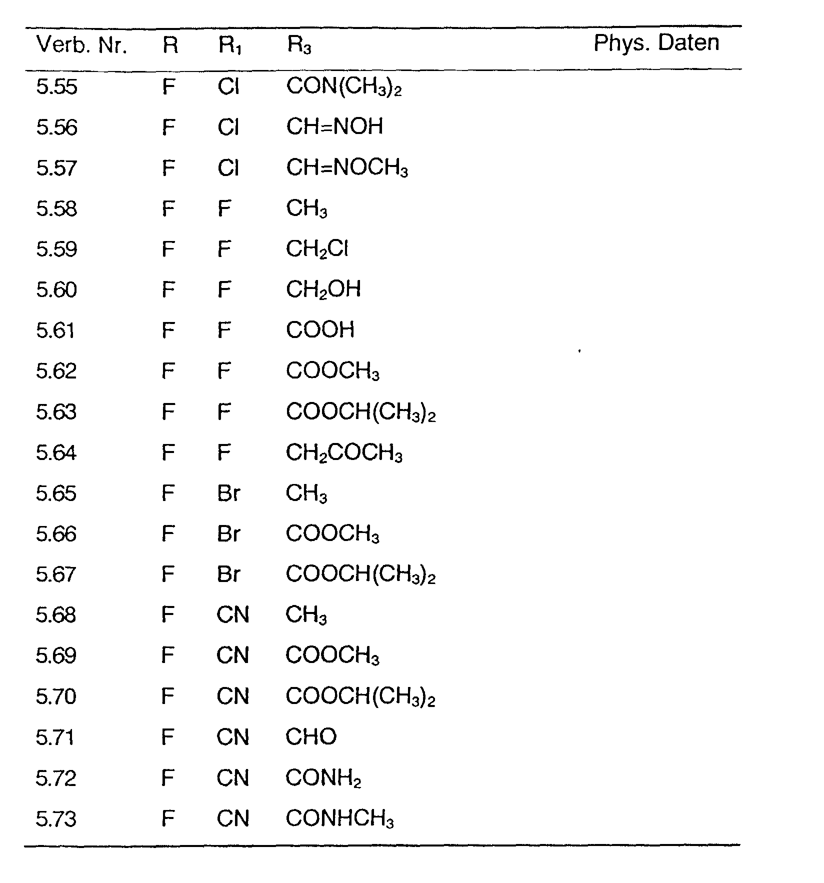

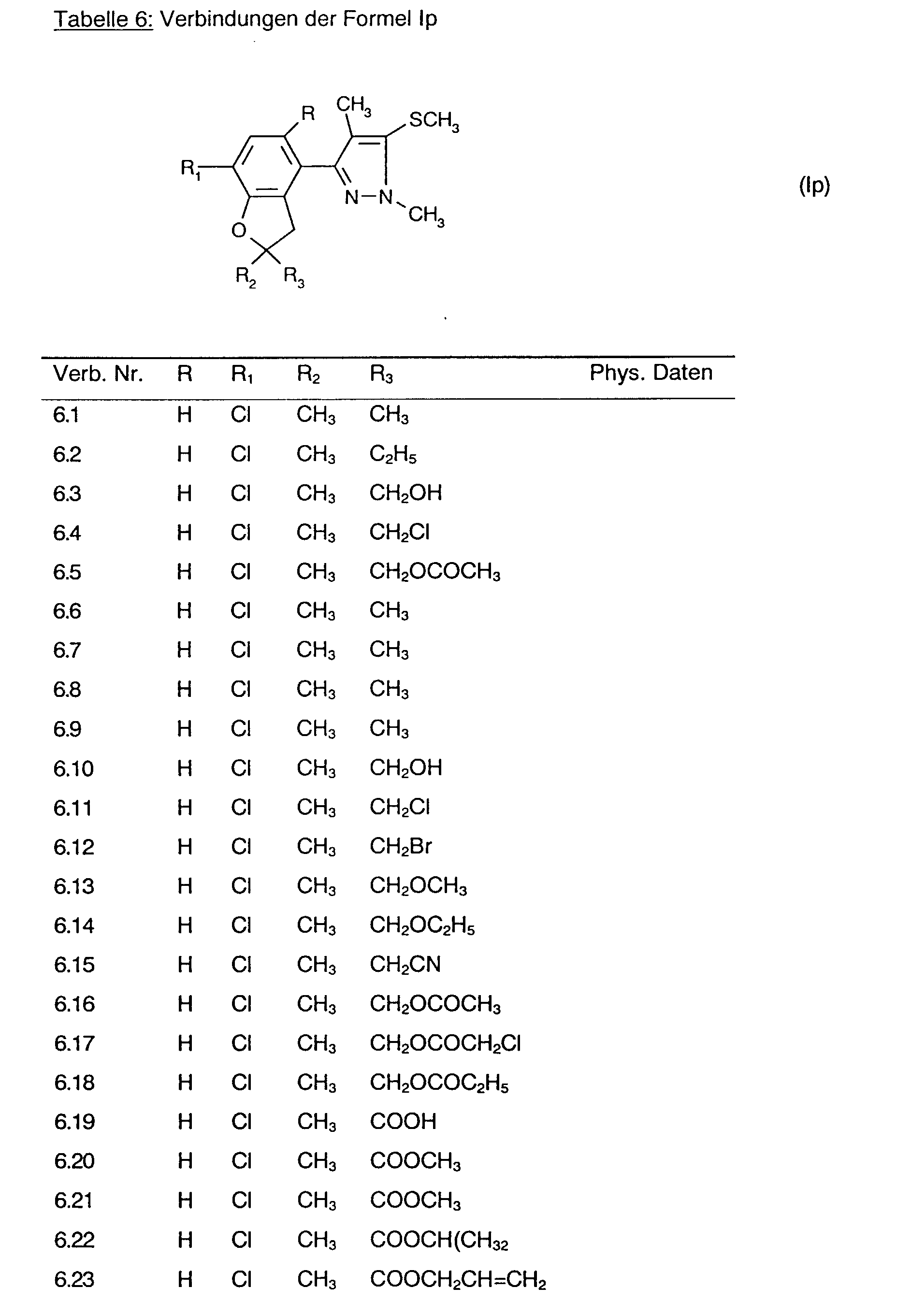

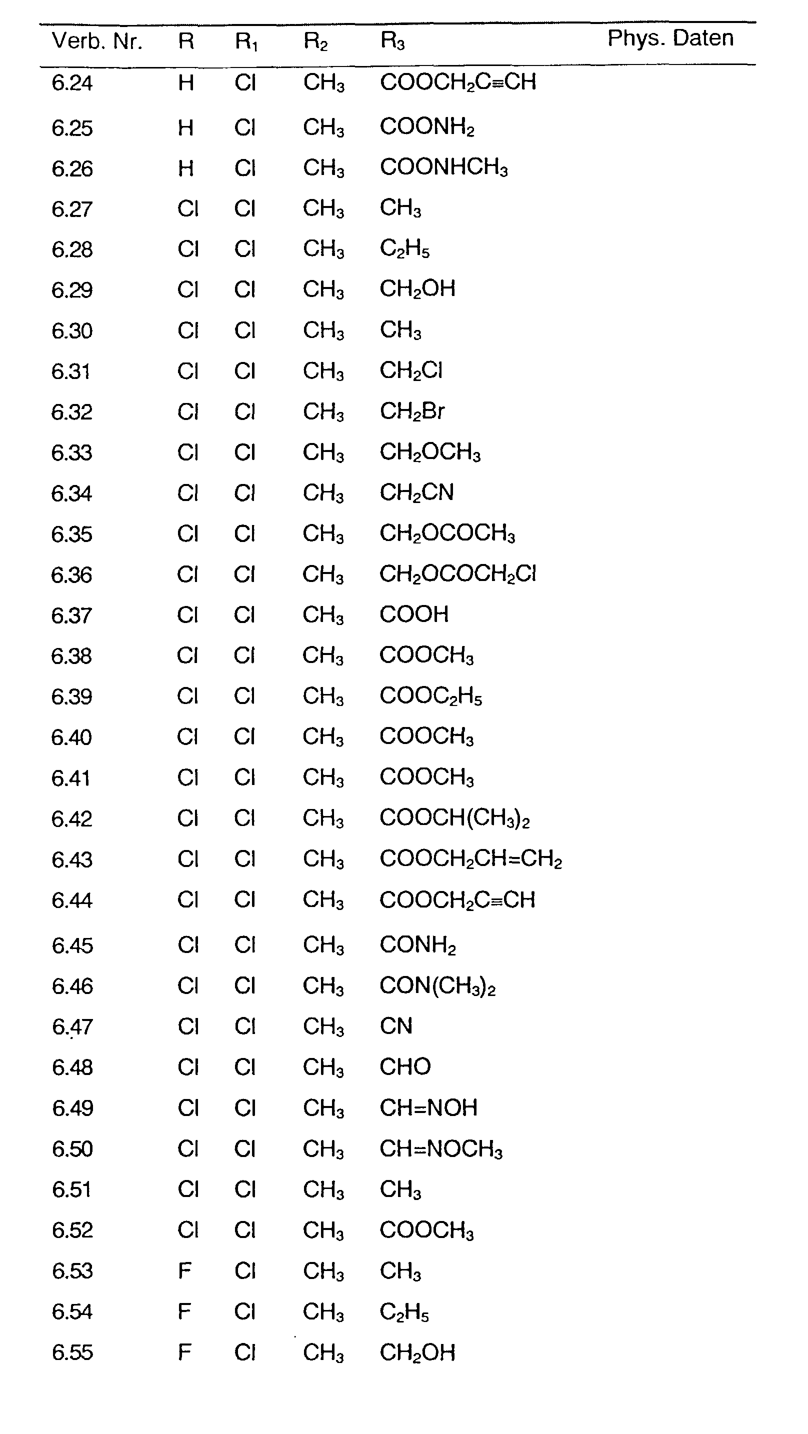

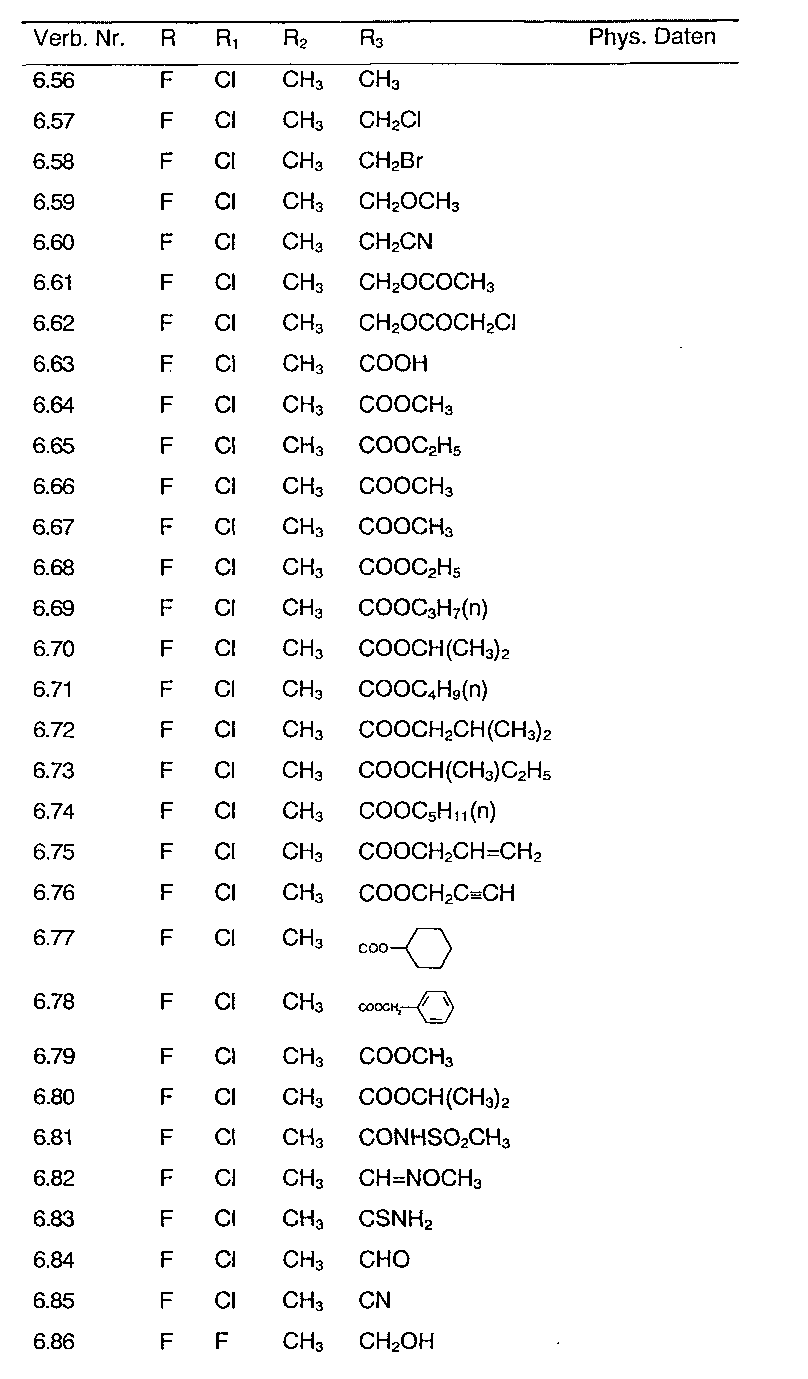

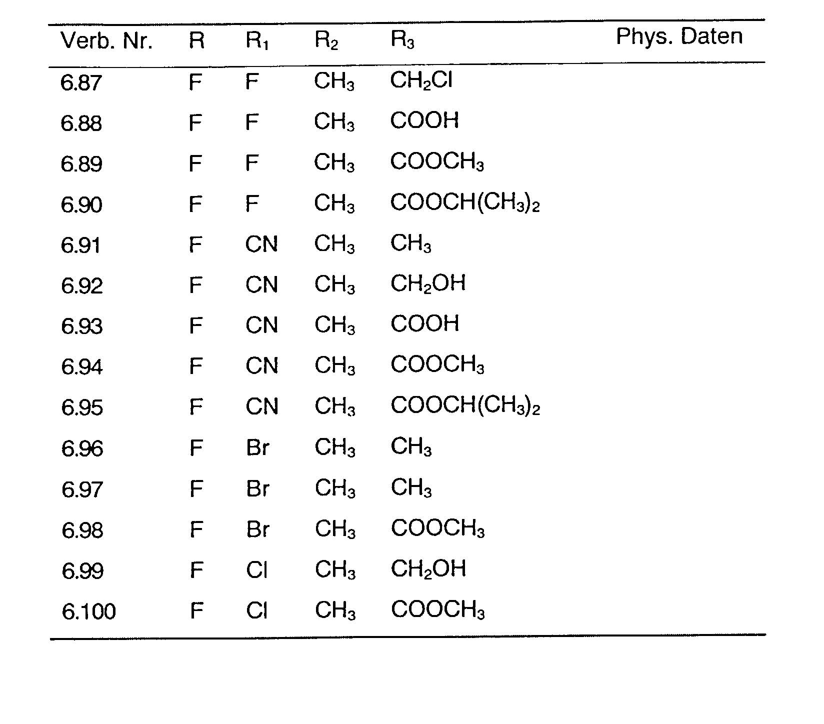

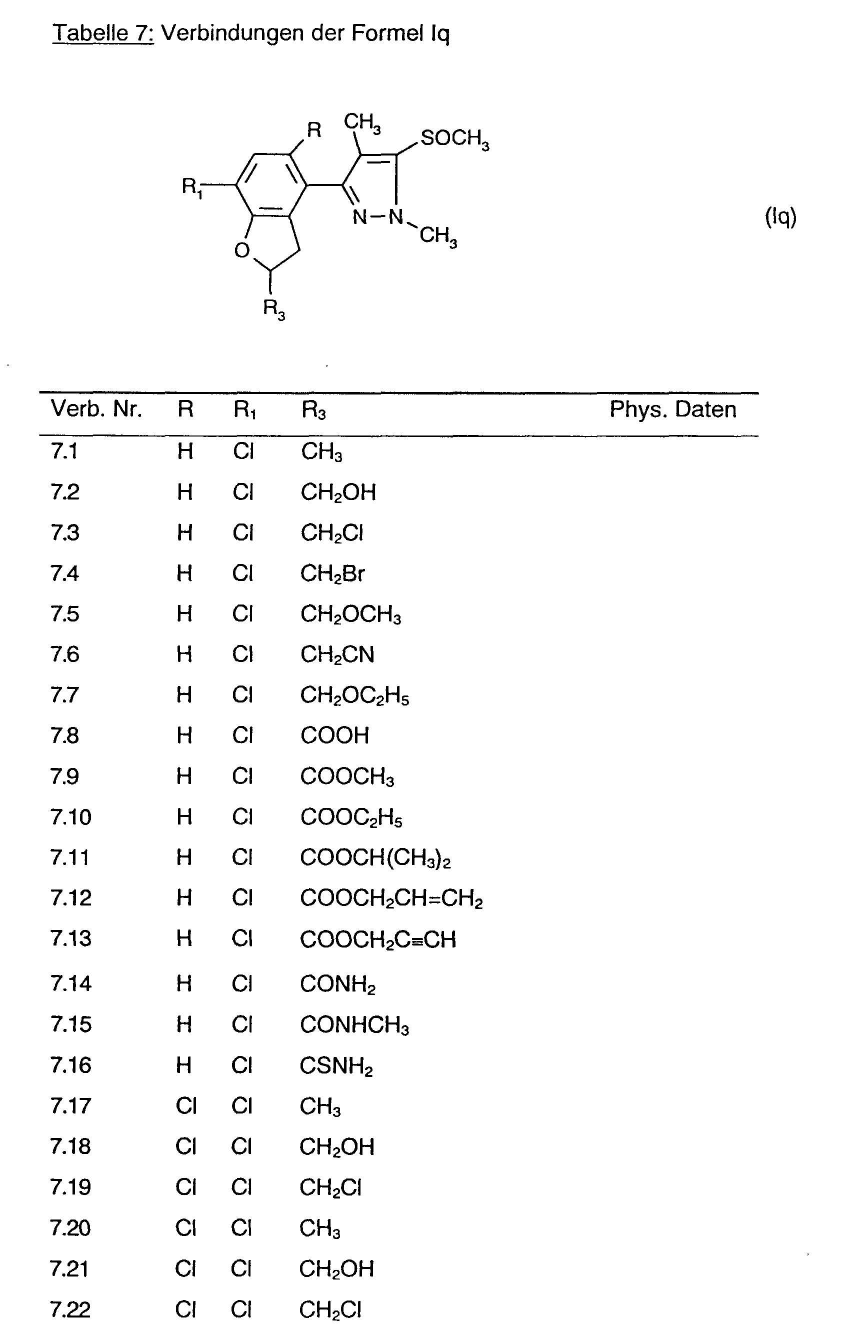

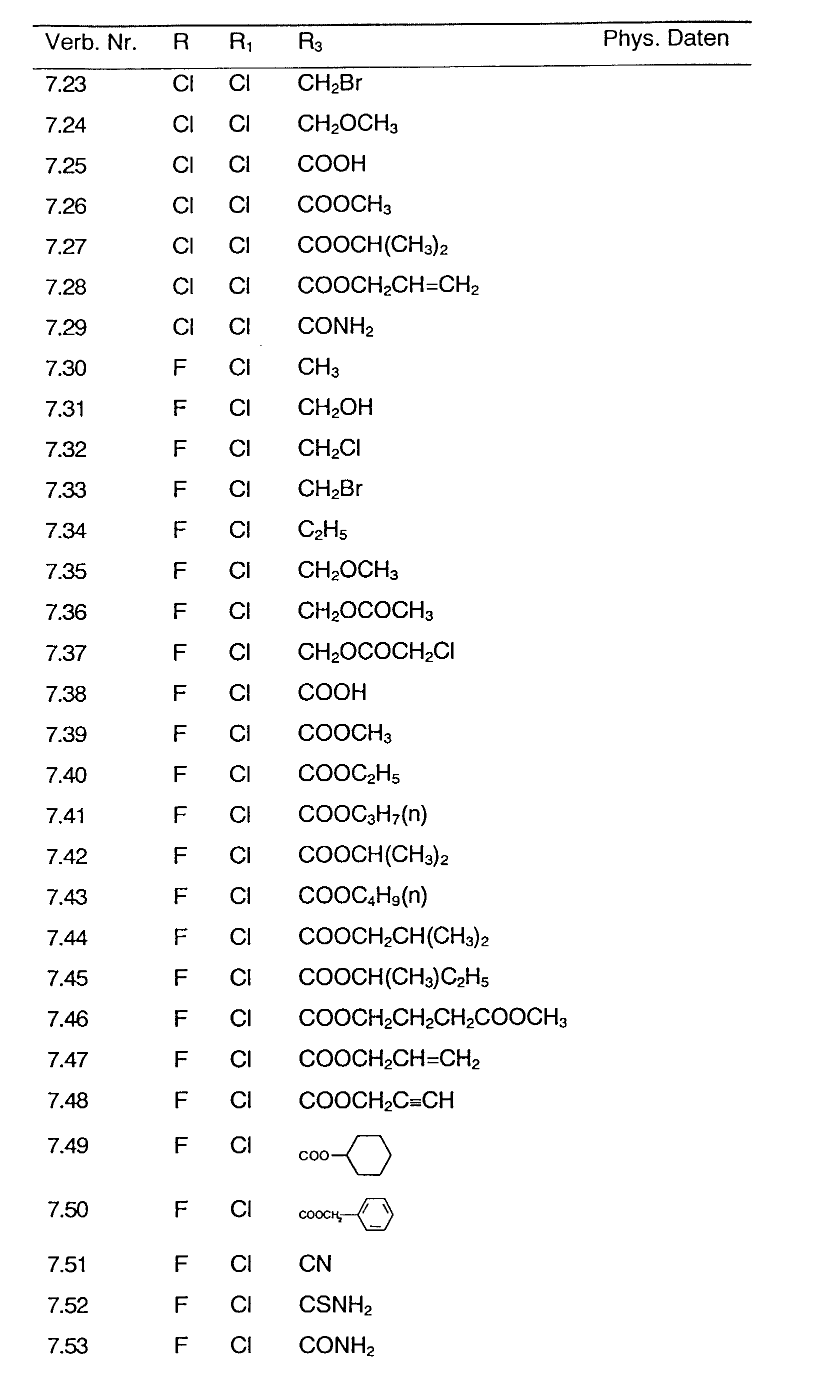

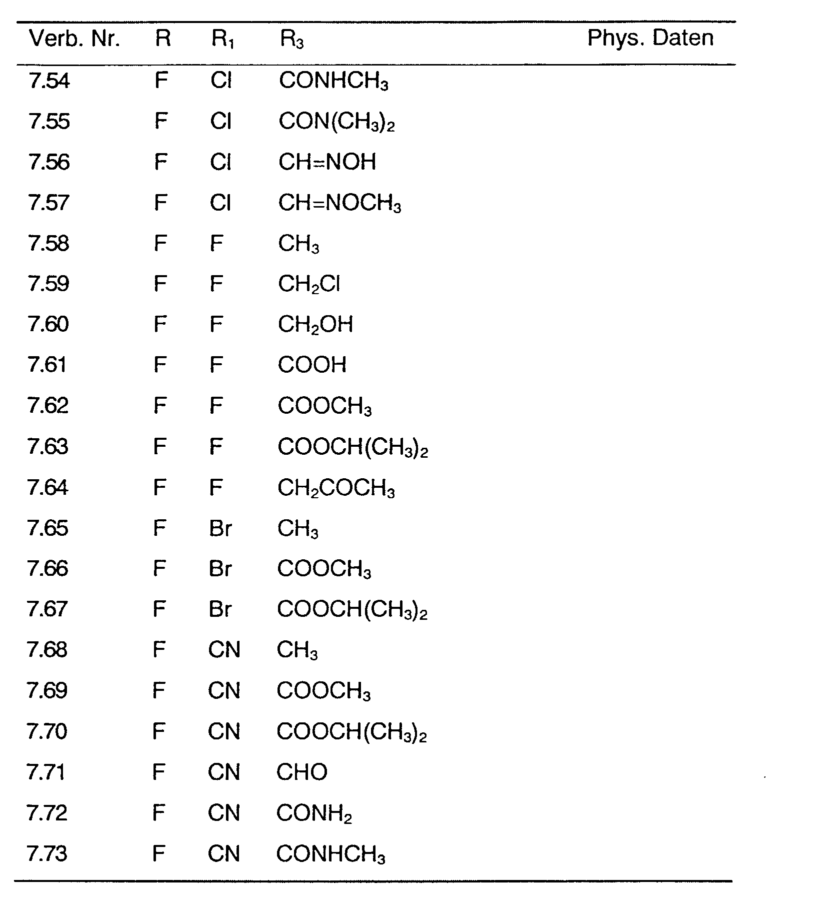

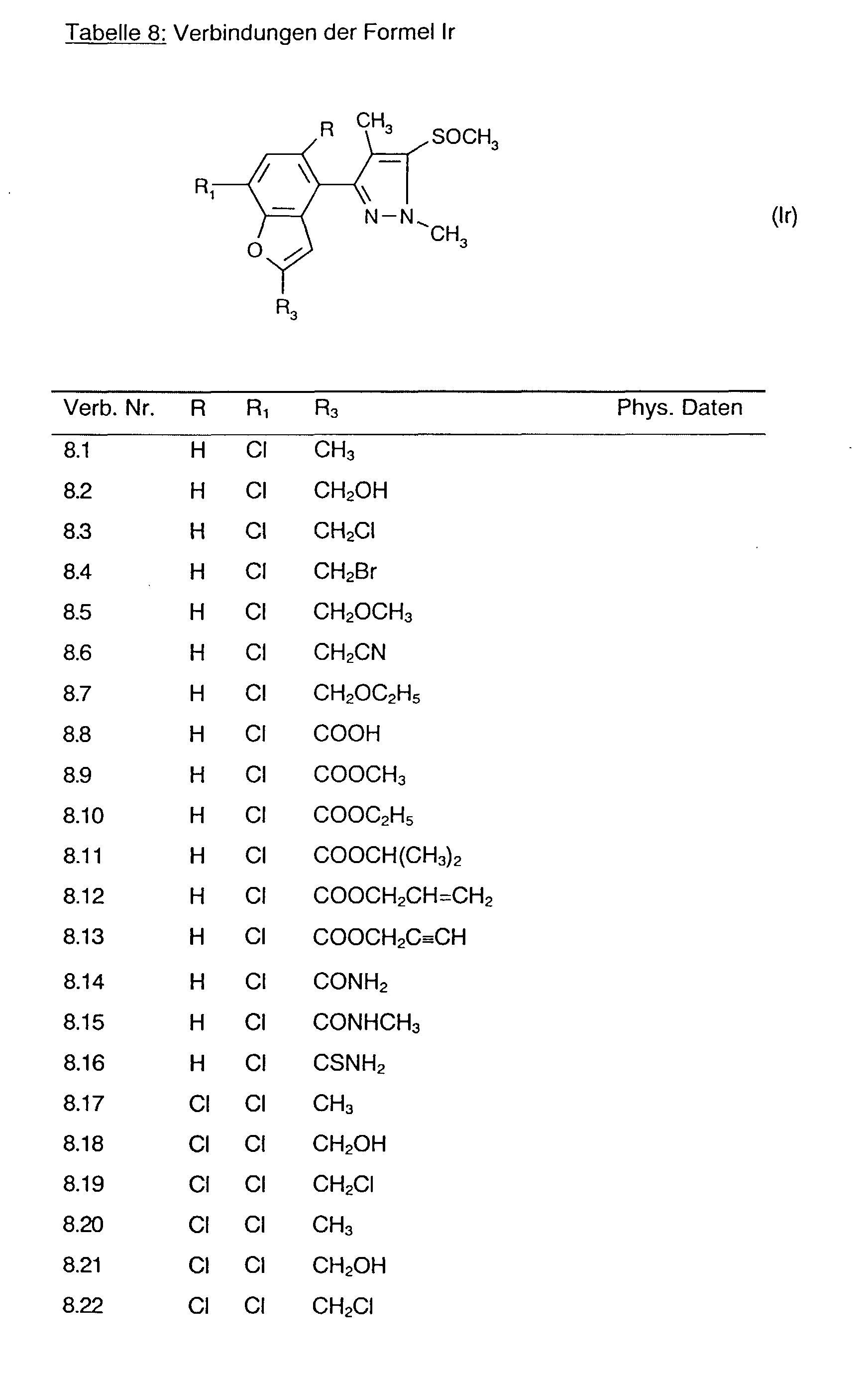

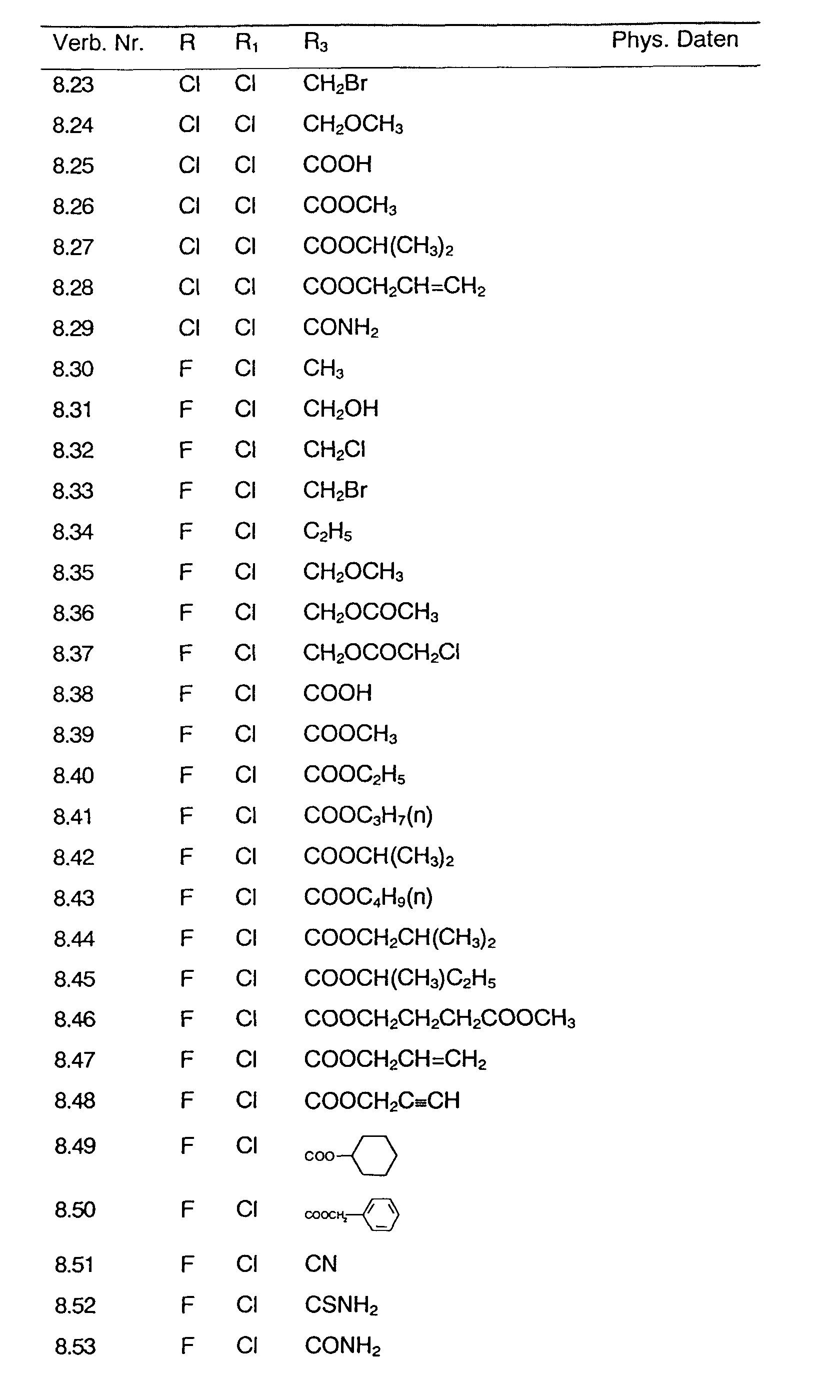

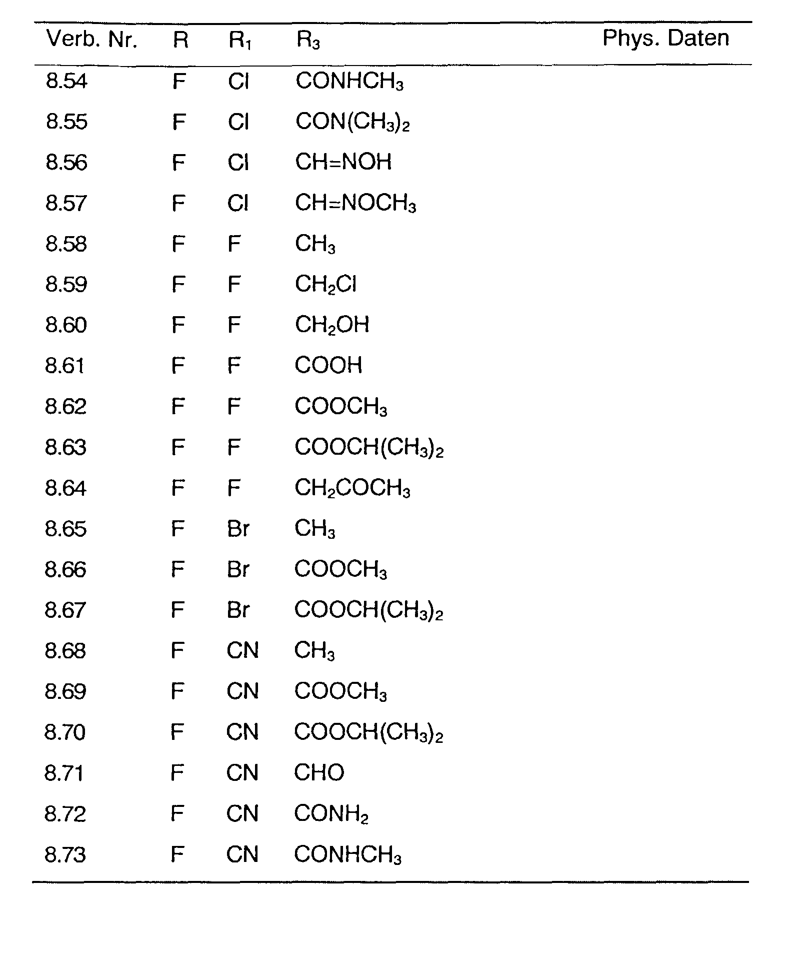

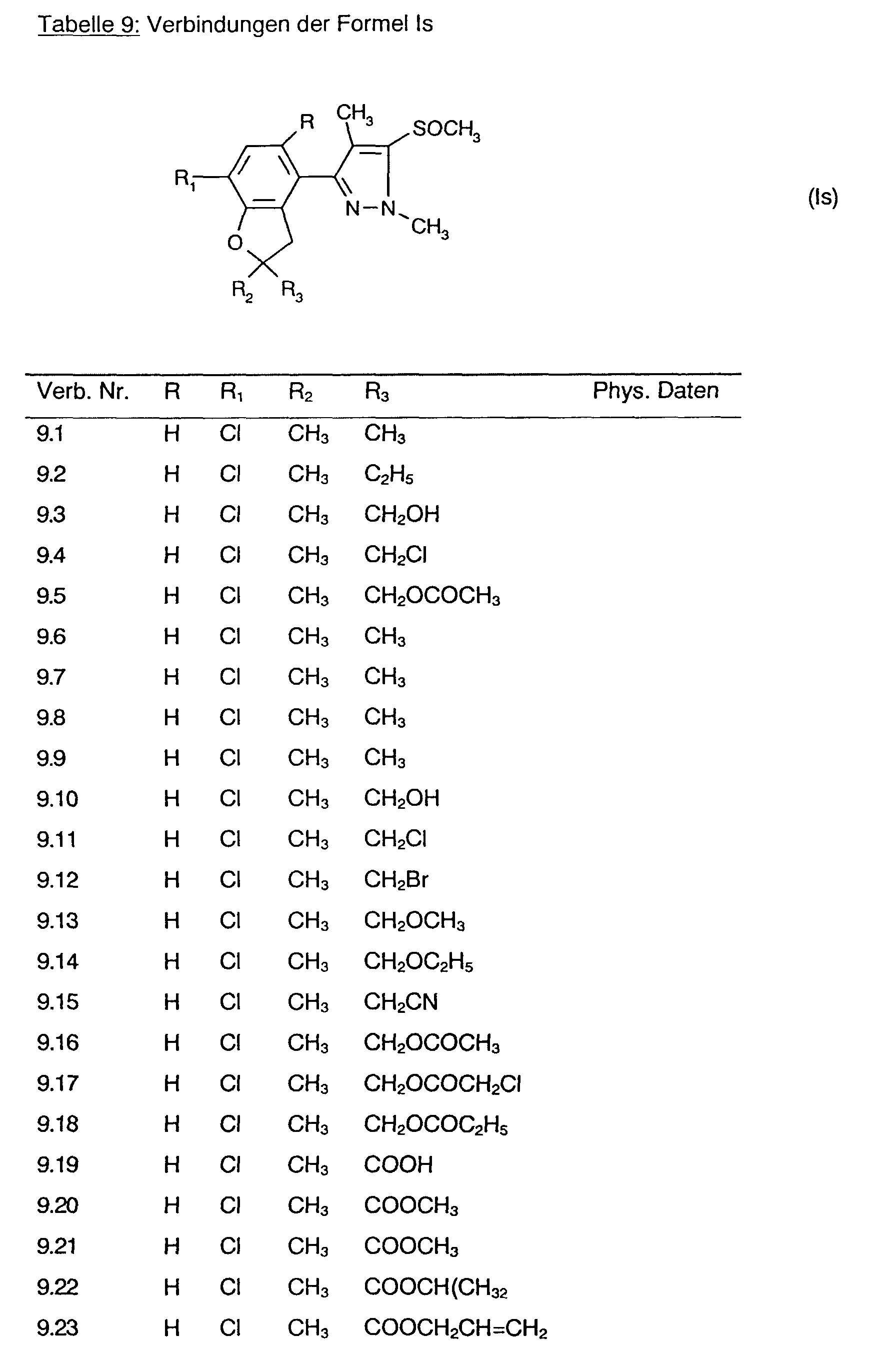

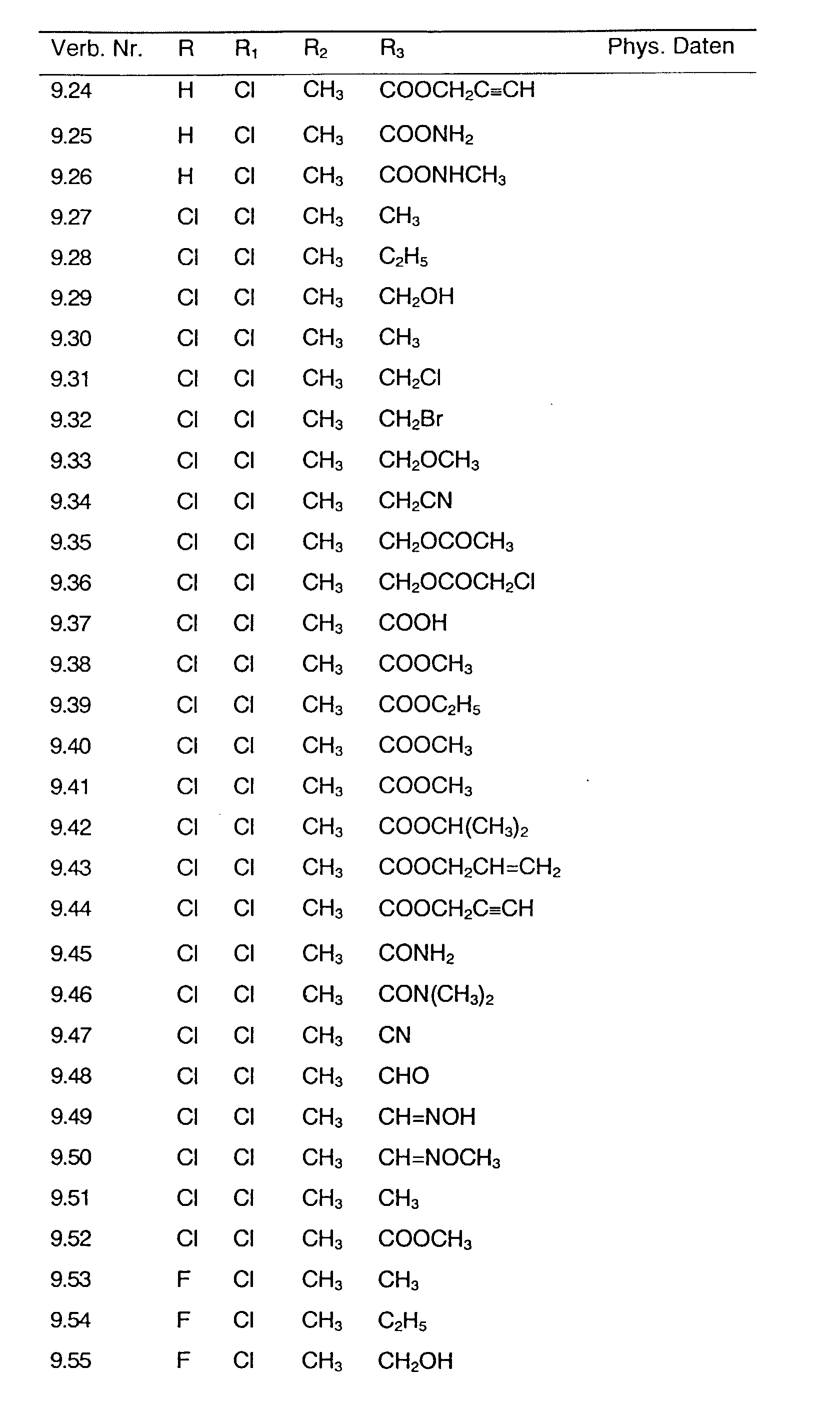

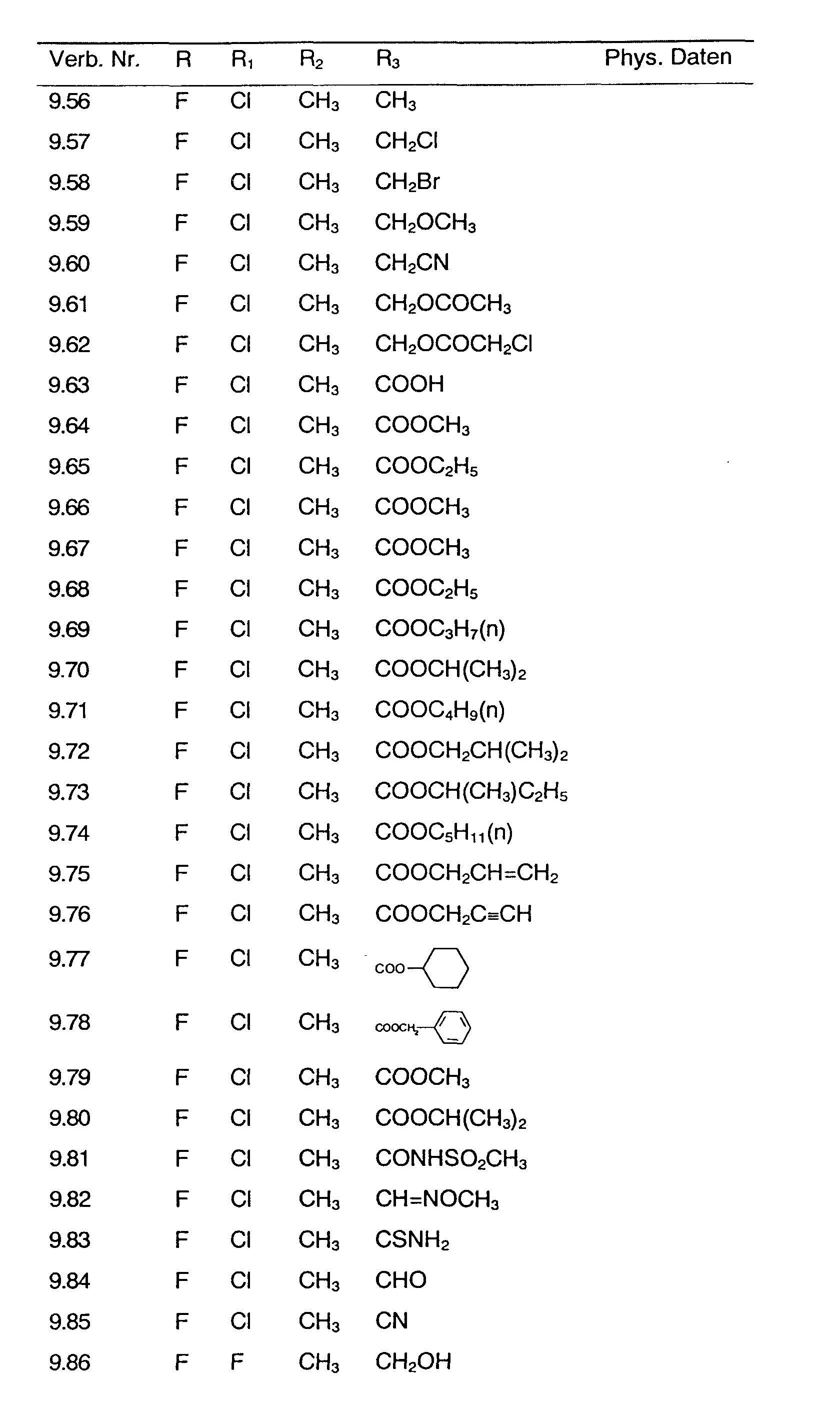

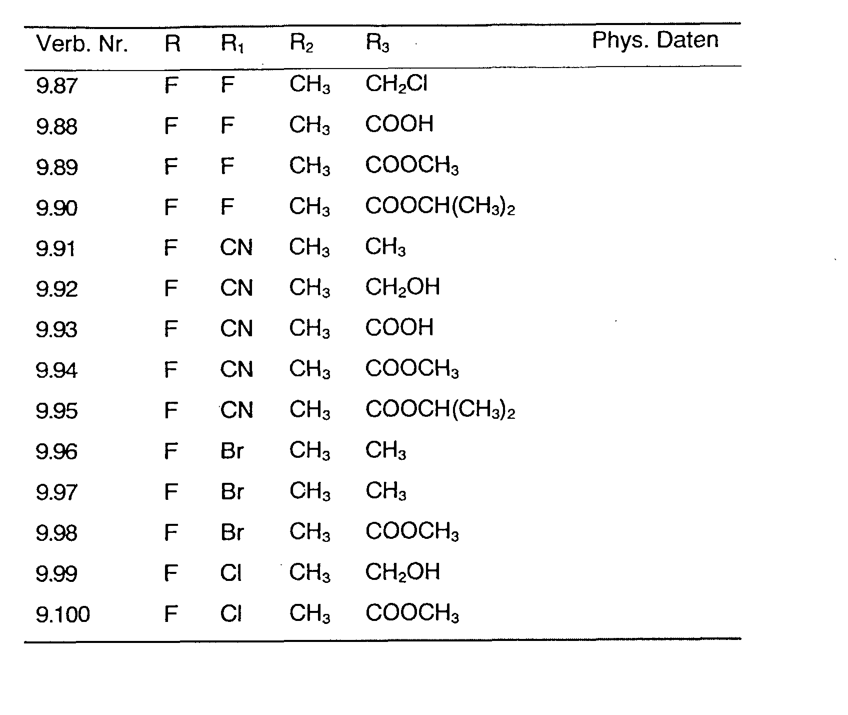

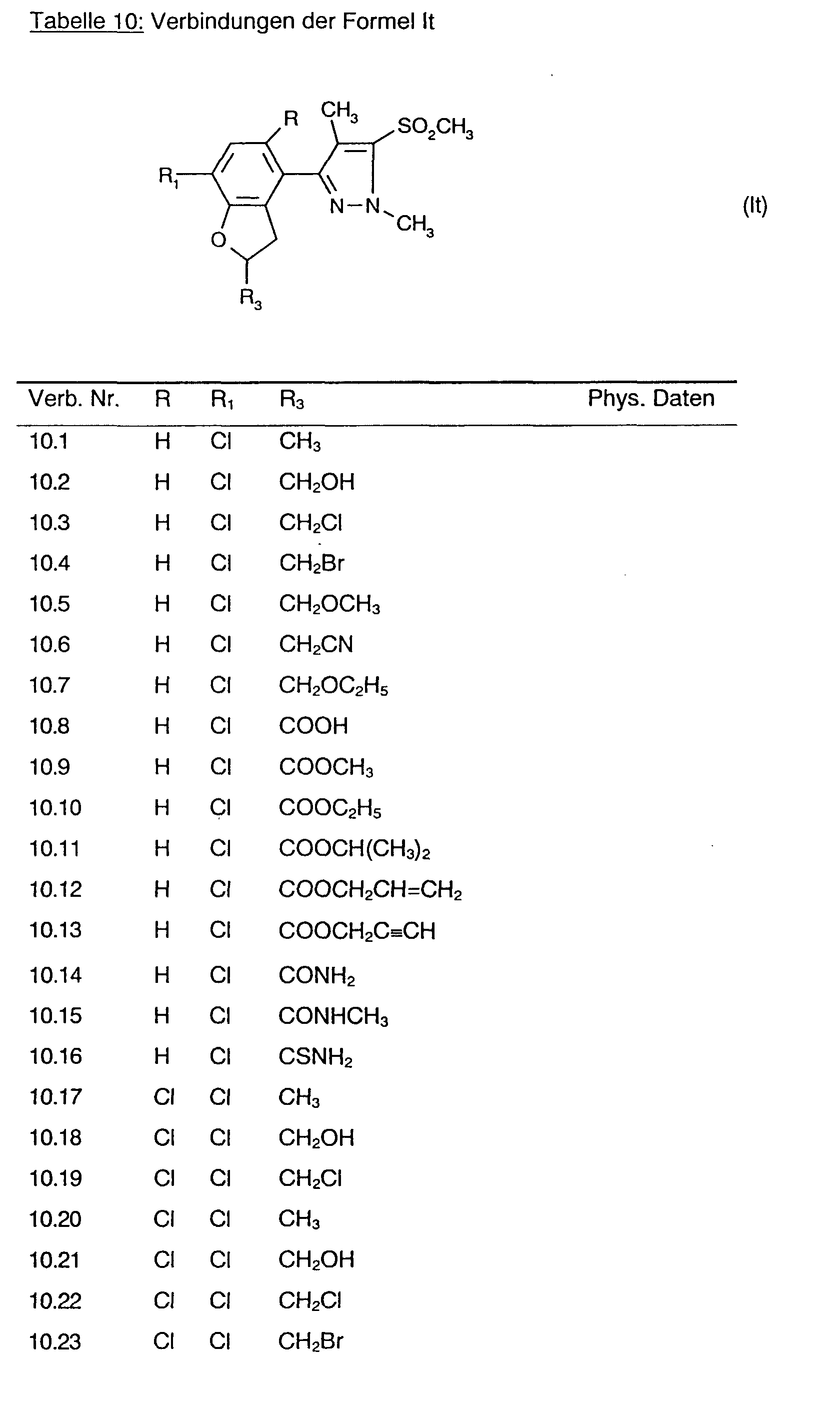

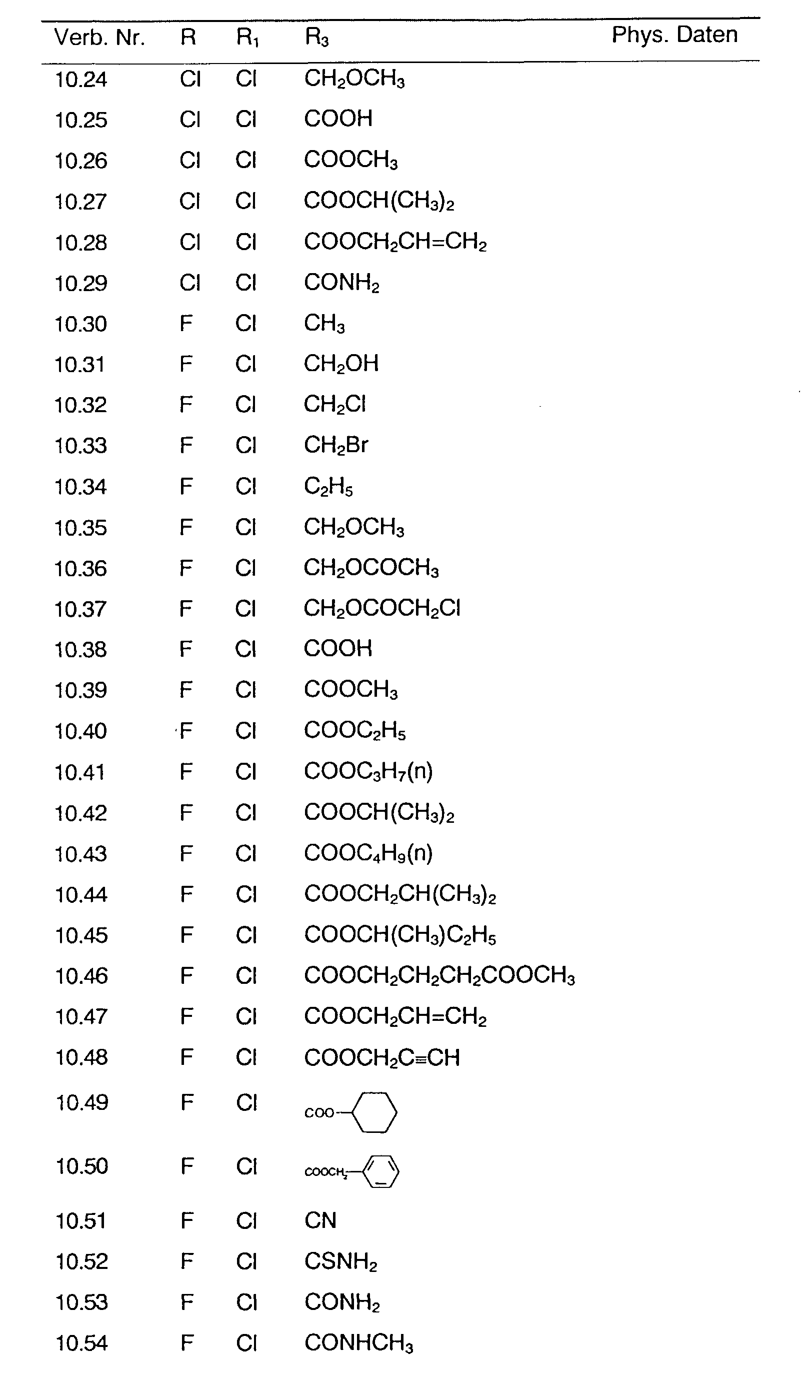

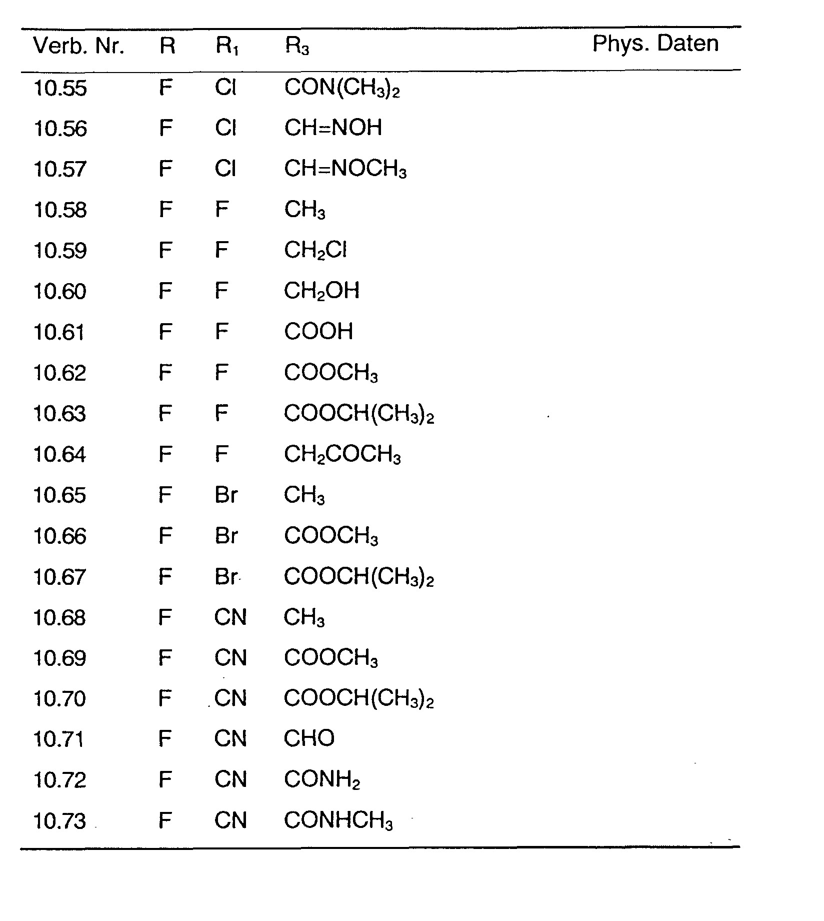

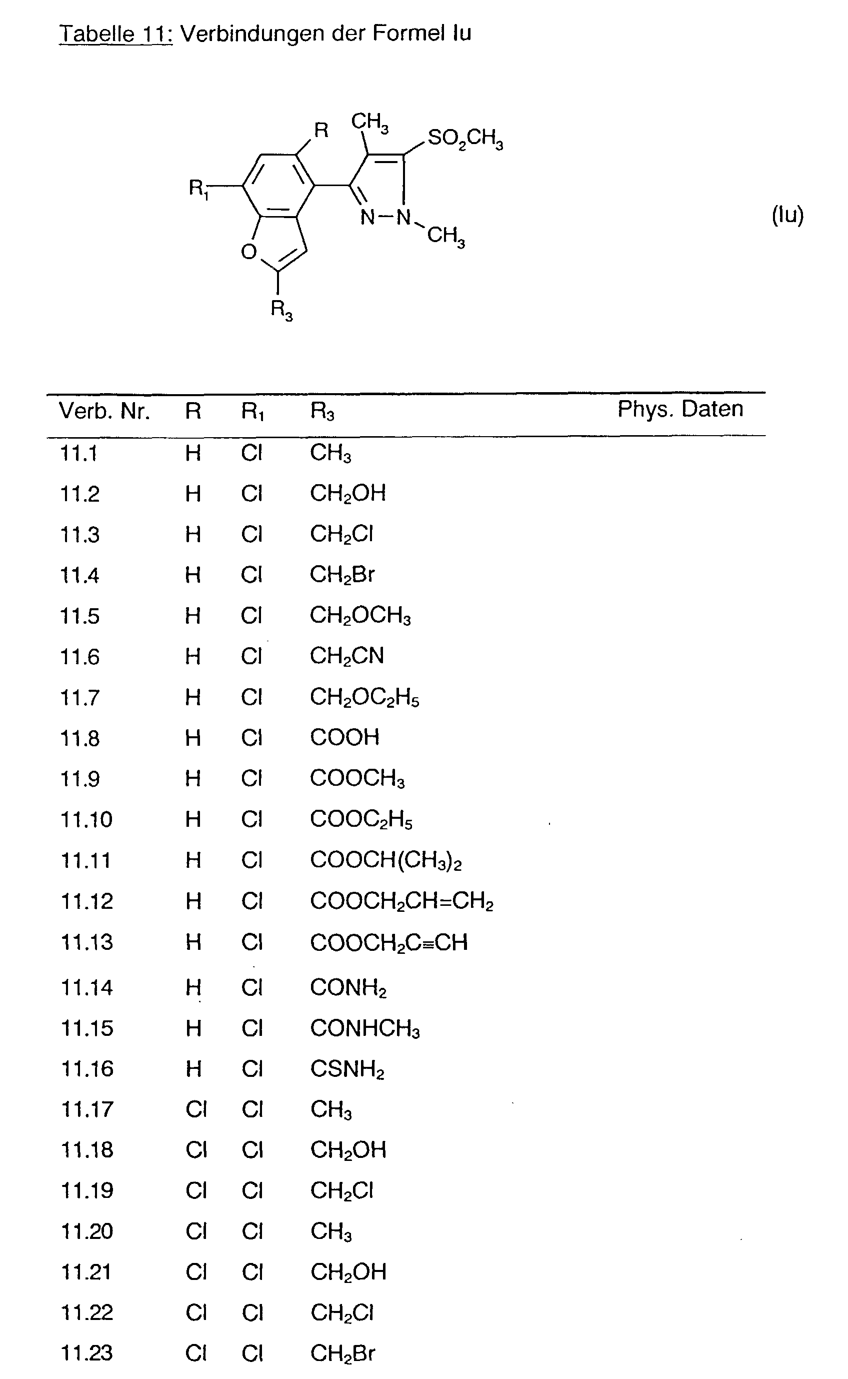

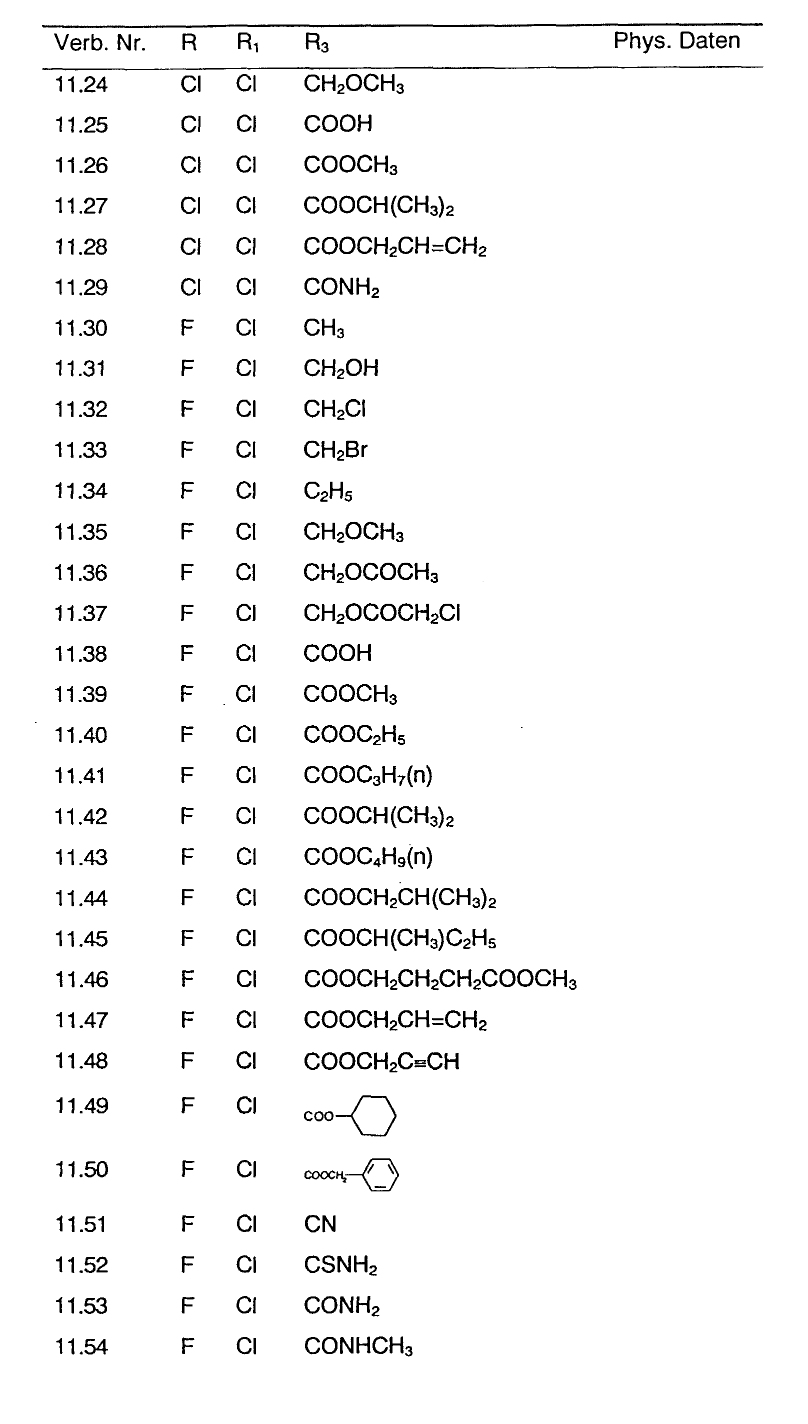

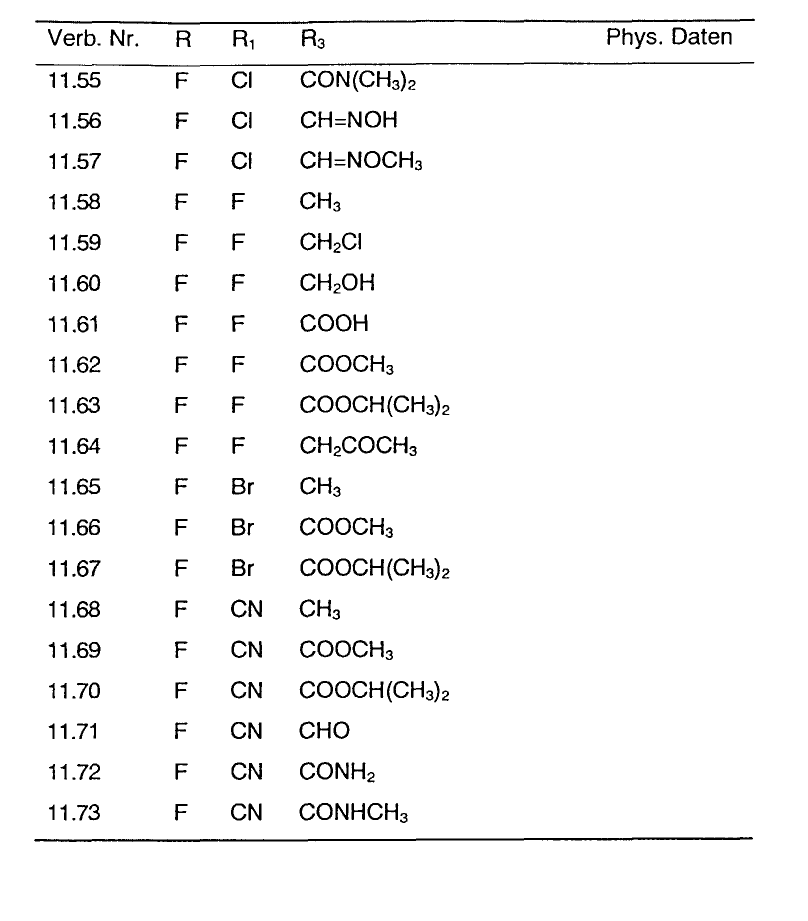

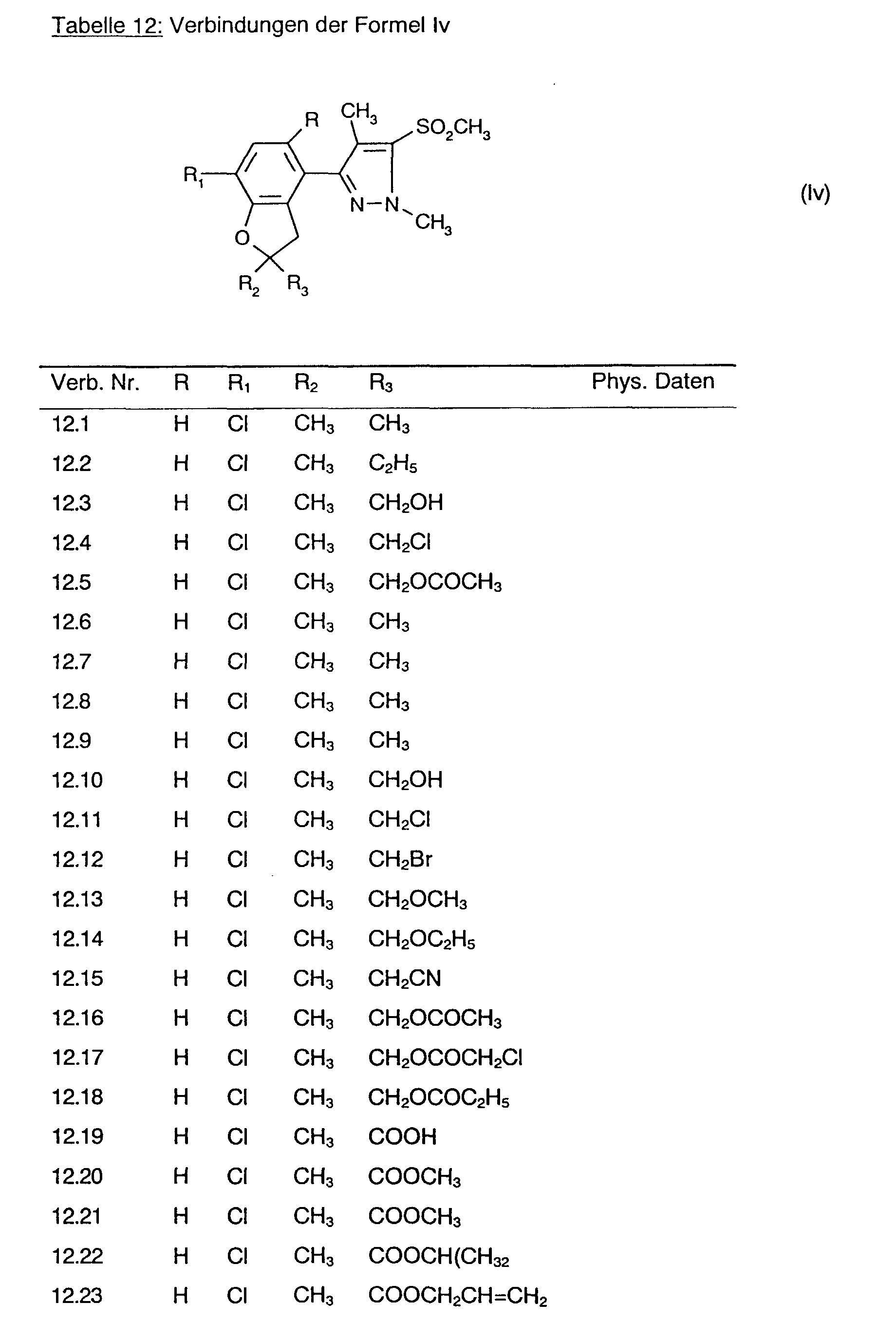

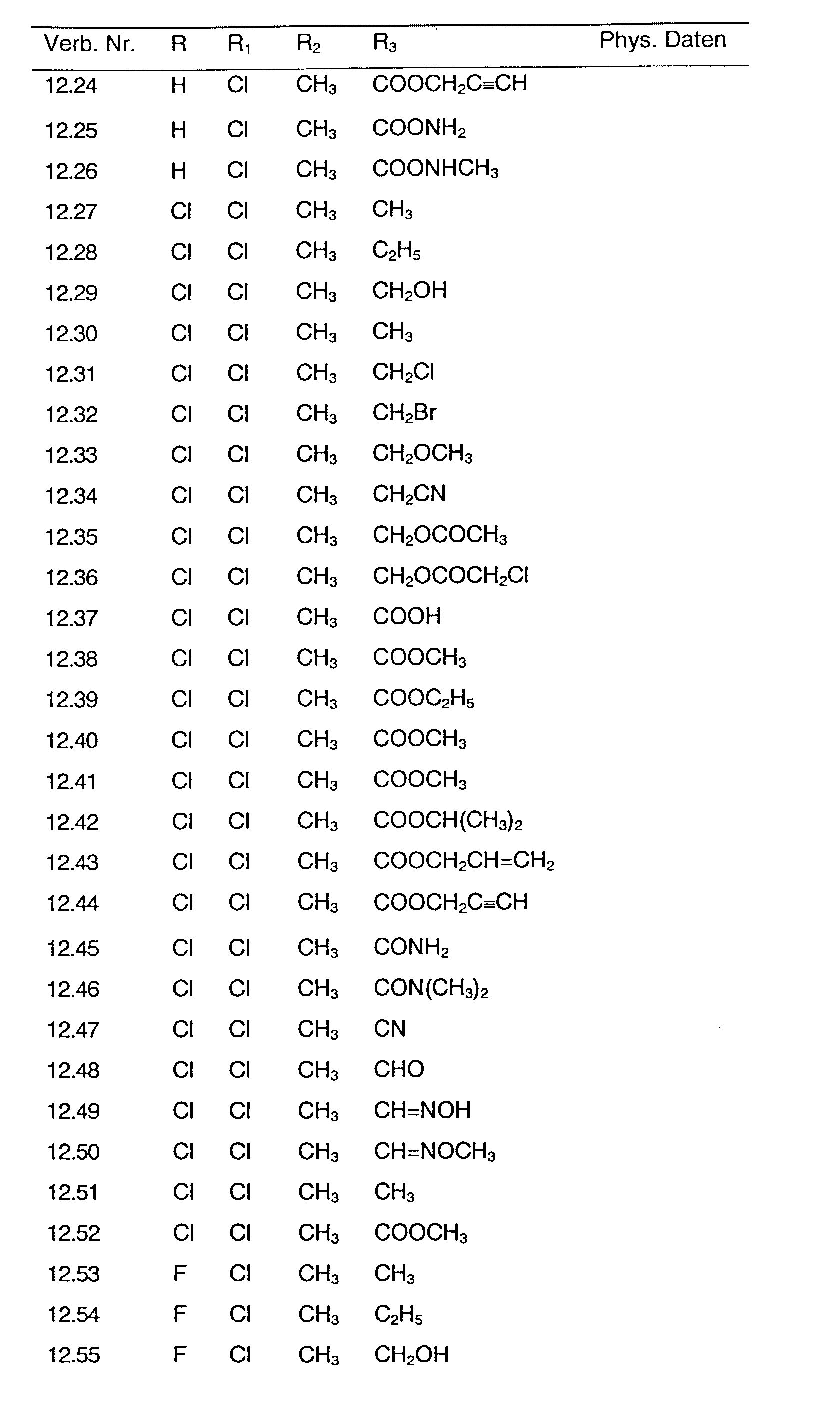

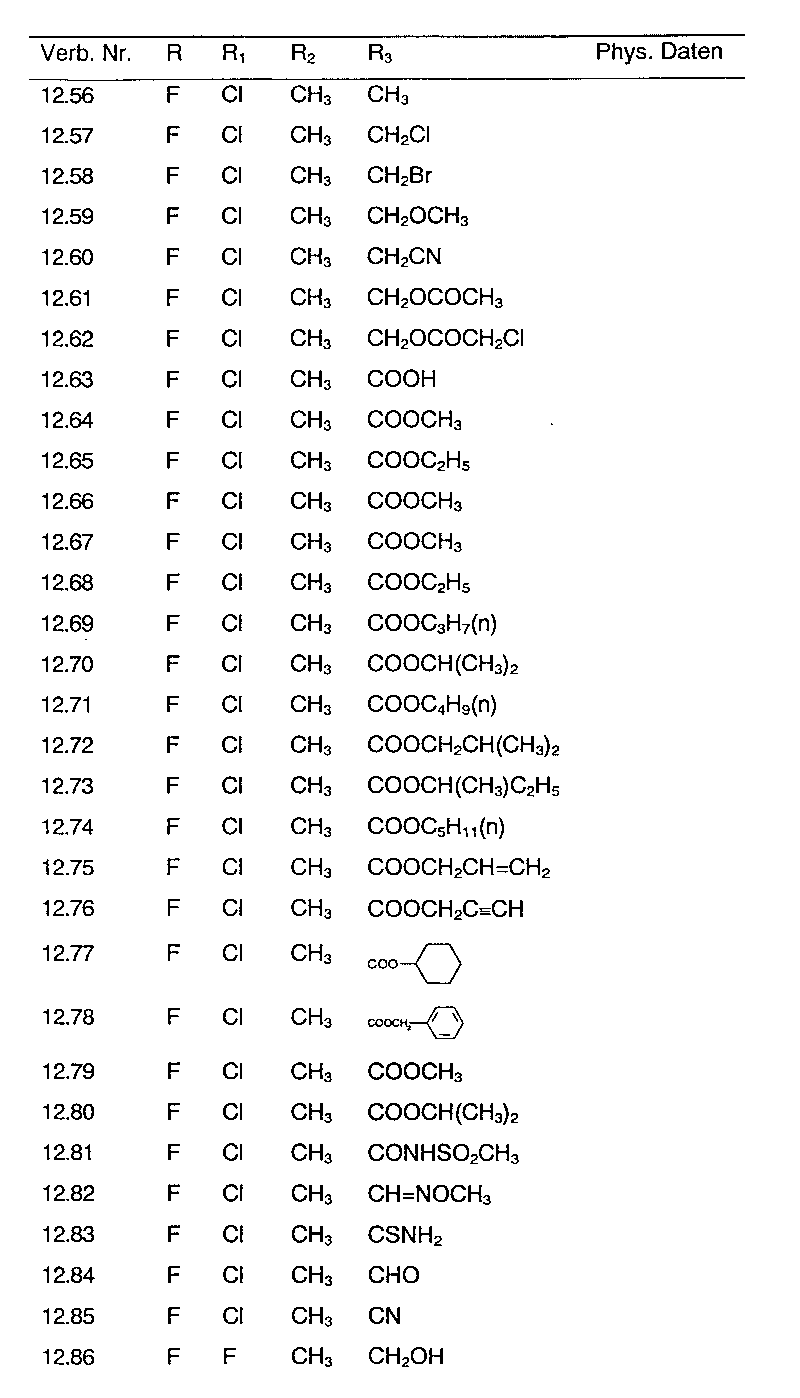

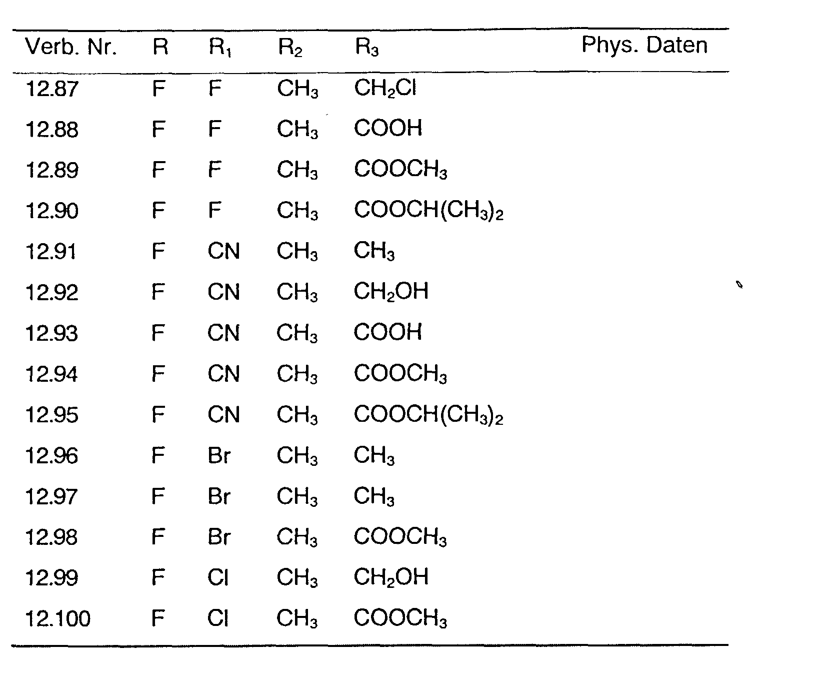

### Formulierungsbeispiele für Wirkstoffe der Formel I

### (% = Gewichtsprozent)

| F1. Emulsionskonzentrate | a) | b) | c) | d) |
|---|---|---|---|---|
| | | | | |
| Wirkstoff gem. Tabellen 1-12 | 5 % | 10 % | 25 % | 50 % |
| Ca-Dodecylbenzolsulfonat | 6 % | 8 % | 6 % | 8 % |
| Ricinusöl-polyglykolether (36 Mol EO) | 4 % | - | 4 % | 4 % |
| Octylphenol-polyglykolether (7-8 Mol EO) | - | 4 % | - | 2 % |
| Cyclohexanon | - | - | 10 % | 20 % |
| Arom. Kohlenwasserstoffgemisch C₉-C₁₂ | 85 % | 78 % | 55 % | 16 % |

Aus solchen Konzentraten können durch Verdünnung mit Wasser Emulsionen jeder gewünschten Konzentration hergestellt werden.

| F2. Lösungen | a) | b) | c) | d) |
|---|---|---|---|---|
| | | | | |
| Wirkstoff gem. Tabellen 1-12 | 5 % | 10 % | 50 % | 90 % |
| 1-Methoxy-3-(3-methoxypropoxy)-propan | - | 20 % | 20 % | - |
| Polyethylenglykol MG 400 | 20 % | 10 % | - | - |
| N-Methyl-2-pyrrolidon | - | - | 30 % | 10 % |
| Arom. Kohlenwasserstoffgemisch C₉-C₁₂ | 75 % | 60 % | - | - |

Die Lösungen sind zur Anwendung in Form kleinster Tropfen geeignet.

| F3. Spritzpulver | a) | b) | c) | d) |
|---|---|---|---|---|
| | | | | |
| Wirkstoff gem. Tabellen 1-12 | 5 % | 25 % | 50 % | 80 % |
| Na-Ligninsulfonat | 4 % | - | 3 % | - |
| Na-Laurylsulfat | 2 % | 3 % | - | 4 % |
| Na-Diisobutyl-naphthalinsulfonat | - | 6 % | 5 % | 6 % |
| Octylphenol-polyglykolether (7-8 Mol EO) | - | 1 % | 2 % | - |
| Hochdisperse Kieselsäure | 1 % | 3 % | 5 % | 10 % |
| Kaolin | 88 % | 62 % | 35 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| F4. Umhüllungs-Granulate | a) | b) | c) |
|---|---|---|---|
| | | | |
| Wirkstoff gem. Tabellen 1-12 | 0.1 % | 5 % | 15 % |
| Hochdisperse Kieselsäure | 0.9 % | 2 % | 2 % |
| Anorg. Trägermaterial | 99.0 % | 93 % | 83 % |
| (Ø 0.1 - 1 mm) | | | |
| wie z.B. CaCO₃ oder SiO₂ | | | |

Der Wirkstoff wird in Methylenchlorid gelöst, auf den Träger aufgesprüht und das Lösungsmittel anschließend im Vakuum abgedampft.

| F5. Umhüllungs-Granulate | a) | b) | c) |
|---|---|---|---|
| | | | |
| Wirkstoff gem. Tabellen 1-12 | 0.1 % | 5 % | 15 % |
| Polyethylenglykol MG 200 | 1.0 % | 2 % | 3 % |
| Hochdisperse Kieselsäure | 0.9 % | 1 % | 2 % |
| Anorg. Trägermaterial | 98.0 % | 92 % | 80 % |
| (Ø 0.1 - 1 mm) | | | |
| wie z.B. CaCO₃ oder SiO₂ | | | |

Der fein gemahlene Wirkstoff wird in einem Mischer auf das mit Polyethylenglykol angefeuchtete Trägermaterial gleichmäßig aufgetragen. Auf diese Weise erhält man staubfreie Umhüllungs-Granulate.

| F6. Extruder-Granulate | a) | b) | c) | d) |
|---|---|---|---|---|
| | | | | |
| Wirkstoff gem. Tabellen 1-12 | 0.1 % | 3 % | 5 % | 15 % |
| Na-Ligninsulfonat | 1.5 % | 2 % | 3 % | 4 % |
| Carboxymethylcellulose | 1.4 % | 2 % | 2 % | 2 % |
| Kaolin | 97.0 % | 93 % | 90 % | 79 % |

Der Wirkstoff wird mit den Zusatzstoffen vermischt, vermahlen und mit Wasser angefeuchtet. Dieses Gemisch wird extrudiert und anschließend im Luftstrom getrocknet.

| F7. Stäubemittel | a) | b) | c) |
|---|---|---|---|
| | | | |
| Wirkstoff gem. Tabellen 1-12 | 0.1 % | 1 % | 5 % |
| Talkum | 39.9 % | 49 % | 35 % |
| Kaolin | 60.0 % | 50 % | 60 % |

Man erhält anwendungsfertige Stäubemittel, indem der Wirkstoff mit den Trägerstoffen vermischt und auf einer geeigneten Mühle vermahlen wird.

| F8. Suspensions-Konzentrate | a) | b) | c) | d) |
|---|---|---|---|---|
| | | | | |
| Wirkstoff gem. Tabellen 1-12 | 3 % | 10 % | 25 % | 50 % |
| Ethylenglykol | 5 % | 5 % | 5 % | 5 % |
| Nonylphenol-polyglykolether | - | 1 % | 2 % | - |
| (15 Mol EO) | | | | |
| Na-Ligninsulfonat | 3 % | 3 % | 4 % | 5 % |
| Carboxymethylcellulose | 1 % | 1 % | 1 % | 1 % |
| 37%ige wäßrige Formaldehyd- Lösung | 0.2 % | 0.2 % | 0.2 % | 0.2 % |
| Silikonöl-Emulsion | 0.8 % | 0.8 % | 0.8 % | 0.8 % |
| Wasser | 87 % | 79 % | 62 % | 38 % |

Der feingemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

### Biologische Beispiele

### Beispiel B1: Herbizidwirkung vor dem Auflaufen der Pflanzen (pre-emergente Wirkung)

Monokotyle und dikotyle Testpflanzen werden in Kunststofftöpfen in Standarderde angesät. Unmittelbar nach der Saat werden die Prüfsubstanzen als wäßrige Suspension oder Emulsion, hergestellt aus einem 25 %igen Emulsionskonzentrat (Beispiel F1, c)), entsprechend der Dosierung von 500 g AS/ha (Nr. 1.59, 1.68, 1.74 und 1.76) bzw. 2000 g AS/ha (Nr. 1.77, 1.78 und 1.80) aufgesprüht (500 l Wasser/ha). Anschließend werden die Testpflanzen im Gewächshaus unter optimalen Bedingungen kultiviert. Nach 3 Wochen Testdauer wird der Versuch mit einer neunstufigen Notenskala ausgewertet (1 = vollständige Schädigung, 9 = keine Wirkung). Boniturnoten von 1 bis 4 (insbesondere 1 bis 3) bedeuten eine gute bis sehr gute Herbizidwirkung.

Testpflanzen: Setaria, Sinapis, Solanum, Stellaria, Ipomoea.

Die erfindungsgemässen Verbindungen zeigen gute Herbizidwirkung.

Beispiele für die gute herbizide Wirksamkeit der Verbindungen der Formel I gibt die Tabelle B1.

**Tabelle B1:**

| Pre-emergente Wirkung: | | | | | |
|---|---|---|---|---|---|
| Testpflanze: | Setaria | Sinapis | Solanum | Stellaria | Ipomoea |
| Wirkstoff Nr. | | | | | |
| 1.59 | 1 | 2 | 1 | 1 | 4 |
| 1.68 | 3 | 7 | 1 | 2 | 4 |
| 1.74 | 4 | 2 | 1 | 2 | 4 |
| 1.76 | 4 | 7 | 1 | 4 | 7 |
| 1.77 | 1 | 3 | -- | 1 | -- |
| 1.78 | 1 | 3 | -- | 1 | -- |
| 1.80 | 1 | 1 | -- | 1 | -- |

Dieselben Resultate werden erhalten, wenn man die Verbindungen der Formel I gemäß den Beispielen F2 bis F8 formuliert.

### Beispiel B2: Post-emergente Herbizid-Wirkung

Monokotyle und dikotyle Testpflanzen werden im Gewächshaus in Kunststofftöpfen mit Standarderde angezogen und im 4- bis 6-Blattstadium mit einer wäßrigen Suspension oder Emulsion der Prüfsubstanzen der Formel I, hergestellt aus einem 25 %igen Emulsionskonzentrat (Beispiel F1, c)), besprüht, entsprechend einer Dosierung von 500 g AS/ha (Nr. 1.59, 1.68, 1.74 und 1.76) bzw. 2000 g AS/ha (Nr. 1.77, 1.78 und 1.80) (500 l Wasser/ha). Anschließend werden die Testpflanzen im Gewächshaus unter optimalen Bedingungen weiterkultiviert. Nach ca. 18 Tagen Testdauer wird der Versuch ausgewertet mit einer neunstufigen Notenskala (1 = vollständige Schädigung, 9 = keine Wirkung). Boniturnoten von 1 bis 4 (insbesondere 1 bis 3) bedeuten eine gute bis sehr gute Herbizidwirkung.

Testpflanzen: Setaria, Sinapis, Solanum, Stellaria, Ipomoea.

Auch in diesem Versuch zeigen die Verbindungen der Formel I starke Herbizidwirkung.

Beispiele für die gute herbizide Wirksamkeit der Verbindungen der Formel I gibt die Tabelle B2.

**Tabelle B2:**

| Post-emergente Wirkung: | | | | | |
|---|---|---|---|---|---|
| Testpflanze: | Setaria | Sinapis | Solanum | Stellaria | Ipomoea |
| Wirkstoff Nr. | | | | | |
| 1.59 | 1 | 1 | 1 | 1 | 1 |
| 1.68 | 4 | 1 | 1 | 2 | 1 |
| 1.74 | 4 | 1 | 1 | 3 | 1 |
| 1.76 | 2 | 1 | 1 | 3 | 1 |
| 1.77 | 1 | 1 | -- | 1 | -- |
| 1.78 | 1 | 1 | -- | 1 | -- |
| 1.80 | 1 | 1 | -- | 1 | -- |

Dieselben Resultate werden erhalten, wenn man die Verbindungen der Formel I gemäß den Beispielen F2 bis F8 formuliert.

## Patentansprüche

1. Verbindungen der Formel I 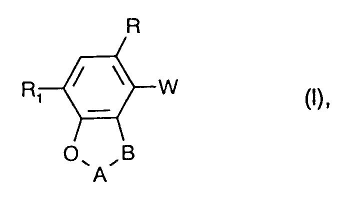 worin
R Wasserstoff, Fluor oder Chlor;
R₁ Wasserstoff, Halogen, Trifluormethyl, C₁-C₄-Halogenalkoxy, Cyano, Nitro oder Amino;
A-B eine Gruppe 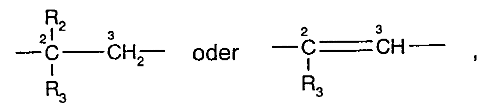 wobei das 2-Kohlenstoffatom an das Sauerstoffatom gebunden ist;
W eine Gruppe 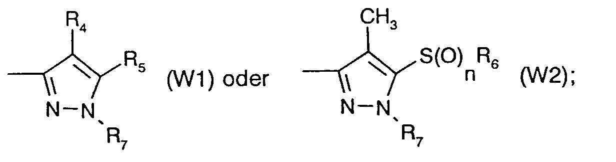
R₂ Wasserstoff oder C₁-C₆-Alkyl;
R₃ C₁-C₆-Alkyl, C₁-C₆-Halogenalkyl, Cyano-C₁-C₄-alkyl, Hydroxy-C₁-C₆-alkyl, C₁-C₆-Alkoxy-C₁-C₆-alkyl, C₃-C₆-Alkenyloxy-C₁-C₄-alkyl, C₃-C₆-Alkinyloxy-C₁-C₄-alkyl, C₁-C₆-Alkylcarbonyloxy-C₁-C₆-alkyl, Carboxyl, C₁-C₆-Alkoxycarbonyl, C₁-C₆-Halogenalkoxycarbonyl, C₃-C₆-Alkenyloxycarbonyl, C₃-C₆-Alkinyloxycarbonyl, C₃-C₆-Cycloalkoxycarbonyl, C₁-C₄-Alkoxy-C₁-C₄-alkoxycarbonyl, C₁-C₄-Alkylaminocarbonyl, Di-C₁-C₄-Alkylaminocarbonyl, Aminocarbonyl, Benzyloxycarbonyl, Phenyloxycarbonyl, C₁-C₄-Alkyl-SO₂NHC(O)-, C₁-C₆-Alkyl-ON=CH-, C₁-C₆-Alkoxycarbonyl-C₁-C₄-alkyl, C₁-C₃-Alkylcarbonyl, CIC(O)-, NH₂C(S)-, OHC- oder Cyano;
R₄ Wasserstoff, Fluor, Chlor oder Brom;
R₅ Carboxyl, C₁-C₆-Alkoxycarbonyl, NH₂C(O)-, NH₂C(S)-, HON=CH-, OHC- oder Cyano;
R₆ Wasserstoff oder C₁-C₄-Alkyl;
n 0, 1 oder 2, mit der Massgabe, dass n 0 ist, wenn R₆ Wasserstoff bedeutet; und
R₇ Wasserstoff oder C₁-C₄-Alkyl ist,
sowie agronomisch verträgliche Salze und Stereoisomere dieser Verbindungen.

2. Verbindungen nach Anspruch 1, worin R₁ Wasserstoff, Fluor, Chlor, Brom, Trifluormethyl oder Cyano ist.

3. Verbindungen nach Anspruch 1, worin R Wasserstoff oder Fluor ist.

4. Verbindungen nach Anspruch 3, worin R Fluor ist.

5. Verbindungen nach Anspruch 1, worin W eine Gruppe 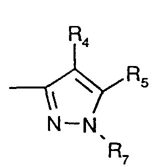 (W1) ist; und R₄, R₅ und R₇ die in Anspruch 1 angegebene Bedeutung haben.

6. Verbindungen nach Anspruch 5, worin R₇ Wasserstoff, Methyl oder Ethyl ist.

7. Verbindungen nach Anspruch 5, worin R₅ Carboxyl, C₁-C₃-Alkoxycarbonyl, NH₂C(S)-, HON=CH-, OHC- oder Cyano ist.

8. Verbindungen nach Anspruch 7, worin R₅ Cyano ist.

9. Verbindungen nach Anspruch 5, worin R₄ Wasserstoff oder Chlor ist.

10. Verbindungen nach Anspruch 1, worin W eine Gruppe 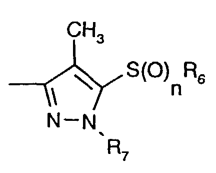 (W2) ist; und R₆, R₇ und n die in Anspruch 1 angegebene Bedeutung haben.

11. Verbindungen nach Anspruch 10, worin n 0 oder 2 ist.

12. Verbindungen nach Anspruch 10, worin R₆ Methyl ist.

13. Verbindungen nach Anspruch 10, worin R₇ Methyl oder Ethyl ist.

14. Verbindungen nach Anspruch 13, worin R₇ Methyl ist.

15. Verbindungen nach Anspruch 1, worin A-B eine Gruppe 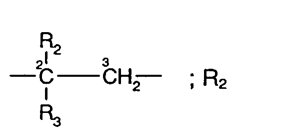 Wasserstoff oder Methyl; und R₃ Wasserstoff, C₁-C₃-Alkyl, C₁- oder C₂-Halogenalkyl, Cyano-C₁-C₄-alkyl, C₁-C₃-Alkylcarbonyloxy-C₁- oder -C₂-alkyl, Carboxyl, C₁-C₃-Alkoxycarbonyl, C₁-C₃-Alkyl-ON=CH-, C₁-C₃-Alkoxycarbonyl-C₁- oder -C₂-alkyl, C₁-C₃-Alkylcarbonyl oder OHC- ist.

16. Verfahren zur Herstellung von Verbindungen der Formel I, worin A-B eine Gruppe 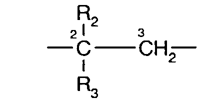 ist; R₂ die in Anspruch 1 angegebene Bedeutung hat; und R₃ C₁-C₆-Alkyl ist, dadurch gekennzeichnet, dass man eine Verbindung der Formel II 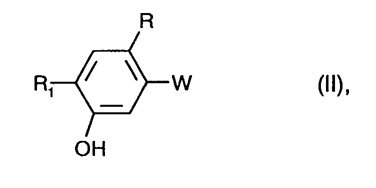 worin R, R₁ und W die in Anspruch 1 angegebene Bedeutung haben, mit einer Verbindung der Formel III 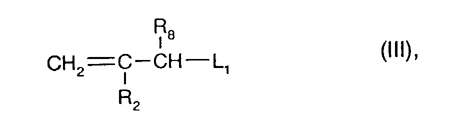 worin R₂ die angegebene Bedeutung hat; R₈ Wasserstoff oder C₁-C₅-Alkyl; und L₁ eine Abgangsgruppe ist, gegebenenfalls in Gegenwart eines inerten organischen Lösungsmittels und einer Base zur Verbindung der Formel IVa 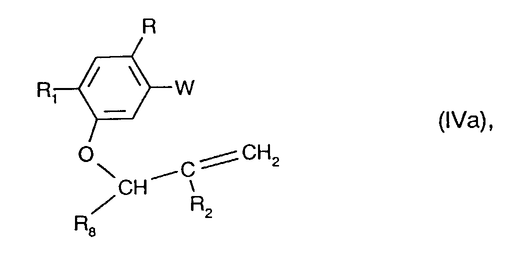 worin R, R₁, R₂, R₈ und W die angegebene Bedeutung haben, reagieren lässt, diese thermisch oder säurekatalytisch in die Verbindung der Formel Va 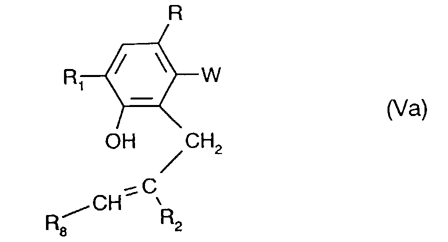 umlagert und anschliessend cyclisiert.

17. Verfahren zur Herstellung von Verbindungen der Formel I, worin A-B eine Gruppe 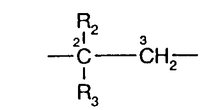 ist; R₂ die in Anspruch 1 angegebene Bedeutung hat; und R₃ Hydroxy-C₁-C₆-alkyl ist, dadurch gekennzeichnet, dass man eine Verbindung der Formel Va 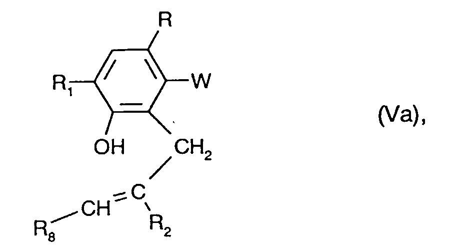 worin R, R₁, R₂ und W die in Anspruch 1 angegebene Bedeutung haben; und R₈ Wasserstoff oder C₁-C₅-Alkyl ist, epoxidiert und anschliessend gegebenenfalls in Gegenwart eines Katalysators cyclisiert.

18. Verfahren zur Herstellung von Verbindungen der Formel I nach Anspruch 1, worin A-B eine Gruppe 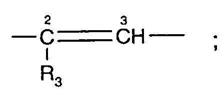 und R₃ C₁-C₆-Alkyl ist, dadurch gekennzeichnet, dass man eine Verbindung der Formel IVb 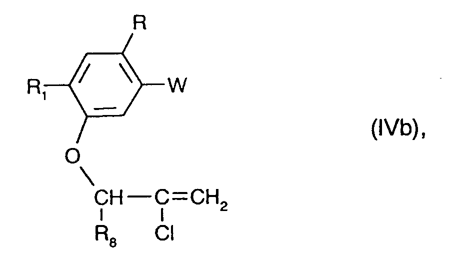 worin R, R₁ und W die in Anspruch 1 angegebene Bedeutung haben; und R₈ Wasserstoff oder C₁-C₅-Alkyl ist, thermisch in die Verbindung der Formel Vb 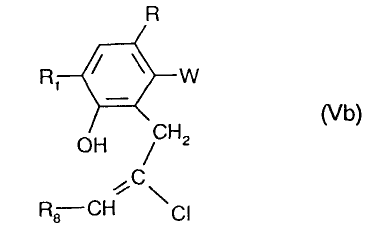 umlagert und anschliessend cyclisiert.

19. Verfahren zur Herstellung von Verbindungen der Formel I nach Anspruch1, worin W eine Gruppe 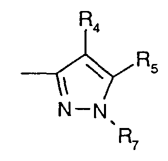 (W1); R₄ Wasserstoff, Fluor, Chlor oder Brom; R₅ C₁-C₆-Alkoxycarbonyl, NH₂C(O)- oder Cyano; und R₇ Wasserstoff oder C₁-C₄-Alkyl bedeutet, dadurch gekennzeichnet, daß man eine Verbindung der Formel VI 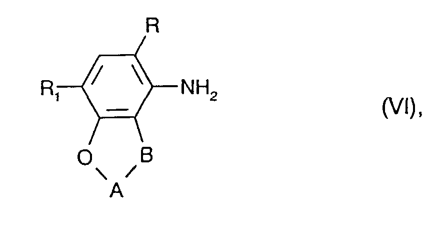 worin R, R₁ und A-B die in Anspruch 1 angegebene Bedeutung besitzen, diazotiert und die Diazoniumgruppe in Gegenwart eines Kupfer(I)-Salzes durch ein Halogen ersetzt und die Verbindung der Formel VII 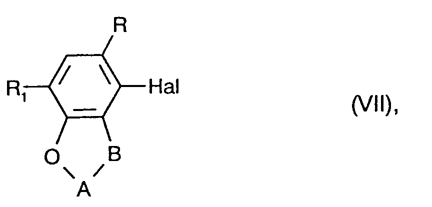 worin Hal Halogen bedeutet, erhält, diese in Gegenwart von Palladium(ll)-chlorid, Triphenylphosphin und n-Butylvinylether zur Verbindung der Formel VIII 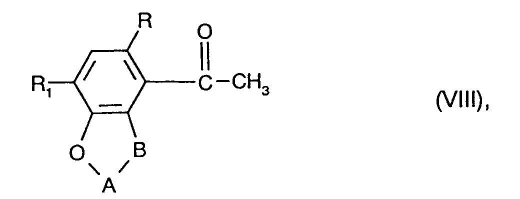 reagieren lässt, anschliessend mit einem Oxalsäuredialkylester der Formel IX 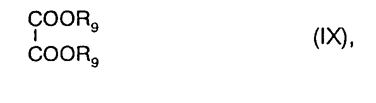 worin R₉ C₁-C₆-Alkyl ist, in Gegenwart einer Base zur Verbindung der Formel X 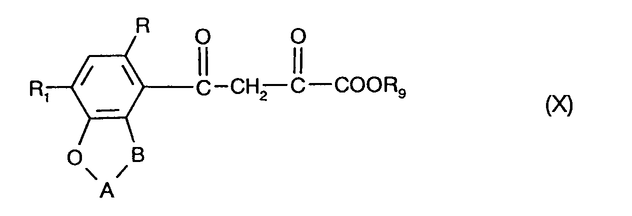 umsetzt, diese in Gegenwart von Hydrazin zur Verbindung der Formel la 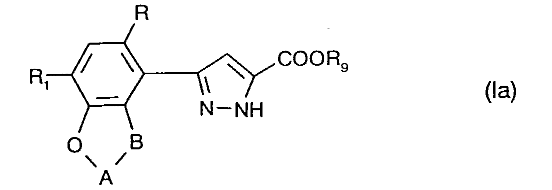 cyclisiert, anschliessend zuerst mit einem Alkylierungsreagens der Formel Xlla
R₇-L₂ (XIIa)
oder der Formel Xllb
R₇OSO₂OR₇ (XIIb),
wobei in den Verbindungen der Formeln Xlla und Xllb der Rest R₇ C₁-C₄-Alkyl und L₂ eine Abgangsgruppe bedeutet, zur Verbindung der Formel Ib 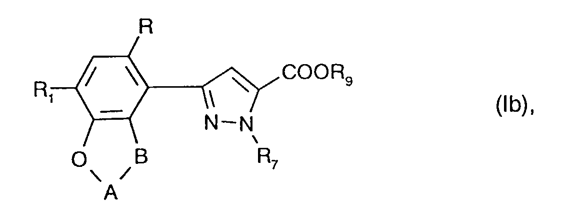 worin R, R₁, R₇, R₉ und A-B die angegebene Bedeutung haben, überführt, und nachfolgend einer Halogenierungsreaktion unterwirft und die Verbindung der Formel Ic 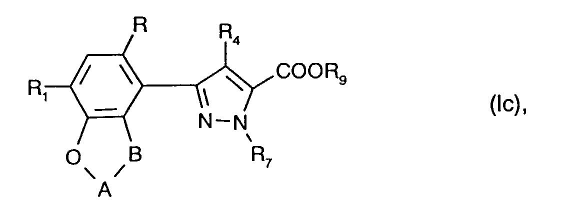 worin R, R₁, R₇, R₉ und A-B die angegebene Bedeutung haben; und R₄ Fluor, Chlor oder Brom bedeutet, erhält, und diese entweder direkt oder via die entsprechende Carbonsäure bzw. das entsprechende Carbonsäurehalogenid der Formel Ic₁ 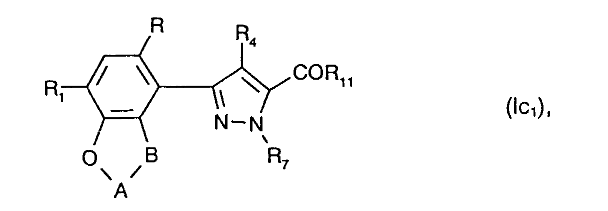 worin R, R₁, R₄, R₇ und A-B die angegebene Bedeutung haben; und R₁₁ Hydroxy oder Halogen bedeutet, mit Ammoniak zum Amid der Formel Id 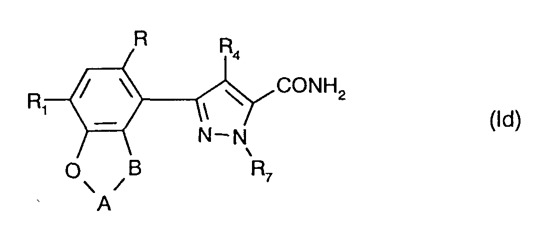 umsetzt und anschliessend dehydratisiert.

20. Verfahren zur Herstellung von Verbindungen der Formel I, worin W eine Gruppe 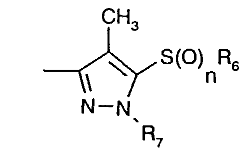 (W2) ist; R₆, R₇ und n die in Anspruch 1 angegebene Bedeutung haben, dadurch gekennzeichnet, dass man eine Verbindung der Formel Xl 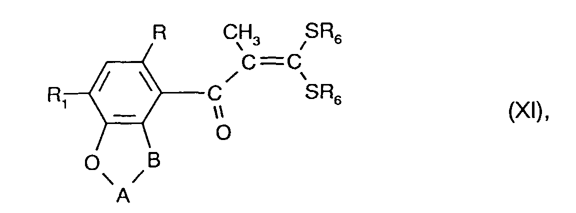 worin R, R₁, R₆ und A-B die in Anspruch 1 angegebene Bedeutung besitzen,
a) mit Hydrazin gegebenenfalls in Gegenwart eines geeigneten Lösungsmittels zur Verbindung der Formel le 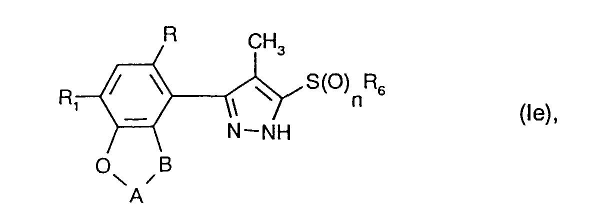 worin n 0 ist, cyclisiert und diese anschliessend mit einer Verbindung der Formel Xlla
R₇-L₂ (XIIa)
oder der Formel Xllb
R₇OSO₂OR₇ (XIIb),
wobei in den Verbindungen der Formeln Xlla und Xllb der Rest R₇ C₁-C₄-Alkyl und L₂ eine Abgangsgruppe bedeutet, zur Verbindung der Formel If 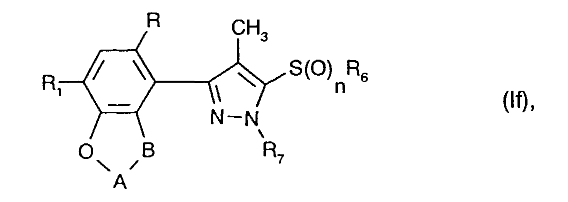 worin R, R₁, R₆, R₇ und A-B die angegebene Bedeutung haben; und n 0 ist, umsetzt und diese anschliessend oxidiert; oder
b) mit einer Verbindung der Formel XIII
NH₂-NH-R₇ (XIII),
worin R₇ die angegebene Bedeutung hat, gegebenenfalls in Gegenwart eines geeigneten Lösungsmittels zur Verbindung der Formel If cyclisiert und anschliessend oxidiert.

21. Verfahren zur Herstellung von Verbindungen der Formel I, worin W eine Gruppe 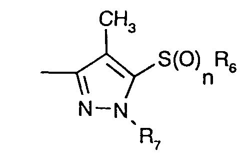 (W2); R₇ Wasserstoff ist; und R₆ und n die in Anspruch 1 angegebene Bedeutung haben, dadurch gekennzeichnet, dass man eine Verbindung der Formel Vllla 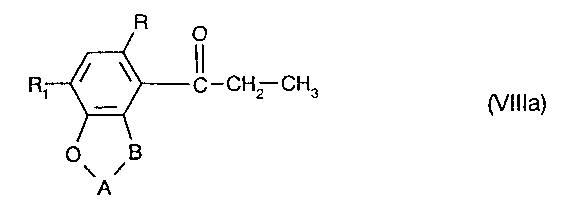 gegebenenfalls in Gegenwart eines Lösungsmittels und einer Base zur Verbindung der Formel XIV 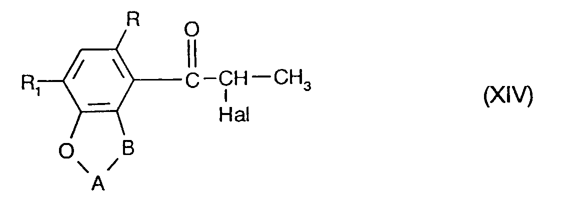 halogeniert, wobei in den Verbindungen der Formeln Vllla und XIV R, R₁ und A-B die in Anspruch 1 angegebene Bedeutung haben, und Hal Halogen ist, und diese mit der Verbindung der Formel XV
NH₂-NH-C(S)S-R₆ (XV),
worin R₆ die angegebene Bedeutung hat, gegebenenfalls in Gegenwart eines Lösungsmittels und einer Base zur Verbindung der Formel XVI 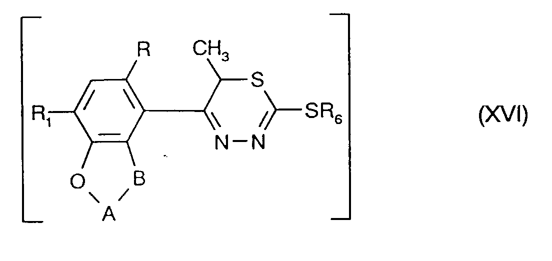 cyclisiert und diese thermisch oder säurekatalytisch einer Ringkontraktion unterwirft (n=0) und anschliessend oxidiert (n=1 bzw. 2).

22. Verbindungen der Formel V 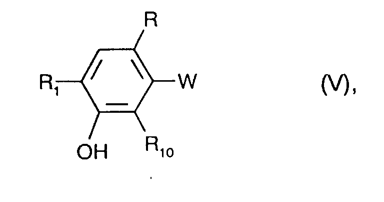 worin R, R₁ und W wie in Anspruch 1 definiert sind; R₁₀ eine Gruppe R₈-CH=C(R₂)-CH₂-oder R₈-CH=C(Cl)-CH₂-; R₂ Wasserstoff oder C₁-C₆-Alkyl; und R₈ Wasserstoff oder C₁-C₅-Alkyl bedeutet.

23. Verbindungen der Formel VIII 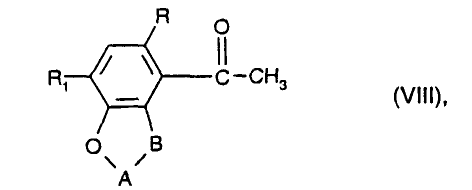 worin R, R₁ und A-B wie in Anspruch 1 definiert sind, wobei die Verbindung 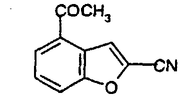 ausgeschlossen ist.

24. Verbindungen der Formel X 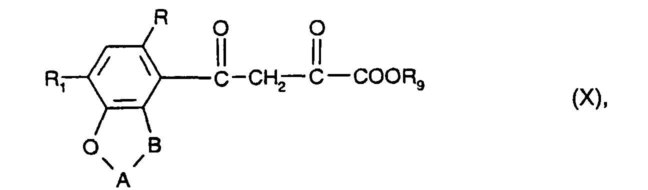 worin R, R₁ und A-B wie in Anspruch 1 definiert sind; und R₉ C₁-C₆-Alkyl ist.

25. Herbizides und den Pflanzenwuchs hemmendes Mittel, dadurch gekennzeichnet, daß es einen herbizid wirksamen Gehalt an Verbindung der Formel I, und einen inerten Träger aufweist.

26. Mittel gemäß Anspruch 25, dadurch gekennzeichnet, daß es zwischen 0,1 % und 95% Wirkstoff der Formel I enthält.

27. Verfahren zur Bekämpfung unerwünschten Pflanzenwachstums, dadurch gekennzeichnet, daß man einen Wirkstoff der Formel I, oder ein diesen Wirkstoff enthaltendes Mittel in einer herbizid wirksamen Menge auf die Pflanzen oder deren Lebensraum appliziert.

28. Verfahren gemäß Anspruch 27, dadurch gekennzeichnet, daß man eine Wirkstoffmenge zwischen 0,001 und 4 kg pro Hektar appliziert.

29. Verfahren zur Hemmung des Pflanzenwachstums, dadurch gekennzeichnet, daß man einen Wirkstoff der Formel I, oder ein diesen Wirkstoff enthaltendes Mittel in einer wirksamen Menge auf die Pflanzen oder deren Lebensraum appliziert.

30. Verwendung eines Mittels gemäß Anspruch 25 zur Bekämpfung von unerwünschtem Pflanzenwachstum.

## Claims

1. A compound of the formula I 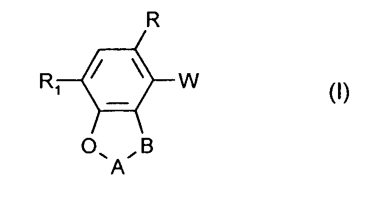 in which
R is hydrogen, fluorine or chlorine;
R₁ is hyrogen, halogen, trifluoromethyl, C₁-C₄haloalkoxy, cyano, nitro or amino;
A-B is a group 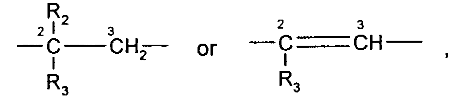 in which the 2 carbon atom is bonded to the oxygen atom;
W is a group 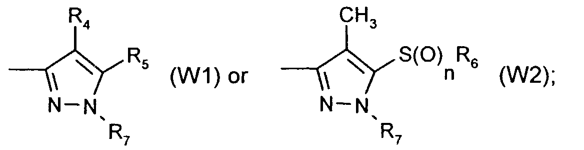
R₂ is hydrogen or C₁-C₆alkyl;
R₃ is C₁-C₆alkyl, C₁-C₆haloalkyl, cyano-C₁-C₄alkyl, hydroxy-C₁-C₆alkyl, C₁-C₆alkoxy-C₁-C₆alkyl, C₃-C₆alkenyloxy-C₁-C₄alkyl, C₃-C₆alkynyloxy-C₁-C₄alkyl, C₁-C₆alkylcarbonyloxy-C₁-C₆alkyl, carboxyl, C₁-C₆alkoxycarbonyl, C₁-C₆haloalkoxycarbonyl, C₃-C₆alkenyloxycarbonyl, C₃-C₆alkynyloxycarbonyl, C₃-C₆cycloalkoxycarbonyl, C₁-C₄alkoxy-C₁-Caalkoxycarbonyl, C₁-C₄alkylaminocarbonyl, di-C₁-C₄alkylaminocarbonyl, aminocarbonyl, benzyloxycarbonyl, phenyloxycarbonyl, C₁-C₄alkyl-SO₂NHC(O)-, C₁-C₆alkyl-ON=CH-, C₁-C₆alkoxycarbonyl-C₁-C₄alkyl, C₁-C₃alkylcarbonyl, ClC(O)-, NH₂C(S)-, OHC- or cyano;
R₄ is hydrogen, fluorine, chlorine or bromine;
R₅ is carboxyl, C₁-C₆alkoxycarbonyl, NH₂C(O)-, NH₂C(S)-, HON=CH-, OHC- or cyano;
R₆ is hydrogen or C₁-C₄alkyl;
n is 0, 1 or 2, with the proviso that n is 0 if R₆ is hydrogen; and
R₇ is hydrogen or C₁-C₄alkyl,
and to agronomically acceptable salt or stereoisomer thereof.

2. A compound according to claim 1 in which R₁ is hydrogen, fluorine, chlorine, bromine, trifluoromethyl or cyano.

3. A compound according to claim 1 in which R is hydrogen or fluorine.

4. A compound according to claim 3 in which R is fluorine.

5. A compound according to claim 1 where W is a group 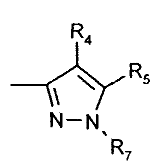 (W1); and R₄, R₅ and R₇ are as defined in claim 1.

6. A compound according to claim 5 in which R₇ is hydrogen, methyl or ethyl.

7. A compound according to claim 5 in which R₅ is carboxyl, C₁-C₃alkoxycarbonyl, NH₂C(S)-, HON=CH-, OHC- or cyano.

8. A compound according to claim 7 in which R₅ is cyano.

9. A compound according to claim 5 in which R₄ is hydrogen or chlorine.

10. A compound according to claim 1 in which W is a group 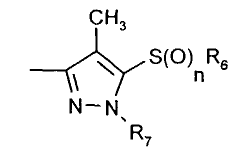 (W2); and R₆, R₇ and n are as defined in claim 1.

11. A compound according to claim 10 in which n is 0 or 2.

12. A compound according to claim 10 in which R₆ is methyl.

13. A compound according to claim 10 in which R₇ is methyl or ethyl.

14. A compound according to claim 13 in which R₇ is methyl.

15. A compound according to claim 1 in which A-B is a group 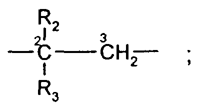 R₂ is hydrogen or methyl; and R₃ is hydrogen, C₁-C₃alkyl, C₁- or C₂haloalkyl, cyano-C₁-C₄alkyl, C₁-C₃alkylcarbonyloxy-C₁- or -C₂alkyl, carboxyl, C₁-C₃alkoxycarbonyl, C₁-C₃alkyl-ON=CH-, C₁-C₃alkoxycarbonyl-C₁- or -C₂alkyl, C₁-C₃alkylcarbonyl or OHC-.

16. A process for the preparation of a compound of the formula I in which A-B is a group 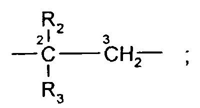 R₂ is as defined in claim 1; and R₃ is C₁-C₆alkyl, which comprises reacting a compound of the formula II 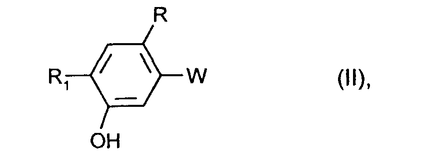 in which R, R₁ and W are as defined in claim 1 with a compound of the formula III 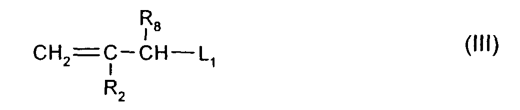 in which R₂ is as defined above; R₈ is hydrogen or C₁-C₅alkyl; and L₁ is a leaving group, if appropriate in the presence of an inert organic solvent and of a base, to give the compound of the formula IVa 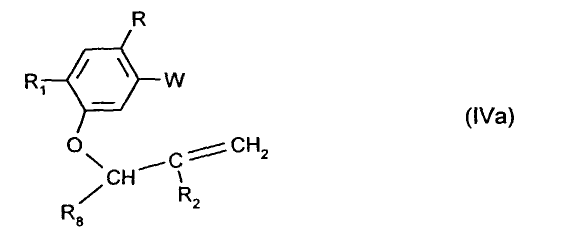 in which R, R₁, R₂, R₈ and W are as defined above, subjecting this compound to a rearrangement reaction, either with exposure to heat or with acid catalysis, to give the compound of the formula Va 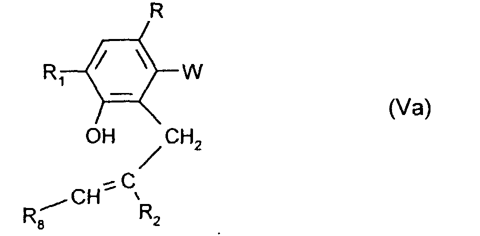 and subsquently cyclizing the latter.

17. A process for the preparation of a compound of the formula I in which A-B is a group 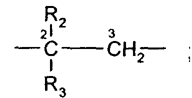 R₂ is as defined in claim 1 and R₃ is hydroxy-C₁-C₆alkyl, which comprises epoxidizing a compound of the formula Va 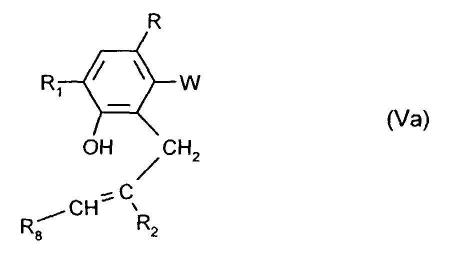 in which R, R₁, R₂ and W are as defined in claim 1; and R₈ is hydrogen or C₁-C₅alkyl, and subsequently, if desired, cyclizing the product in the presence of a catalyst.

18. A process for the preparation of a compound of the formula I as claimed in claim 1 in which A-B is a group 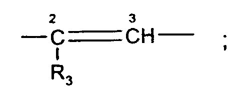 and R₃ is C₁-C₆alkyl, which comprises subjecting a compound of the formula IVb 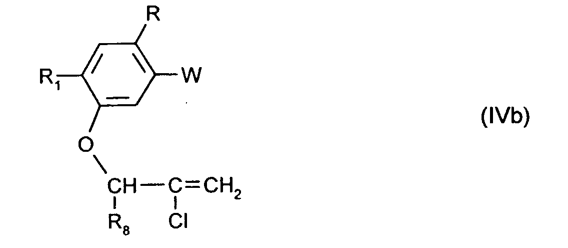 in which R, R₁ and W are as defined in claim 1; and R₈ is hydrogen or C₁-C₅alkyl to a rearrangement reaction with exposure to heat to give the compound of the formula Vb 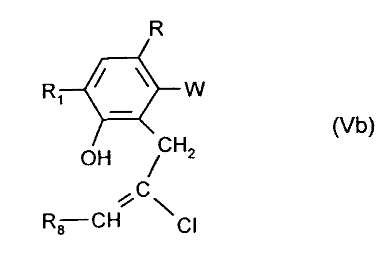 and subsequently cyclizing the latter.

19. A process for the preparation of a compound of the formula I according to claim 1 in which W is a group 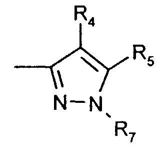 (W1); R₄ is hydrogen, fluorine, chlorine or bromine; R₅ is C₁-C₆alkoxywarbonyl, NH₂C(O)- or cyano; and R₇ is hydrogen or C₁-C₄alkyl, which comprises diazotizing a compound of the formula VI 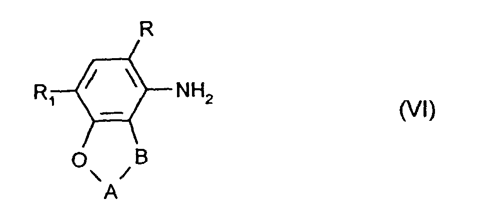 in which R, R₁ and A-B have the meaning given in claim 1, replacing the diazonium group by a halogen in the presence of a copper(l) salt, thus obtaining the compound of the formula VII 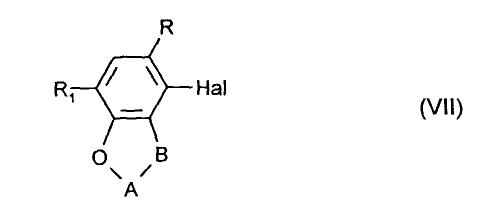 in which Hal is halogen, reacting this compound in the presence of palladium(ll) chloride, triphenylphosphine and n-butyl vinyl ether to give the compound of the formula VIII 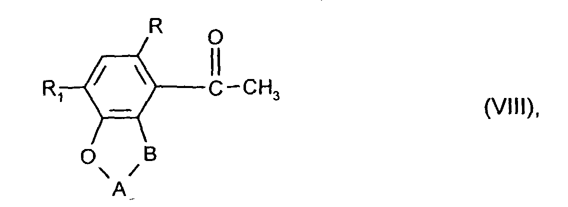 subsequently reacting this compound with a dialkyloxalate of the formula IX 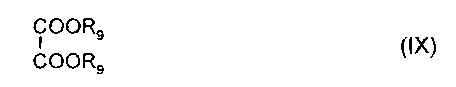 in which R₉ is C₁-C₆alkyl, in the presence of a base, to give the compound of the formula X 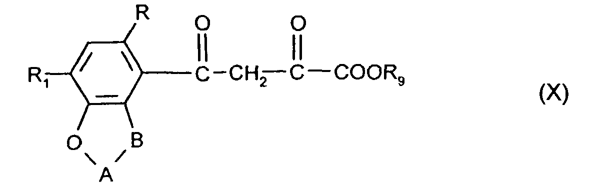 cyclizing the latter in the presence of hydrazine to give the compound of the formula la 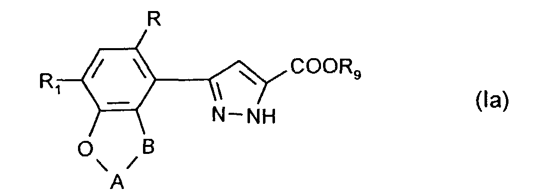 subsequently converting the latter first with an alkylating reagent of the formula Xlla
R₇-L₂ (XIIa)
or of the formula Xllb
R₇OSO₂OR₇ (XIIb)
where the radical R₇ in the compounds of the formulae Xlla and Xllb is C₁-C₄alkyl and L₂ is a leaving group to give the compound of the formula Ib 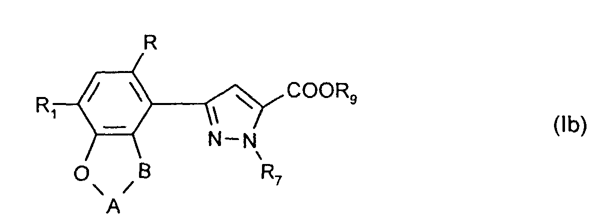 in which R, R₁, R₇, R₉ and A-B are as defined above, and subsequently subjecting the product to a halogenation reaction, thus obtaining the compound of the formula Ic 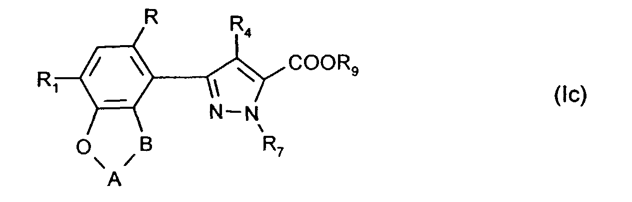 in which R, R₁, R₇, R₉ and A-B are as defined above; and R₄ is fluorine, chlorine or bromine, and reacting this compound either directly or via the corresponding carboxylic acid or the corresponding carboxylic acid halide of the formula Ic₁ 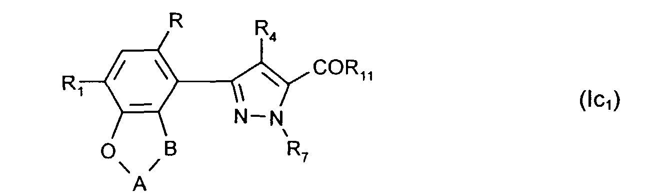 in which R, R₁, R₄, R₇ and A-B are as defined above; and R₁₁ is hydroxyl or halogen, with ammonia to give the amide of the formula Id 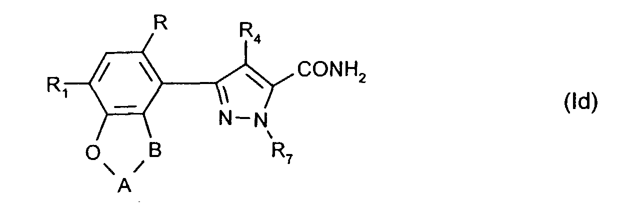 and subsequently dehydrating the latter.

20. A process for the preparation of a compound of the formula I in which W is a group 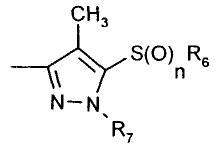 (W2); R₆, R₇ and n are as defined in claim 1, which comprises
a) cyclizing a compound of the formula Xl 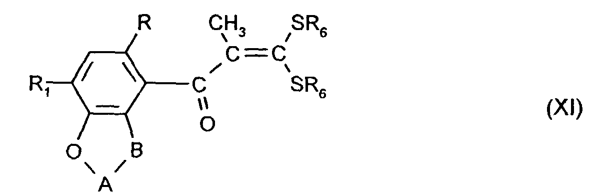 in which R, R₁, R₆ and A-B are as defined in claim I with hydrazine, in the presence or absence of a suitable solvent, to give the compound of the formula le 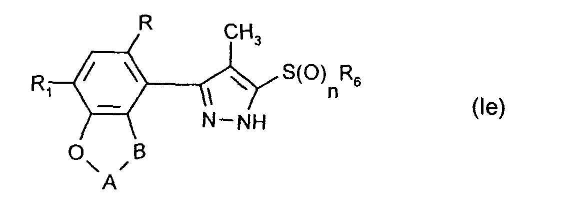 in which n is 0 and subsequently reacting this compound with a compound of the formula Xlla
R₇-L₂ (XIIa)
or of the formula Xllb
R₇OSO₂OR₇ (XIIb)
the radical R₇ in the compounds of the formulae Xlla and Xllb being C₁-C₄alkyl and L₂ being a leaving group to give the compound of the formula If 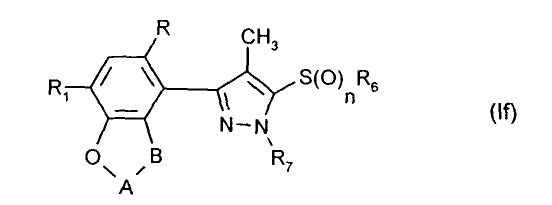 in which R, R₁, R₆, R₇ and A-B are as defined above; and n is 0, and subsequently oxidizing the product; or
b) cyclizing a compound of the formula XIII
(XIII)NH₂-NH-R₇ (XIII)
in which R₇ is as defined above, in the presence or absence of a suitable solvent, to give the compound of the formula If and subsequently oxidizing this compound.

21. A process for the preparation of a compound of the formula I in which W is a group 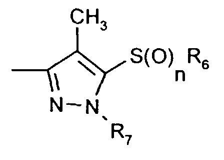 (W2); R₇ is hydrogen; and R₆ and n are as defined in claim 1, which comprises halogenating a compound of the formula Vllla 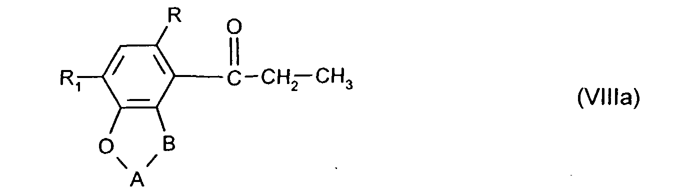 in the presence or absence of a solvent and of a base, to give the compound of the formula XIV 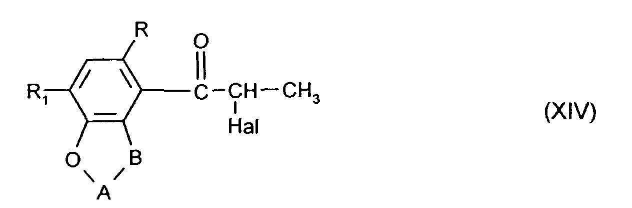 R, R₁ and A-B in the compounds of the formulae Villa and XIV being as defined in claim 1 and Hal being halogen and cyclizing this compound with the compound of the formula XV
NH₂-NH-C(S)S-R₆ (XV)
in which R₆ is as defined above, in the presence or absence of a solvent and of a base, to give the compound of the formula XVI 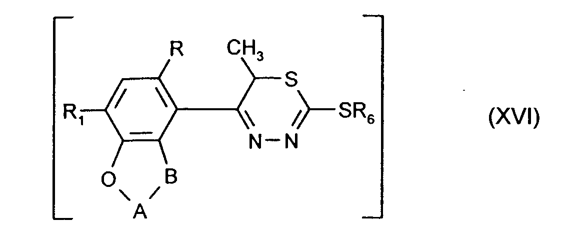 and subjecting the latter to a ring contraction (n=0), either with exposure to heat or with acid catalysis, and subsequently oxiding the product (n=1 or 2).

22. A compound of the formula V 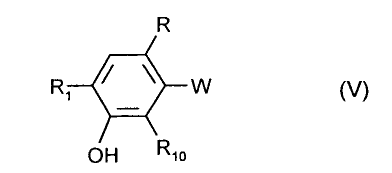 in which R, R₁ and W are as defined in claim 1; R₁₀ is a group R₈-CH=C(R₂)-CH₂-or R₈-CH=C(Cl)-CH₂-; R₂ is hydrogen or C₁-C₆alkyl; and R₈ is hydrogen or C₁-C₅alkyl.

23. A compound of the formula VIII 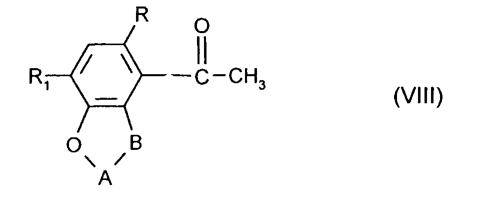 in which R, R₁ and A-B are as defined in claim 1, the compound 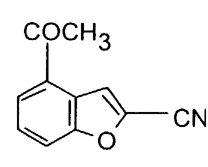 being excluded.

24. A compound of the formula X 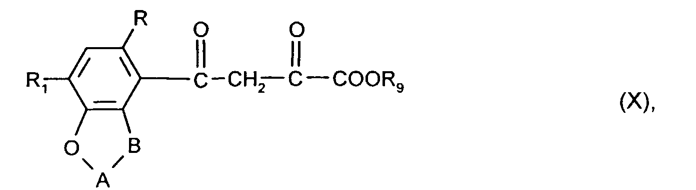 in which R, R₁ and A-B are as defined in claim 1; and R₉ is C₁-C₆alkyl.

25. A herbicidal and plant-growth-inhibiting composition, which comprises a herbicidally effective amount of compound of the formula I and an inert carrier.

26. A composition according to claim 25, which comprises between 0.1% and 95% of active ingredient of the formula I.

27. A method of controlling undesirable plant growth, which comprises applying a herbicidally active amount of an active ingredient of the formula I or of a compositoin which comprises this active ingredient to the plants or their environment.

28. A method according to claim 27, which comprises applying an amount of active ingredient of between 0.001 and 4 kg per hectare.

29. A method of inhibiting plant growth, which comprises applying an effective amount of an active ingredient of the formula I or of a composition which comprises this active ingredient to the plants or their environment.

30. The use of a composition according to claim 25 for controlling undesirable plant growth.

## Revendications

1. Composés de formule I 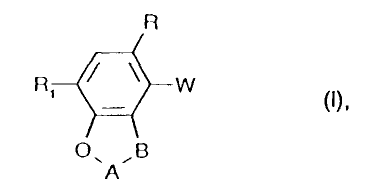 dans laquelle
R représente un atome d'hydrogène, de fluor ou de chlore ;
R₁ représente un atome d'hydrogène ou d'halogène ou un groupe trifluorométhyle, halogénoalcoxy en C₁-C₄, cyano, nitro ou amino ;
A-B représente un groupe 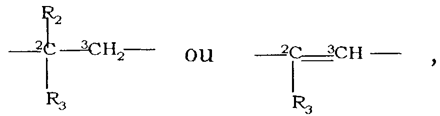 l'atome de carbone en position 2 étant lié à l'atome d'oxygène :
W est un groupe 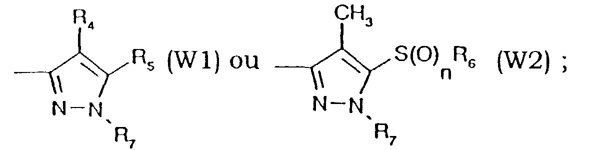
R₂ représente un atome d'hydrogène ou un groupe alkyle en C₁-C₆ ;
R₃ représente un groupe alkyle en C₁-C₆, halogénoalkyle en C₁-C₆, cyano-alkyle(C₁-C₄), hydroxyalkyle en C₁-C₆, alcoxy(C₁-C₆)-alkyle-(C₁-C₆), alcényloxy(C₃-C₆)-alkyle(C₁-C₄), alcynyloxy(C₃-C₆)-alkyle-(C₁-C₄), alkyl(C₁-C₆)carbonyloxy-alkyle(C₁-C₆), carboxy, alcoxy-(C₁-C₆)carbonyle, halogénoalcoxy(C₁-C₆)carbonyle, alcényloxy-(C₃-C₆)carbonyle, alcynyloxy(C₃-C₆)carbonyle, cycloalcoxy(C₃-C₆)-carbonyle, alcoxy(C₁-C₄)-alcoxy(C₁-C₄)carbonyle, alkyl(C₁-C₄)aminocarbonyle, dialkyl(C₁-C₄)aminocarbonyle, aminocarbonyle, benzyloxycarbonyle, phényloxycarbonyle, alkyl(C₁-C₄)-SO₂NHC(O)-, alkyl-(C₁-C₆)-ON=CH-, alcoxy(C₁-C₆)carbonyl-alkyle(C₁-C₄), alkyl(C₁-C₃)-carbonyle, ClC(O)-, NH₂C(S)-, OHC- ou cyano ;
R₄ représente un atome d'hydrogène, de fluor, chlore ou brome ;
R₅ représente un groupe carboxy, alcoxy(C₁-C₆)carbonyle, NH₂C(O)-, NH₂C(S)-, HON=CH-, OHC- ou cyano ;
R₆ représente un atome d'hydrogène ou un groupe alkyle en C₁-C₄ ;
n représente 0, 1 ou 2, étant entendu que n est égal à 0 lorsque R₆ représente un atome d'hydrogène ; et
R₇ est un atome d'hydrogène ou un groupe alkyle en C₁-C₄,
et stéréoisomères et sels agronomiquement acceptables de tels composés.

2. Composés selon la revendication 1, dans lesquels R₁ est un atome d'hydrogène, de fluor, chlore ou brome, ou le groupe trifluorométhyle ou cyano.

3. Composés selon la revendication 1, dans lesquels R est un atome d'hydrogène ou de fluor.

4. Composés selon la revendication 3, dans lesquels R est un atome de fluor.

5. Composés selon la revendication 1, dans lesquels W est un groupe 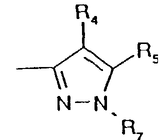 (W1) ; et R₄, R₅ et R₇ ont les significations données dans la revendication 1.

6. Composés selon la revendication 5, dans lesquels R₇ est un atome d'hydrogène ou le groupe méthyle ou éthyle.

7. Composés selon la revendication 5, dans lesquels R₅ est un groupe carboxy, alcoxy(C₁-C₃)carbonyle, NH₂C(S)-, HON=CH-, OHC- ou cyano.

8. Composés selon la revendication 7, dans lesquels R₅ est le groupe cyano.

9. Composés selon la revendication 5, dans lesquels R₄ est un atome d'hydrogène ou de chlore.

10. Composés selon la revendication 1, dans lesquels W est un groupe 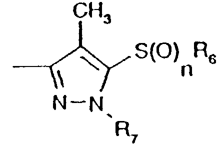 (W2); et R₆, R₇ et n ont les significations données dans la revendication 1.

11. Composés selon la revendication 10, dans lesquels n est 0 ou 2.

12. Composés selon la revendication 10, dans lesquels R₆ est le groupe méthyle.

13. Composés selon la revendication 10, dans lesquels R₇ est le groupe méthyle ou éthyle.

14. Composés selon la revendication 13, dans lesquels R₇ est le groupe méthyle.

15. Composés selon la revendication 1, dans lesquels A-B est un groupe 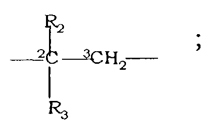 R₂ est un atome d'hydrogène ou le groupe méthyle ; et R₃ est un atome d'hydrogène ou un groupe alkyle en C₁-C₃, halogénoalkyle en C₁ ou C₂, cyanoalkyl(C₁-C₄), alkyl(C₁-C₃)carbonyloxyalkyle(C₁ ou C₂), carboxy, alcoxy(C₁-C₃)carbonyle, alkyl(C₁-C₃)-ON=CH-, alcoxy(C₁-C₃)carbonyl-alkyle(C₁ ou C₂), alkyl(C₁-C₃)carbonyle ou OHC-.

16. Procédé pour la préparation des composés de formule I dans lesquels A-B est un groupe 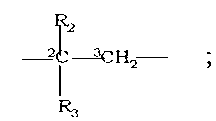 R₂ a la signification donnée dans la revendication 1 ; et R₃ est un groupe alkyle en C₁-C₆, caractérisé en ce que l'on fait réagir un composé de formule II 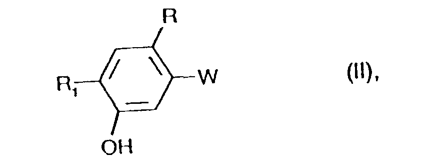 dans laquelle R, R₁ et W ont les significations données à propos de la formule I, avec un composé de formule III 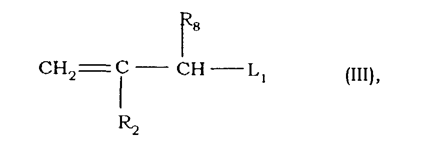 dans laquelle R₂ a la signification donnée ; R₈ représente un atome d'hydrogène ou un groupe alkyle en C₁-C₅ ; et L₁ représente un groupe partant, éventuellement en présence d'un solvant organique inerte et d'une base, pour aboutir au composé de formule IVa 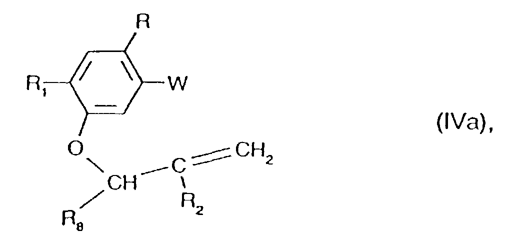 dans laquelle R, R₁, R₂, R₈ et W ont les significations indiquées, ou on soumet ce composé à une transposition thermique ou catalytique avec catalyse par un acide, pour aboutir au composé de formule Va 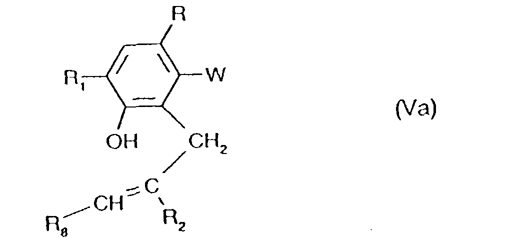 et on soumet ensuite ce dernier à une cyclisation.

17. Procédé pour la préparation des composés de formule I dans lesquels A-B est un groupe 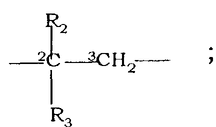 R₂ a la signification donnée dans la revendication 1 ; et R₃ est un groupe hydroxyalkyle en C₁-C₆, caractérisé en ce que l'on soumet à une époxydation un composé de formule Va 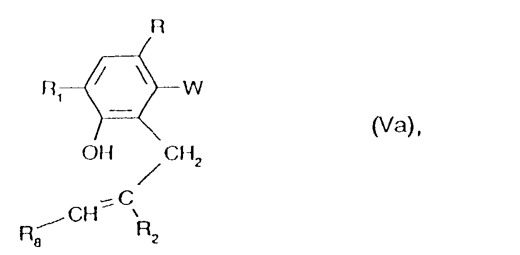 dans laquelle R, R₁, R₂ et W ont les significations données dans la revendication 1 ; et R₆ est un atome d'hydrogène ou un groupe alkyle en C₁-C₅, et éventuellement on soumet ensuite le produit à une cyclisation en présence d'un catalyseur.

18. Procédé pour la préparation des composés de formule I selon la revendication 1, dans lesquels A-B est un groupe 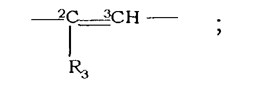 et R₃ est un groupe alkyle en C₁-C₆, caractérisé en ce que l'on soumet à une transposition thermique un composé de formule IVb 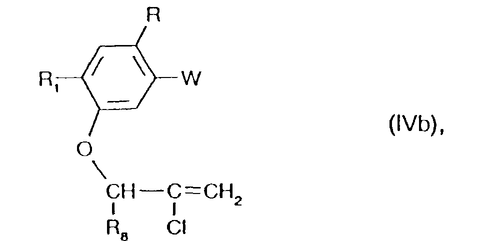 dans laquelle R, R₁ et W ont les significations données dans la revendication 1 ; et R₈ est un atome d'hydrogène ou un groupe alkyle en C₁-C₅, pour aboutir au composé de formule Vb 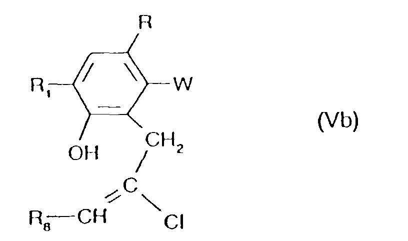 et on soumet ensuite ce dernier à une cyclisation.

19. Procédé pour la préparation des composés de formule I selon la revendication 1, dans lesquels W est un groupe 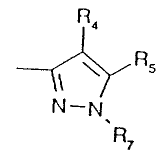 (W1) ; R₄ représente un atome d'hydrogène, de fluor, chlore ou brome ; R₅ représente un groupe alcoxy(C₁-C₆)carbonyle, NH₂C(O)- ou cyano ; et R₇ représente un atome d'hydrogène ou un groupe alkyle en C₁-C₄, caractérisé en ce que l'on soumet à une diazotation un composé de formule VI 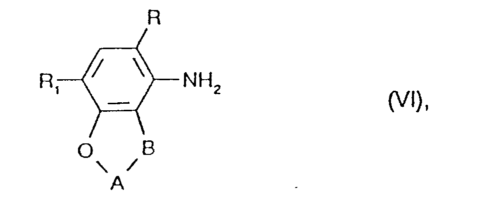 dans laquelle R, R₁ et A-B ont les significations données dans la revendication 1, et on remplace par un atome d'halogène le groupe diazonium en présence d'un sel cuivreux, et on obtient le composé de formule VII 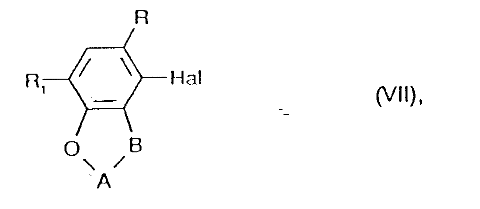 dans laquelle Hal représente un atome d'halogène, on fait réagir celui-ci en présence de chlorure de palladium-(II), triphénylphosphine et éther n-butylvinylique, pour aboutir au composé de formule VIII 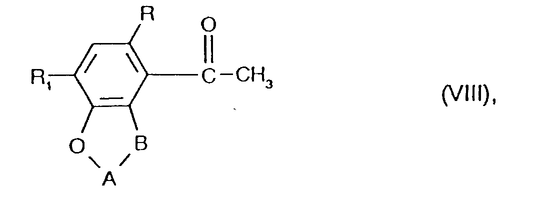 on fait ensuite réagir ce dernier avec un oxalate de dialkyle de formule IX 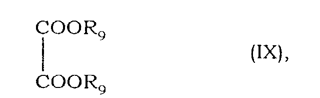 dans laquelle R₉ est un groupe alkyle en C₁-C₆, en présence d'une base, pour aboutir au composé de formule X 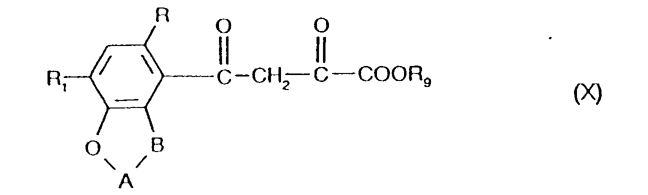 on soumet ce dernier à une cyclisation en présence d'hydrazine, pour aboutir au composé de formule Ia 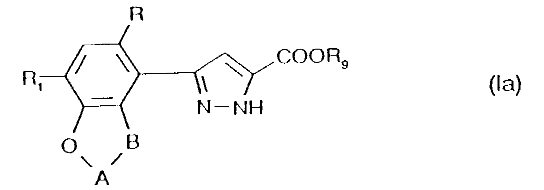 ensuite d'abord on convertit ce dernier, à l'aide d'un réactif d'alkylation de formule XIIa
R₇-L₂ (XIIa)
ou de formule XIIb
R₇OSO₂OR₇ (XIIb),
dans les composés de formule XIIa et XIIb, le radical R₇ représentant un groupe alkyle en C₁-C₄ et L₂ représentant un groupe partant, en le composé de formule Ib 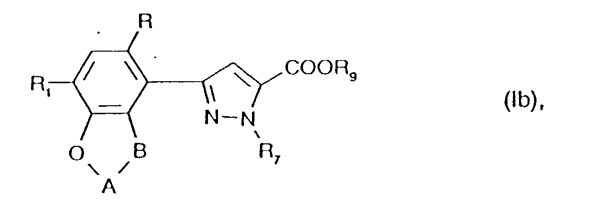 dans laquelle R, R₁, R₇, R₉ et A-B ont les significations données, et ensuite on soumet ce dernier à une réaction d'halogénation, et on obtient le composé de formule Ic 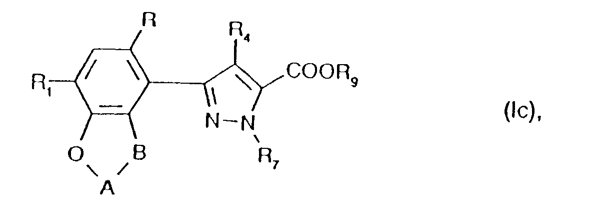 dans laquelle R, R₁, R₇, R₉ et A-B ont les significations indiquées ; et R₄ représente un atome de fluor, chlore ou brome, et on le fait réagir avec de l'ammoniac, soit directement, soit en passant par l'acide carboxylique correspondant ou l'halogénure de carbonyle correspondant de formule Ic₁ 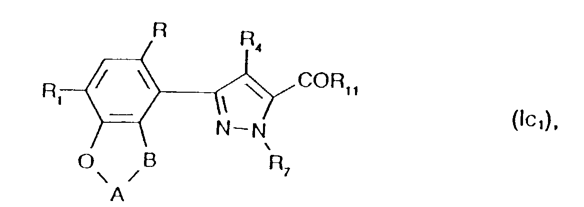 dans laquelle R, R₁, R₄, R₇ et A-B ont les significations indiquées ; et R₁₁ représente le groupe hydroxy ou un atome d'halogène, pour aboutir à l'amide de formule Id 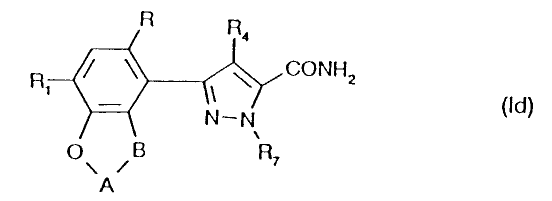 qui est ensuite déshydraté.

20. Procédé pour la préparation des composés de formule I dans lesquels W est un groupe 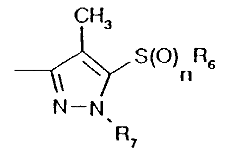 (W2) ; et R₆, R₇ et n ont les significations données dans la revendication I, caractérisé en ce que l'on soumet à une cyclisation un composé de formule XI 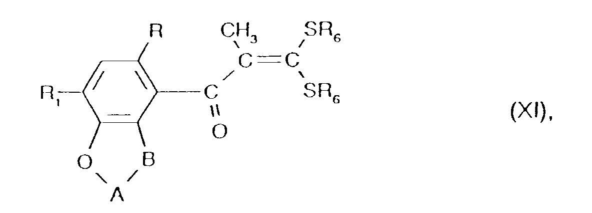 dans laquelle R, R₁, R₆ et A-B ont les significations données dans la revendication 1,
a) avec de l'hydrazine, éventuellement en présence d'un solvant convenable, pour aboutir au composé de formule le 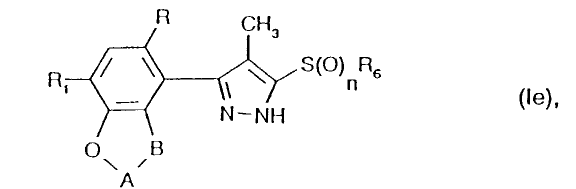 dans laquelle n est égal à 0, et on fait ensuite réagir ce dernier avec un composé de formule XIIa
R₇-L₂ (XIIa)
ou de formule XIIb
R₇OSO₂OR₇ (XIIb),
dans les composés de formule XIIa et XIIb, le radical R₇ représentant un groupe alkyle en C₁-C₄ et L₂ représentant un groupe partant, pour aboutir au composé de formule If 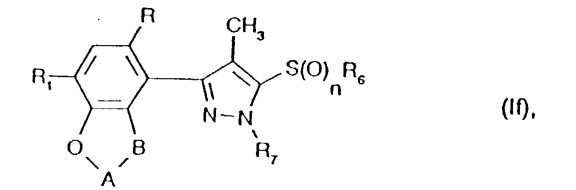 dans laquelle R, R₁, R₆, R₇ et A-B ont les significations indiquées ; et
n est 0, et ensuite on l'oxyde ; ou
b) avec un composé de formule XIII
NH₂-NH-R₇ (XIII)
dans laquelle R₇ a la signification donnée, éventuellement en présence d'un solvant convenable, pour aboutir au composé de formule If, et celui-ci est ensuite oxydé.

21. Procédé pour la préparation des composés de formule I dans lesquels W est un groupe 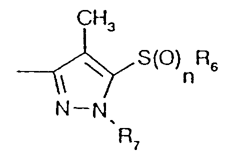 (W2) ; R₇ est un atome d'hydrogène ; et R₆ et n ont les significations données dans la revendication I, caractérisé en ce que l'on soumet à une halogénation un composé de formule VIIIa 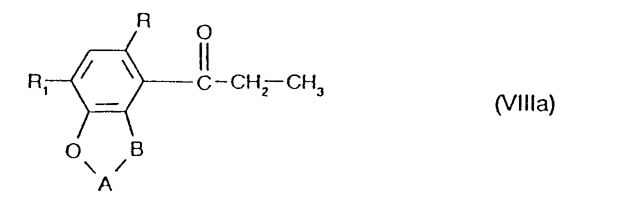 éventuellement en présence d'un solvant et d'une base, pour aboutir au composé de formule XIV 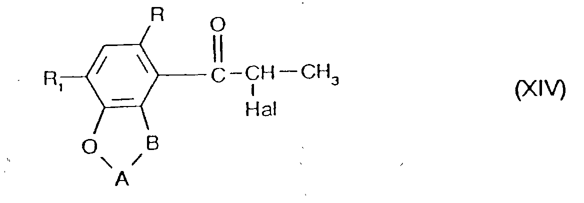 dans les composés de formule VIIIa et XIV, R, R₁ et A-B ayant les significations données dans la revendication I, et Hal étant un atome d'halogène, et on soumet ce dernier à une cyclisation avec un composé de formule XV
NH₂-NH-C(S)S-R₆ (XV)
dans laquelle R₆ a la signification indiquée, éventuellement en présence d'un solvant et d'une base, pour aboutir au composé de formule XVI 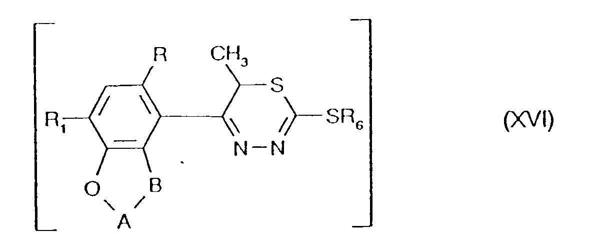 et on soumet ce dernier à une contraction de cycle par voie thermique ou catalytique avec catalyse par un acide (n = 0), et ensuite on l'oxyde (n = 1 ou 2).

22. Composés de formule V 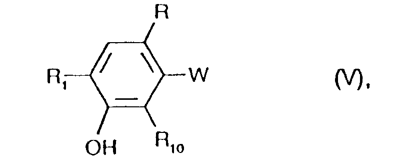 dans laquelle R, R₁ et W sont tels que définis dans la revendication 1 ; R₁₀ représente un groupe R₈-CH=C(R₂)-CH₂- ou R₈-CH=C(Cl)-CH₂- ; R₂ représente un atome d'hydrogène ou un groupe alkyle en C₁-C₆ ; et R₈ représente un atome d'hydrogène ou un groupe alkyle en C₁-C₅.

23. Composés de formule VIII 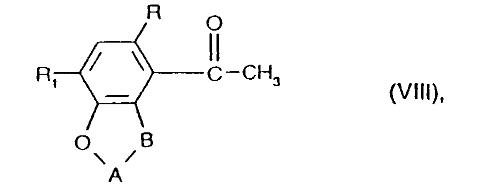 dans laquelle R, R₁ et A-B sont tels que définis dans la revendication 1, le composé 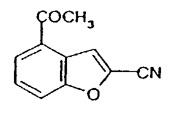 étant exclu.

24. Composés de formule X 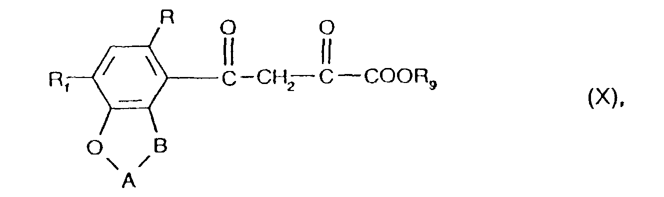 dans laquelle R, R₁ et A-B sont tels que définis dans la revendication 1 ; et R₆ et un groupe alkyle en C₁-C₆.

25. Produit herbicide et inhibant la croissance de plantes, caractérisé en ce qu'il présente une teneur à activité herbicide en un composé de formule I, et comporte un support inerte.

26. Produit selon la revendication 25, caractérisé en ce qu'il contient entre 0,1 % et 95 % de substance active de formule I.

27. Procédé pour la lutte contre la croissance indésirable de plantes, caractérisé en ce que l'on applique sur les plantes ou leur espace vital une substance active de formule I ou un produit contenant cette substance active, en une quantité à activité herbicide.

28. Procédé selon la revendication 27, caractérisé en ce que l'on applique une quantité de substance active comprise entre 0,001 et 4 kg par hectare.

29. Procédé pour l'inhibition de la croissance de plantes, caractérisé en ce que l'on applique sur les plantes ou leur espace vital en une quantité efficace une substance active de formule I ou un produit contenant cette substance active.

30. Utilisation d'un produit selon la revendication 25, pour la lutte contre la croissance indésirable de plantes.
